# EUROPEAN PATENT SPECIFICATION

(11) **EP 1 105 728 B1**
(45) Date of publication and mention of the grant of the patent: **13.04.2005**
(21) Application number: 99943759.3
(22) Date of filing: 18.08.1999
(51) Int. Cl.: G01N 33/50, C12Q 1/68, G01N 33/68, A61K 31/40, A61K 31/535, A61K 31/55

(54) **MODULATING MULTIPLE LINEAGE KINASE PROTEINS**
MODULIERUNG VON MLK- (MULTIPLE LINEAGE KINASE) PROTEINEN
MODULATION DE PROTEINES KINASE A LIGNEE MULTIPLE

(30) Priority: 26.08.1998 US 97980 P
(43) Date of publication of application: 13.06.2001
(73) Proprietor: CEPHALON, INC., West Chester, PA 19380 (US)
(72) Inventor: MARONEY, Anna, Media, PA 19063 (US); WALTON, Kevin, M., Old Saybrook, CT 06475 (US); DIONNE, Craig, A., Downington, PA 19335 (US); NEFF, Nicola, Wallingford, PA 19086 (US); KNIGHT, Ernest, Jr., Hilton Head Island, SC 29928 (US); GLICKSMAN, Marcie, A., Swarthmore, PA 19089 (US)
(74) Representative: Hallybone, Huw George
(86) International application number: PCT/US1999/018864
(87) International publication number: WO 2000/013015

(56) References cited:
- WO-A-99/58982
- US-A- 5 475 110
- US-A- 5 756 494
- MARONEY AC, GLICKSMAN MA, BASMA AN, WALTON KM, KNIGHT E JR, MURPHY CA, BARTLETT BA, FINN JP, ANGELES T ET AL. : "Motoneuron apoptosis is blocked by CEP-1347 (KT 7515), a novel inhibitor of the JNK signaling pathway" JOURNAL OF NEUROSCIENCE, vol. 18, no. 1, 1 January 1998 (1998-01-01), pages 104-111, XP000870100 cited in the application
- GLICKSMAN MA, CHIU AY, DIONNE CA, HARTY M, KANEKO M, MURAKATA C, OPPENHEIM RW, PREVETTE D, SENGELAUB DR, VAUGHT JL, NEFF NT: "CEP-1347/KT7515 prevents motor neuronal programmed cell death and injury-induced dedifferentiation in vivo" JOURNAL OF NEUROBIOLOGY, vol. 34, no. 4, 15 June 1998 (1998-06-15), pages 361-370, XP000870310 cited in the application
- GLICKSMAN MA, PRANTNER JE, MEYER SL, FORBES ME, DASGUPTA M, LEWIS ME, NEFF N: "K-252a and staurosporine promote choline acetyltransferase activity in rat spinal cord cultures" JOURNAL OF NEUROCHEMISTRY, vol. 61, no. 1, July 1993 (1993-07), pages 210-221, XP000870452 cited in the application
- M KANEKO, Y SAITO, H SAITO, T MATSUMOTO, Y MATSUDA, J L VAUGHT, C A DIONNE, T S ANGELES, M A GLICKSMAN, N T NEFF ET AL.: "Neurotrophic 3,9-Bis[(alkylthio)methyl]- and -Bis(alkoxymethyl)-K-252a Derivatives" JOURNAL OF MEDICINAL CHEMISTRY, vol. 40, no. 12, 1997, pages 1863-1869, XP002128804 cited in the application
- T S ANGELES, C STEFFLER, B A BARTLETT, R L HUDKINS, R M STEPHENS, D R KAPLAN, C A DIONNE: "Enzyme-linked Immunosorbent Assay for trkA Tyrosine Kinase Activity" ANALYTICAL BIOCHEMISTRY, vol. 236, 1996, pages 49-55, XP002128805 cited in the application
- E KNIGHT, T J CONNORS, A C MARONEY, T S ANGELES, R L HUDKIN, C A DIONNE: "A Radioactive Binding Assay for Inhibitors of trkA Kinase" ANALYTICAL BIOCHEMISTRY, vol. 247, 1997, pages 376-381, XP002128806 cited in the application
- FANGER GR, GERWINS P, WIDMANN C, JARPE MB, JOHNSON GL: "MEKKs, GCKs, MLKs, PAKs, TAKs, and tpls: upstream regulators of the c-Jun amino-terminal kinases?" CURRENT OPINION IN GENETICS & DEVELOPMENT, vol. 7, no. 1, February 1997 (1997-02), pages 67-74, XP000870102

## Description

The present invention is directed, in part, to methods for modulating members of the multiple lineage kinase (MLK) family, methods for identifying compounds which modulate a multiple lineage kinase protein and either promote cell survival or promote cell death, methods for identifying compounds which may be useful in the treatment of neurodegenerative disorders and/or inflammation, and methods of treating neurodegenerative disorders with compounds which inhibit a multiple lineage kinase protein.

### Background of the Invention

The MLK family comprises a group of proteins in which the protein sequence of the kinase domains of the family members closely resemble the MAPKKKs but have greater similarity to each other than to other MAPKKKs. MLK family members comprise a portion of very complex kinase cascades such as, for example, the stress-signaling cascade, which involves modulation of, *inter alia*, the c-Jun N-terminal kinase (JNK), which in turn modulates, *inter alia*, transcription factors including c-Jun, ATF2, and ELK-1. JNK is described in U.S. Patents 5,534,426,5,593,884,5,605,808, and WO 95/03324, each of which is incorporated herein by reference in its entirety.

The MLK family includes, in part, the following groups: 1) multiple lineage kinase 1 (MLK1); 2) multiple lineage kinase 2 (MLK2); 3) multiple lineage kinase 3 (MLK3); 4) leucine zipper bearing kinase (LZK); 5) dual leucine zipper bearing kinase (DLK); and 6) multiple lineage kinase 6 (MLK6). MLK1 has a catalytic domain similar to both kinases specific for Tyr and Ser/Thr. Dorow, *et al*., *Eur. J*. *Biochem*., **1993**, *213*, 701-710. MLK2 also has a catalytic domain similar to both kinases specific for Tyr or Ser/Thr. Dorow, *et al*., *Eur. J*. *Biochem.,* **1993**, 213, 701-710. NILK2 is also known as MST. Katoh, *et al*., *Oncogene,* **1995,** *10,* 1447-1451. MLK3 comprises a protein that, in addition to the kinase domain, contains two leucine zippers with an adjacent carboxy-terminal basic region, and a proline rich region. Ing, *et al*., *Oncogene,* **1994**, *9*, 1745-1750. MLK3 is also known as SPRK (Gallo, *et al*., *J Biol*. *Chem*., **1994,** *269,* 15092-15100), and PTK1 (Ezoe, *et al*., *Oncogene*, **1994**, *9*, 935-938). LZK is a leucine zipper bearing kinase. Sakuma, *et al*., *J*. *Biol*. *Chem*., **1997,** *272,* 28622-28629. DLK has a kinase domain and two putative leucine zipper motifs. Holzman, *et al*., *J*. *Biol*. *Chem.,* **1994,** *269*, 30808-30817. DLK is also known as ZPK (Reddy, *et al*., *Biochem. Biophys. Res. Comm*., **1994,** *202*, 613-620) and MUK(Hirai, *et al*., *Oncogene*, **1996,** *12*, 641-650). Members of the MLK family are also described in, for example, U.S. Patents 5,676,945, 5,554,523, WO 93/15201, Canadian Patent 2,148,898, Diener, *et al*., *Proc. Natl*. *Acad*. *Sci*. *USA,* **1997,** *94*, 9687-9692, DeAizpurua, *et al*., *J*. *Biol*. *Chem*., **1997**, *272*, 16364-16373, *Tung, et al*., *Oncogene,* **1997,** *14*, 653-659, Sells, *et al*., *Trends in Cell Biol*., **1997,** 7, 161-167, Mata, *et al*., *J*. *Biol*. *Chem*., **1996,** *271,* 16888-16896, Hirai, *et al*., *J*. *Biol*. *Chem*., **1997,** *272,* 15167-15173, Fan, *et al*., *J*. *Biol*. *Chem*., **1996,** *271*, 24788-24793, Blouin, *et al*., *DNA and Cell Biol*., **1996,** *15*, 631-642, Pombo, *et al*., *Nature*, **1995,** *377*, 750-754, Kiefer, *et al*., *EMBO J*., **1996,** *15*, 7013-7025, Hu, *et al*., *Genes* & *Dev*., **1996,** *10*, 2251-2264, Su, *et al*., *EMBO J.,* **1997,** *16*, 1279-1290, and Dorow, *et al*., *Eur*. *J. Biochem*., **1995**, *234*, 492-500. Recently, another MLK-related kinase was identified in the EST database. The DNA sequence of this clone, MLK6, is described by seven overlapping entries. Their clone ID numbers are: 1007489, 1460085, 510915, 666323, F5555, 482188 and 178522, the sequences of each which are incorporated herein by reference in their entirety. Each of the references cited in the present paragraph is incorporated herein by reference in its entirety.

Recently, stable expression of ZPK has been shown to reduce the proliferative capacity of NIH 3T3 fibroblasts as measured by a colony formation assay. Bergeron, *et al*., *Biochem*. *Biophys. Res. Comm.,* **1997,** *231*, 153-155. Bergeron, *et al*., however, failed to provide any data showing that ZPK modulated the activity of a ZPK substrate or whether ZPK promoted cell death.

Expression of a construct encoding Myc-MLK2 in Swiss 3T3 cells has been shown to lead to apoptosis approximately 20 hours after injection. Nagata, *et al*., *EMBO J*., **1998**, *17*, 149-158.

Applicants have developed numerous indolo and indeno compounds which, *inter alia*, inhibit cell growth associated with hyperproliferative states and inhibit death in a variety of embryonic cultures, such as dorsal root ganglion, striatal, superior cervical ganglia and motoneurons. U.S. Patents 5,475,110, 5,591,855, 5,594,009, 5,461,146, 5,621,100, 5,621,101, 5,705,511, and 5,756,494, each of which is assigned to the assignee of the present application, and each of which is incorporated herein by reference in its entirety. Compounds recited in U.S. Patent 5,705,511 having formula G are referred to in the present application as having formula I. Applicants have also shown that motoneuron apoptosis is inhibited by a derivative of K-252a, an indolocarbazole which also modulates the stress-signaling cascade. Maroney, *et al*., *J. Neurosci.,* **1998,** *18,* 104-111, which is incorporated herein by reference in its entirety.

Due to the inadequacies of screening compounds which modulate members of the stress signaling cascade and promote either cell death or cell survival, there continues to be a need for new, selective methods of screening compounds. In addition, there continues to be a need for screening assays for therapeutics which may be useful in treating inflammation and neurodegenerative disorders. The present invention is directed to these, as well as other, important ends.

### Summary of the Invention

The present invention provides methods for identifying compounds which modulate activity of a multiple lineage kinase protein and promote cell survival comprising the steps of contacting the cell containing the multiple lineage kinase protein with the compound, determining whether the compound decreases activity of the multiple lineage kinase protein, and determining whether the compound promotes cell survival.

The present invention also provides methods for identifying compounds which modulate activity of a multiple lineage kinase protein and promote cell death comprising the steps of contacting the cell containing the multiple lineage kinase protein with the compound, determining whether the compound increases activity of the multiple lineage kinase protein, and determining whether the compound promotes cell death.

The present invention also provides methods for identifying compounds which may be useful in treating neurodegenerative disorders comprising contacting a cell or cell extract containing a multiple lineage kinase protein with the compound and determining whether the compound decreases activity of the multiple lineage kinase protein.

The present invention also provides methods for identifying compounds which may be useful in treating inflammation comprising contacting a cell or cell extract containing a multiple lineage kinase protein with the compound and determining whether the compound decreases activity of the multiple lineage kinase protein.

The present invention also provides methods for treating a mammal having or suspected of having a neurodegenerative disorder comprising administering to said mammal a compound which inhibits or reduces multiple lineage kinase protein activity.

The present invention also provides methods for treating a mammal having inflammation comprising administering to said mammal a compound which inhibits or reduces multiple lineage kinase protein activity.

The present invention also provides methods for modulating the activity of a multiple linage kinase protein comprising contacting the protein or a cell containing the protein with a compound having formula II: 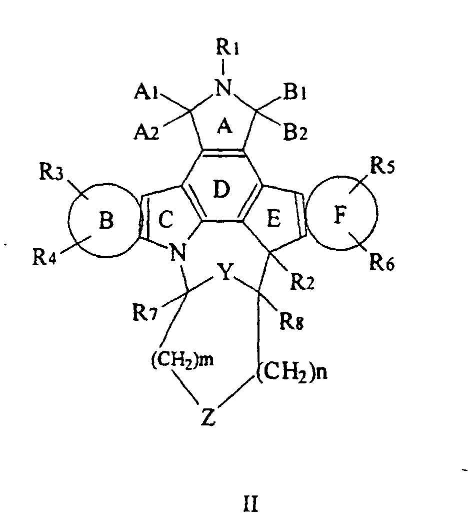 wherein:
ring B and ring F, independently, and each together with the carbon atoms to which they are attached, are selected from the group consisting of:
   an unsaturated 6-membered carbocyclic aromatic ring in which from 1 to 3 carbon atoms may be replaced by nitrogen atoms;
   an unsaturated 5-membered carbocyclic aromatic ring; and
   an unsaturated 5-membered carbocyclic aromatic ring in which either
      one carbon atom is replaced with an oxygen, nitrogen, or sulfur atom;
      two carbon atoms are replaced with a sulfur and a nitrogen atom, an oxygen and a nitrogen atom, or two nitrogen atoms; or
      three carbon atoms are replaced with three nitrogen atoms;
R¹ is selected from the group consisting of:
   H, substituted or unsubstituted alkyl having from 1 to 4 carbons, substituted or unsubstituted aryl, substituted or unsubstituted arylalkyl, substituted or unsubstituted heteroaryl, or substituted or unsubstituted heteroarylalkyl;
   -C(=O)R⁹, where R⁹ is selected from the group consisting of alkyl, aryl and heteroaryl;
   -OR¹⁰, where R¹⁰ is selected from the group consisting of H and alkyl having from 1 to 4 carbons;
   -C(=O)NH₂, -NR¹¹R¹², -(CH₂)ₚNR¹¹R¹², -(CH₂)ₚOR¹⁰, -O(CH₂)ₚOR₁₀ and -O(CH₂)ₚNR¹¹R¹², wherein p is from 1 to 4; and wherein either
      R¹¹ and R¹² are each independently selected from the group consisting ofH and alkyl having from 1 to 4 carbons; or
      R¹¹ and R¹² together form a linking group of the formula -(CH₂)₂-X¹-(CH₂)₂-, wherein X¹ is selected from the group consisting of -O-, -S-, and -CH₂-;
R² is selected from the group consisting of H, alkyl having from 1 to 4 carbons, -OH, alkoxy having from 1 to 4 carbons, -OC(=O)R⁹, -OC(=O)NR¹¹R¹², -O(CH₂)ₚNR¹¹R¹², -O(CH₂)ₚOR¹⁰, substituted or unsubstituted arylalkyl having from 6 to 10 carbons, and substituted or unsubstituted heteroarylalkyl;
R³, R⁴, R⁵ and R⁶ are each independently selected from the group consisting of:
   H, aryl, heteroaryl, F, Cl, Br, I, -CN, CF₃, -NO₂,-OH, -OR⁹, -O(CH₂)ₚNR¹¹R¹², -OC(=O)R⁹, -OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹; -CH₂OR¹⁴, wherein R¹⁴ is the residue of an amino acid after the hydroxyl group of the carboxyl group is removed;
   -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, or -CH=NNR²R^{2A} wherein R^{2A} is the same as R²;
   -S(O)_{y}R² -(CH₂)ₚS(O)_{y}R⁹, -CH₂S(O)_{y}R¹⁴ wherein y is 0,1 or 2;
   alkyl having from 1 to 8 carbons, alkenyl having from 2 to 8 carbons, and alkynyl having 2 to 8 carbons, wherein
      each alkyl, alkenyl, or alkynyl group is unsubstituted; or
      each alkyl, alkenyl, or alkynyl group is substituted with 1 to 3 groups selected from the group consisting of aryl having from 6 to 10 carbons, heteroaryl, arylalkoxy, heterocycloalkoxy, hydroxyalkoxy, alkyloxy-alkoxy, hydroxyalkylthio, alkoxy-alkylthio, F, Cl, Br, I, -CN, -NO₂, -OH, -OR⁹, -X²(CH₂)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR²,
      -O-tetrahydropyranyl, -NR¹¹R¹², -NR¹⁰C(=O)R⁹, -NR¹⁰CO₂R⁹, -NR¹⁰C(=O)NR¹¹R¹², -NHC(=NH)NH₂, NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴, and a monosaccharide having from 5 to 7 carbons wherein each hydroxyl group of the monosaccharide is independently either unsubstituted or is replaced by H, alkyl having from 1 to 4 carbons, alkylcarbonyloxy having from 2 to 5 carbons, or alkoxy having from of 1 to 4 carbons;
X² is O, S, or NR¹⁰;
R⁷ and R⁸ are each independently selected from the group consisting ofH, alkyl having from 1 to 4 carbons, alkoxy having from 1 to 4 carbons, substituted or unsubstituted arylalkyl having from 6 to 10 carbons, substituted or unsubstituted heteroarylalkyl, -(CH₂)ₚOR¹⁰, -(CH₂)ₚOC(=O)NR¹¹R¹², and -(CH₂)ₚNR¹¹R¹²; or R⁷ and R⁸ together form a linking group of the formula -CH₂-X³-CH₂-, wherein X³ is X² or a bond;
m and n are each independently 0, 1, or 2;
Y is selected from the group consisting of -O-, -S-, -N(R¹⁰)-, -N⁺(O⁻)(R¹⁰)-, -N(OR¹⁰)-, and -CH₂-;
Z is selected from the group consisting of a bond, -O-, -CH=CH-, -S-, -C(=O)-, -CH(OR¹⁰)-, -N(R¹⁰)-, -N(OR¹⁰)-, CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, and -CH(O(C=O)R⁹)CH(OC(=O)R^{9A})-, wherein R^{9A} is the same as R⁹;
R¹⁵ and R¹⁶ are independently selected from the group consisting of H, -OH, -C(=O)R¹⁰, -O(C=O)R⁹, hydroxyalkyl, and -CO₂R¹⁰;
R¹⁷ is selected from the group consisting of H, alkyl, aryl, and heteroaryl;
A¹ and A² are selected from the group consisting ofH, H; H, OR²; H, -SR²; H, -N(R²)₂; and a group wherein A¹ and A² together form a moiety selected from the group consisting of =O, =S, and =NR²;
B¹ and B² are selected from the group consisting of H, H; H, -OR²; H, -SR²; H, -N(R²)₂; and a group wherein B¹ and B² together form a moiety selected from the group consisting of =O, =S, and =NR²;
with the proviso that at least one of the pairs A¹ and A², or B¹ and B², form =O.

The present invention also provides methods for modulating the activity of a multiple linage kinase protein comprising contacting the protein or a cell containing the protein with a compound having formula III: 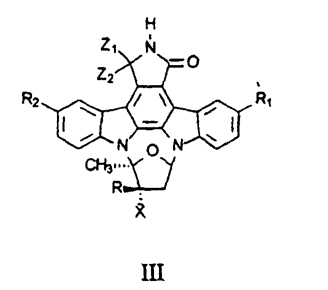 wherein
Z₁ is H and Z₂ is H or Z₁ and Z₂ together form =O;
R₁ is selected from the group consisting of H, Cl, CH₂SO₂C₂H₅, Br, CH₂S(CH₂)₂NH₂, CH₂S(CH₂)₂N(CH₃)₂, CH₂S(CH₂)₂NH₂ n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-propyl, CH=NNH-C(=NH)NH₂, CH=N-N(CH₃)₂, CH₂S(CH₂)₂NH-n-C₄H₉, CH₂OCH₂OCH₂CH₃,CH₂S[3-(1,2,4-triazine)], CH₂CH₂SCH₃; 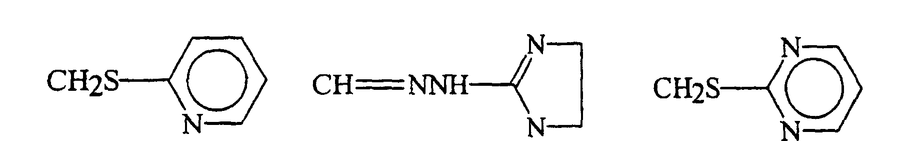 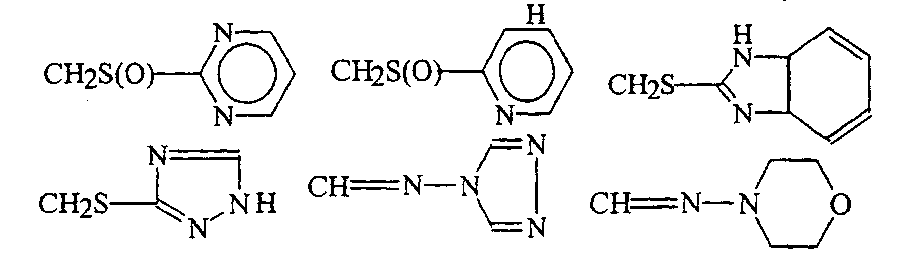 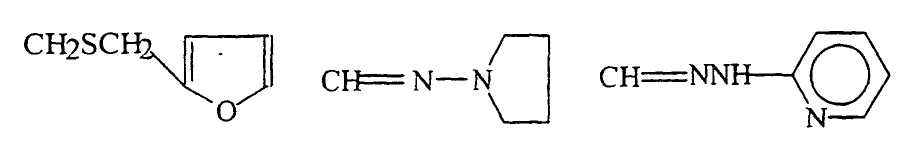 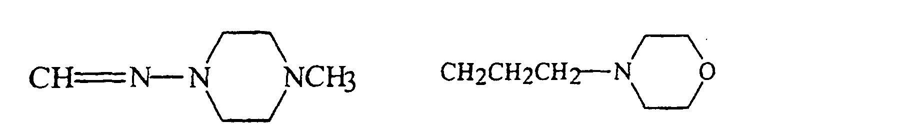 R₂ is selected from the group consisting of H, Br, Cl, I, CH₂S(CH₂)₂N(CH₃)₂, NHCONHC₂H₅, CH₂SC₂H₅, CH₂OCH₂OCH₂CH₃, CH₂S[3-(1,2,4-triazine)], CH₂CH₂SCH₃, and CH₂OH;
X is selected from the group consisting of H, CH₂OH, CH₂NH-SerineH, CO₂CH₃, CONHC₆H₅, CH₂NHCO₂C₆H₅, CH₂NHCO₂CH₃, CH₂N₃, CONHC₂H₅, CH₂NH-Glycine, CON(CH₃)₂, -CH₂NHCO₂-, CONH₂, CONHC₃H₇, CH₂NH-Serine, CH₂SOCH₃, CH=NOH, CH₂NH-Proline, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-hexyl, -CONHCH₃, and CO₂(CH₂)₄CH₃; or one of the following formulas 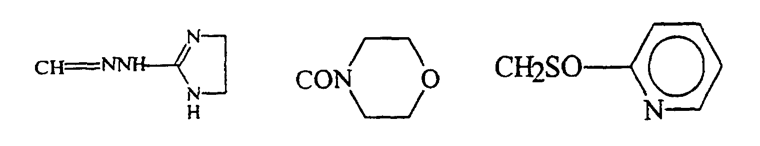 and
R is selected from the group consisting of OH, and OCH₃.

The present invention also provides methods of modulating the activity of a multiple linage kinase protein comprising contacting the protein or a cell containing the protein with a compound having formula IV: 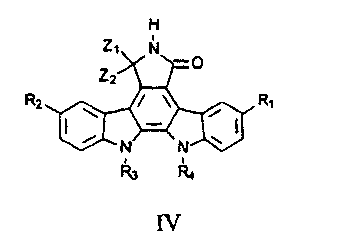 wherein
Z₁ is H and Z₂ is H or Z₁ and Z₂ together form =O;
R₁ is H or Br;
R₂ is H;
R₃ is H, CH₂CH=CH₂, CH₂CH₂CH₂OH, or
and
R₄ is H, CH₂CH=CH₂ or CH₂CH₂CH₂OH.

### Brief Description of the Drawings

For the purpose of illustrating embodiments of the present invention, there are shown in the drawings certain features. It should be understood, however, that this invention is not limited to the precise embodiments shown.
Figure **1** is a schematic drawing showing a general preparation of bridged indenopyrrolocarbazoles.
Figure **2** is a schematic drawing showing a general preparation of bridged indenopyrrolocarbazoles.
Figure **3** is a schematic drawing showing a preparation of resin-bound indenopyrrolocarbazoles.
Figure **4** is a schematic drawing showing the preparation of protected, soluble indenopyrro locarbazoles.
Figure **5** is a schematic drawing showing the preparation of intermediate **V**.
Figure **6** is a schematic drawing showing the preparation of bridged indenopyrrolocarbazoles using method **A**.
Figure **7** is a schematic drawing showing the preparation of bridged indenopyrrolocarbazoles using method **B.**
Figure **8** is a schematic drawing showing the preparation of **B** ring-substituted bridged indenopyrrolocarbazoles.
Figure **9** is a schematic drawing showing the derivatization of the **E** ring of bridged indenopyrrolocarbazoles.
Figure **10** shows a graph of two separate experiments depicting the amount of viable neuronally differentiated PC-12 cells remaining after 5 days of culturing in the absence of NGF. Results are expressed as percent of NGF control within each group (vector control in the absence of NGF, n=12; all other groups, n=3). The difference between vector control and stable pools of cells expressing a dominant negative MLK-3 mutant in the absence ofNGF is statistically significant as determined by a two-sided T-test (p<0.05).
Figure **11A** shows the phosphorylation of kinase-dead GST-SEK-1 by baculovirus-expressed FLAG-MLK-3 (mixture of full-length and kinase domain) using a radioactive gel-based assay.
Figure **11B** shows ³²P-labeted phosphorylated myelin basic protein product formed as a result of a kinase reaction catalyzed by baculovirus-expressed FLAG-MLK-3 (mixture of full-length and kinase domain) or GST-MLK-3 kinase domain.
Figure **12** is an immunoblot analysis showing the phosphorylation of kinase-dead GST-SEK-1 by baculovirus-expressed FLAG-MLK-3 (mixture of full-length and kinase domain) as detected by a phospho-specific SEK-1 antibody.
Figure **13** shows the phosphorylation of myelin basic protein by bacterially-expressed GST-MLK-3 kinase domain using the (o) multiscreen trichloroacetic acid precipitation assay, or the (•) phosphocellulose membrane method.
Figure **14** shows a saturation binding curve of [³H]K252a incubated with lysate of MLK-3 baculovirus infected insect cells.
Figure **15A** shows the amount of ³²P-labelled c-jun in an immunoprecipitation/kinase reaction from cells overexpressing MLK-3, MLK-2 or DLK and treated with either 0.025% DMSO (control) or 500 nM K-252a.
Figure **15B** shows a graph quantifying the percent activity remaining in immunoprecipitate/kinase reactions from samples described in FIG. 15A. Columns represent the average of duplicate samples where the error bar indicates the range of the mean.
Figure **15C** shows the amount of ³²P-labelled c-jun in an immunoprecipitation/kinase reaction from cells overexpressing HA-JNK1 alone or with MEKK1 at various amounts of cDNA as indicated and treated with either 0.025% DMSO (control) or 500 nM of Compound III-3 (see, Table 3). Columns represent the average of duplicate samples where the error bar indicates the range of the mean.
Figure **16** shows that Compound III-3 promotes neuronal survival in a concentration-dependent fashion. Dissociated neurons were cultured from sympathetic ganglia (SG) (**A**), dorsal root ganglia (DRG) (**B**), ciliary ganglia (CG) (**C**), and motoneurons (MN) (**D**), in the presence or absence of the indicated trophic factors. Cells were counted 48 h after plating as described in materials and methods. Data represent means ± SD of triplicate or quadruplicate determinations. Shown is one of three experiments.
Figure **17** shows phase-contrast micrographs of cultures of E 12 DRG **(A, E),** E9 sympathetic **(B, F),** E8 ciliary **(C, G)** and E5.5 motor neurons **(D, H)** after 48 h in culture (24 h for ciliary neurons) in the presence of the respective neurotrophic factor (20 ng/ml NGF for sympathetic and sensory neurons 10 ng/ml CNTF for ciliary neurons, 30 µg/ml muscle extract (MEX) for motoneurons **(A-D)** or in the presence of 1 µM Compound III-3 **(E-H).** Bar = 200 µm.
Figure **18** shows a photomicrograph of dorsal root ganglia explants in vitro. Explants from chick DRG (E9) were plated in 96-well plates medium containing 0.05% BSA. After a 2 h attachment period, additions were made: **(A)** control DMSO; **(B)** 20 ng/ml NGF; **(C)** 250 nM Compound III-3. Forty-eight h later, medium was removed and explants were fixed with 4% paraformaldehyde in phosphate-buffered saline.
Figure **19** shows the number of chick lumbar motor neurons surviving on E10 after daily treatment (E5-9) with specified doses of Compound III-3. Presented data are the mean ±S.D. of 5-6 animals/treatment group. The reported experiment was repeated two times. The data are from one representative experiment and represent one side of the lumbar column. *p<0.01, **p<0.001, Student t test between Compound III-3 and control groups with Bonferroni correction.
Figure **20** shows the number of motor neurons in the female rat spinal nucleus of the bulbocavernosus (SNB) surviving on PN10 or PN60 after daily treatment (PN1-5) with Compound III-3, or control vehicle (5% Solutol™). On PN10 (**A**, **B**) or PN 60 (**B**), rats were sacrificed and the region of the spinal cord containing the SNB was dissected and processed for histology; Cresylecht violet-stained motor neurons were then counted in serial section of the lumbar 5-sacral 1 region of the spinal cord as described previously (Wingfield, *et al*., *Steroids*, **1975,** *26,* 311-327). Experimental data are the means ± S.E.M. from 4-8 animals/treatment group.

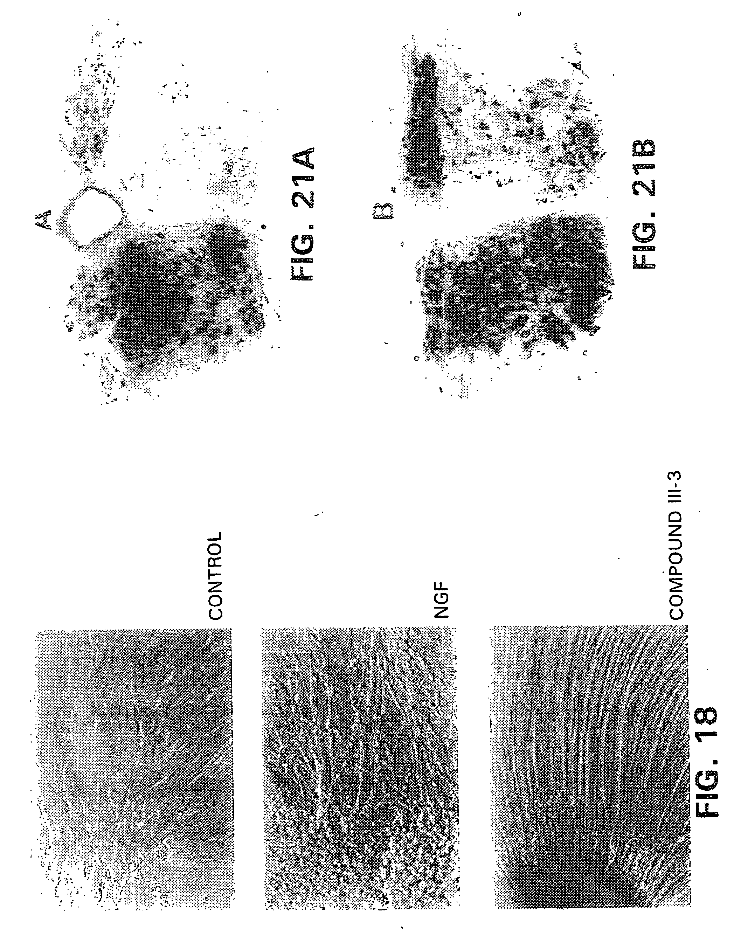

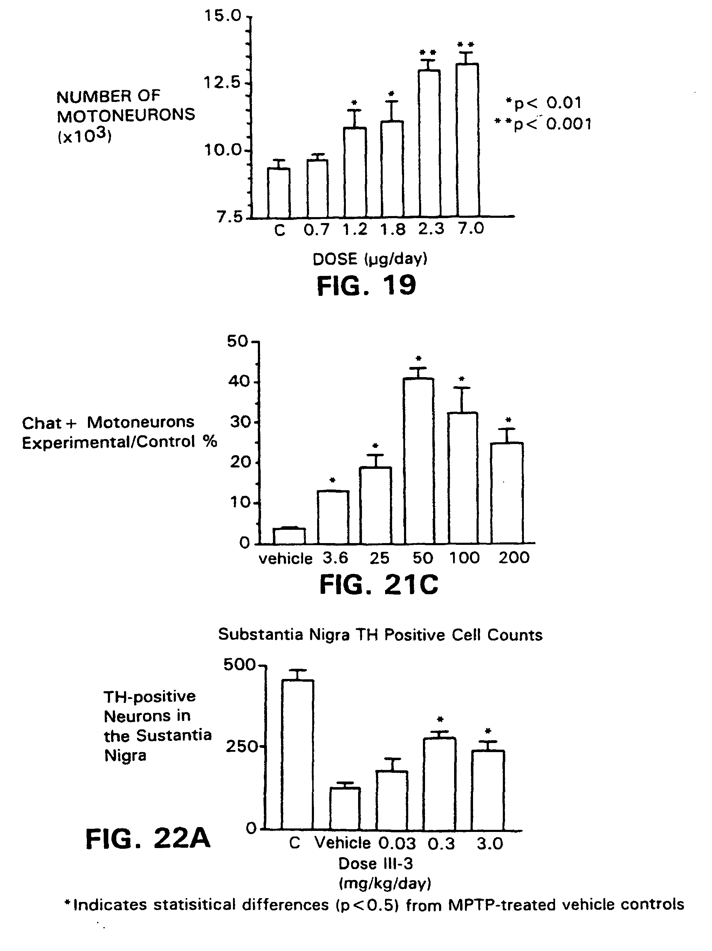

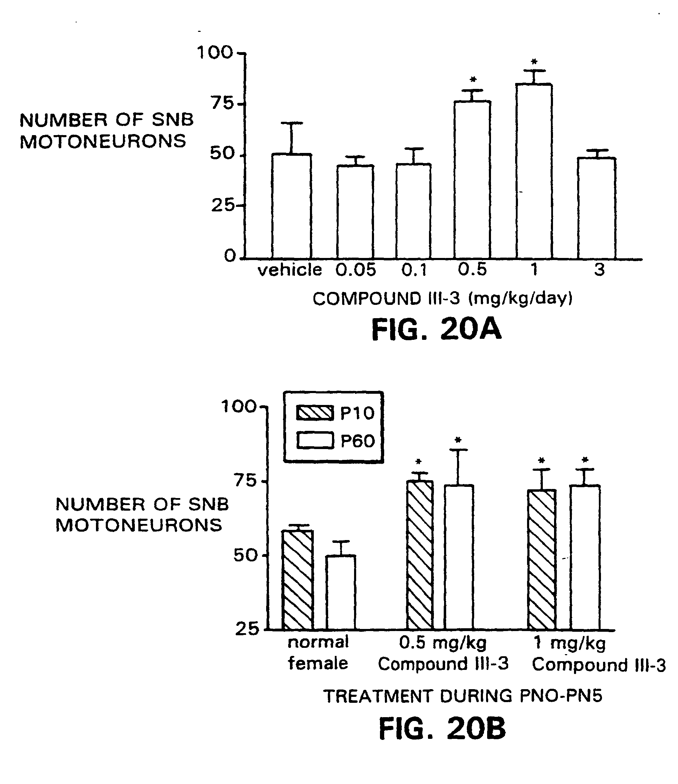

Figure **21** shows loss of CHAT immunoreactivity after hypoglossal axotomy in the adult rat after treatment with Compound III-3. Photomicrographs of the hypoglossal nucleus after transection of the hypoglossal nerve and treatment with **(A)** vehicle solution along (5% Solutol™) and **(B)** 200 µg of Compound III-3 applied at the site of the transection. **(C)** Number of ChAT-immunoreactive hypoglossal motor neurons after treatments described in (A) and (B) above. Results are expressed as the percentage of ChAT-immunoreactive motor neurons with 100% defined as that number of ChAT-immunoreactive motor neurons in the contralateral, unlesioned hypoglossal nucleus.
Figure **22** shows inhibition of the MLK-3 pathway demonstrates *in vivo* efficacy and blockage of phosphorylation events downstream. Figure **22A** shows increase of substantia nigra tyrosine hydroxylase immunoreactive neurons after MPTP lesion upon systematic administration of Compound III-3. Figure **22B** is a representative immunoblot showing MPTP induced increase in levels of phosphorylated MKK4. Figure **22C** depicts a representative immunoblot and ELISA showing attenuation of MPTP induced phosphorylated MKK4 in the presence of Compound III-3..
Figure **23** shows the induction of IL-2 in Jurkat cells. Figure **23A** shows the time course of IL-2 induction. Figure **23B** shows inhibition of IL-2 induction by Compound III-3. Figure **23C** shows inhibition of IL-2 induction by Compound III-3 and Compound I-4.

### Detailed Description of the Invention

As employed above and throughout the disclosure, the following terms, unless otherwise indicated, shall be understood to have the following meanings.

"Apoptosis" refers to a specific morphological form of cell death characterized by fragmentation of cells and their nuclei into membrane-bound particles. Apoptosis can be triggered by, for example, treatment with apoptosis-inducing compounds such as etoposide, staurosporine, tumor necrosis factor-α, ceramide, and the like, or by conditions such as x-irradiation.

The term "cell death" refers to death of cells by apoptosis, necrotic, or other means widely known to those skilled in the art. "Cell death" can be characterized, for example, as a decrease in total cell numbers of cells or a decrease in cell viability compared to untreated control populations of cells. Compounds which "promote cell death" result in a decrease in cell numbers or a decrease in cell viability as compared to control populations. In contrast, compounds which "promote cell survival" result in an increase in cell numbers or cell viability, or which slow or reduce the rate of cell death.

The terms "reacts selectively" or "binds specifically" describe compounds which physically or chemically interact directly with an MLK protein. In contrast, compounds which do not "react selectively" or "bind specifically" may effect proteins downstream or upstream of the MLK protein, and thus may effect the activity of MLK proteins, but do not physically or chemically interact directly with an MLK protein.

The term "modulates" refers to increasing or decreasing an activity of a particular protein or substrate thereof.

The present invention is directed, in part, to methods for identifying compounds which modulate activity of a MLK protein and promote either cell survival or cell death. Compounds which result in increased MLK protein activity may promote cell death, whereas compounds which result in decreased MLK protein activity may promote cell survival.

The MLK protein can be any protein identified as belonging to the MLK class of proteins. Preferably, the MLK protein is selected from the group consisting of MLK1, MLK2, MLK3 (SPRK, PTK1), LZK, DLK (ZPK, MUK), and MLK6 which are described above. In preferred embodiments of the invention, the methods identify compounds which directly interact or bind with the MLK protein as determined by binding assays, kinase assays, or other equivalent assays.

In order to identify compounds which modulate MLK protein activity and promote cell survival or cell death, a cell or cells containing the MLK protein is contacted with the test compound. The contacting can take place in buffers or media well known to those skilled in the art. In addition, varying numbers of cells and concentrations of test compounds can be used. Whether the test compound increases or decreases activity of the MLK protein is determined. In addition, whether the test compound promotes cell survival or cell death is also determined.

The cells which are contacted with the test compounds can be any mammalian cell. Preferably, the cell is a neuronal cell. Preferably, the cell is involved in a neurodegenerative disease. For purposes of the present invention, a "neurodegenerative disease," a "neurodegenerative disorder," and a "neurodegenerative condition" are interchangeable and are used to describe any disease or disorder involving neuronal cells or cells involved in the neuronal system, including, but not limited to, Alzheimer's disease, motor neuron disease, amyotrophic lateral sclerosis, Parkinson's disease, cerebrovascular disease, ischemic conditions, AIDS dementia, epilepsy, Huntington's disease, and concussive or penetrating injuries to the brain or spinal cord.

MLK protein activity can be determined by a number of techniques. For example, MLK activity can be determined by measuring the activity of a substrate ofthe MLK protein. Such substrates are well known and readily discernable to those skilled in the art. Preferably, the substrate is a member of the mitogen activated protein kinase kinase family or mitogen activated protein kinase family or substrates further down the pathway which includes, but is not limited to, a protein selected from the group consisting of JNK1, JNK2, JNK3, ERK1, ERK2, p38α, p38β, p38γ, p38δ, MEK1, MEK2, MKK3, MKK4 (SEK1), MEK5, MKK6, MKK7, jun, ATF2, ELK1, and the mammalian homolog of AEX-3, and also general substrates of Ser/Thr protein kinases such as myelin basic protein (MBP). Reagents and methods for measuring the activity of the substrates are also known to those skilled in the art. The presence of MLK can also be determined by measuring the amount of the MLK protein or mRNA encoding the MLK protein. Reagents, including antibodies and oligonucleotide probes, as well as methods of measuring the amount of DNA, or protein, including Northern and Western blots, are well known to those skilled in the art. MLK protein activity can also be determined by an *in vitro* kinase assay. *In vitro* kinase assays are well known to the skilled artisan. Other techniques for measuring protein activity are known to those skilled in the art and are intended to be covered by the present invention. Thus, one skilled in the art can determine whether the test compound modulates, *i*.*e*., increases or decreases, MLK protein activity.

Whether or not the test compound promotes cell survival or cell death can be determined in a number of ways. Preferably, promotion of cell survival or cell death is determined by using cells at risk of dying and comparing the amount of cells which were contacted with the test compound and remain alive with the amount of cells which were not contacted with the test compound and remain alive. Preferably, the cells are primary embryonic motoneuron cells which are pre-programmed to die. Primary embryonic motoneuron cells are described in Maroney, *et al*., *J*. *Neurosci*., **1998,** *18*, 104-111, which is incorporated herein by reference in its entirety. Primary embryonic motoneuron cells will die unless rescued by the test compound. Thus, a greater number of living motoneuron cells in the population of motoneuron cells treated with the test compound as compared to the number of motoneuron cells in the population of motoneuron cells which were not treated with the test compound is indicative of a test compound which promotes cell survival. In contrast, a lesser number of living motoneuron cells in the population ofmotoneuron cells treated with the test compound as compared to the number of living motoneuron cells in the population of motoneuron cells which were not treated with the test compound is indicative of a test compound which promotes cell death.

In another embodiment of the invention, normal cells, or wild-type cells, are converted to be cells at risk of dying by overexpressing the MLK protein, as described below in the Examples, and then contacted with the test compound. Cells overexpressing MLK proteins may die unless rescued by the test compound. Overexpression of MLK proteins can be accomplished using vectors capable of expressing the particular protein inside a cell. Expression vectors are well known to those skilled in the art. In addition, methods of preparing expression vectors are also well known to those skilled in the art. Expression vectors which express any of the MLK proteins can be prepared in a manner similar to those described in the Examples. A greater number of living cells in the population of overexpressing cells treated with the test compound as compared to the number of living cells in the population of overexpressing cells which were not treated with the test compound is indicative of a test compound which promotes cell survival. In contrast, a lesser number of living cells in the population of overexpressing cells treated with the test compound as compared to the number of living cells in the population of overexpressing cells which were not treated with the test compound is indicative of a test compound which promotes cell death.

In another embodiment of the invention, promotion of cell survival is determined by observing or measuring a decrease in apoptosis. Cytoplasmic shrinkage and nuclear condensation are associated with apoptosis. Thus, one skilled in the art can measure a decrease in apoptosis by measuring or observing a decrease in cytoplasmic shrinkage and/or nuclear condensation. In addition, one skilled in the art can measure apoptosis by employing conventional staining techniques.

In other embodiments of the invention. normal, wild-type neuronal cells can be used to identify compounds which promote cell death. Normal neuronal cells will survive unless they are induced to die by the test compound. A lesser number of living cells in the population of normal cells treated with the test compound as compared to the number of living cells in the population of normal cells which were not treated with the test compound is indicative of a test compound which promotes cell death. In contrast, a greater or equal number of living cells in the population of normal cells treated with the test compound as compared to the number of living cells in the population of normal cells which were not treated with the test compound is not indicative of a test compound which promotes cell death.

The present invention is also directed, in part, to methods for modulating the activity of an MLK protein comprising contacting the protein or a cell containing the protein with a compound having formula G (denoted formula I herein) set forth in U.S. Patent No. 5,705,511, which is assigned to the assignee of the present application and is incorporated herein by reference in its entirety.

The present invention is also directed, in part, to methods for modulating the activity of an MLK protein comprising contacting the protein or a cell containing the protein with a compound having formula III below: 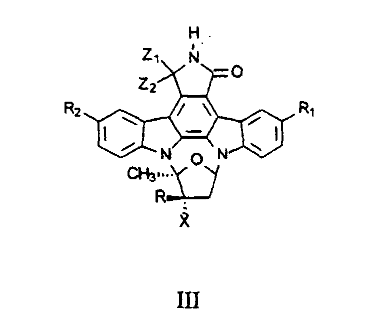 wherein:
Z₁ is H and Z₂ is H or Z₁ and Z₂ together form =O;
R₁ is selected from the group consisting of H, Cl, CH₂SO₂C₂H₅, Br, CH₂S(CH₂)₂NH₂, CH₂S(CH₂)₂N(CH₃)₂, CH₂S(CH₂)₂NH₂ n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O n-propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-triazine)], CH₂CH₂SCH₃, 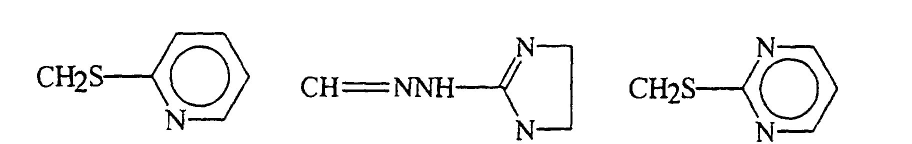 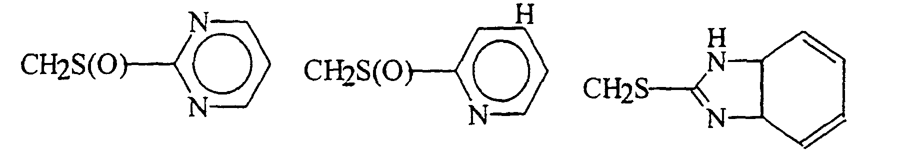 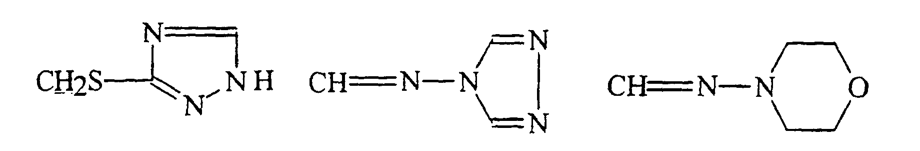 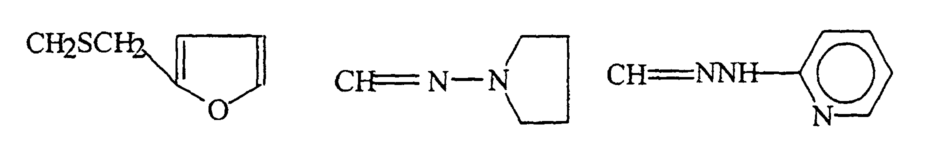 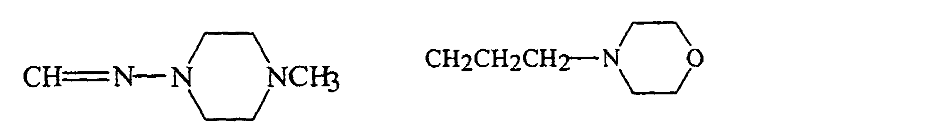
R₂ is selected from the group consisting ofH, Br, Cl, I, CH₂S(CH₂)₂N(CH₃)₂, NHCONHC₂H₅, CH₂SC₂H₅, CH₂OCH₂OCH₂CH₃, CH₂S[3-(1,2,4-triazine)], CH₂CH₂SCH₃, and CH₂OH;
X is selected from the group consisting of H, CH₂OH, CH₂NH-SerineH, CO₂CH₃, CONHC₆H₅, CH₂NHCO₂C₆H₅, CH₂NHCO₂CH₃, CH₂N₃, CONHC₂H₅, CH₂NH-Glycine, CON(CH₃)₂, -CH₂NHCO₂-, CONH₂, CONHC₃H₇, CH₂NH-Serine, CH₂SOCH₃, CH=NOH, CH₂NH-Proline, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-hexyl, CONHCH₃, and CO₂(CH₂)₄CH₃; or one of the following formulas 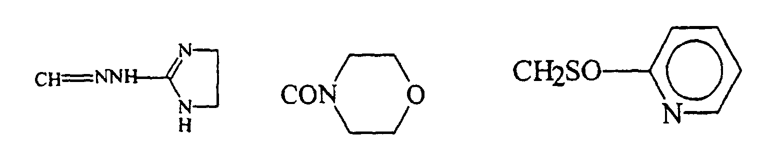 and
R is selected from the group consisting of OH, and OCH₃.

In preferred embodiments of the invention, Z, and Z₂ are H, X is CO₂CH₃, R₁ is NHCONHC₂H₅, R₂ is CH₂CH₂(2-Pyridyl), and R is OH. In other preferred embodiments of the invention, Z, and Z₂ are H, X is CO₂CH₃; R₁ and R₂ are CH₂OCH₂OCH₂CH₃, and R is OH; or Z₁ and Z₂ are H, X is CO₂CH₃, R₁ and R₂ are CH₂SCH₂CH₃, and R is OH; or Z₁, Z₂, R₁, and R₂ are H, X is CO₂CH₃; and R is OH; or Z₁, Z₂, R₁, and R₂ are H, X is CO₂(CH₂)₄CH₃, and R is OH; or Z₁, Z₂, and R₁, are H, R₂ is CH₂OH, X is CO₂CH₃, and R is OH; or Z₁, and Z₂ are H, R₁ and R₂ are H₂S[3-(1,2,4-triazine)], X is CO₂CH₃, and R is OH; or Z₁, and Z₂ are H, R₁ is Br, R₂ is I, X is CO₂CH₃; and R is OH; or Z₁, and Z₂ are H, R₁ and R₂ are CH₂CH₂SCH₃, X is CO₂CH₃, and R is OH; or Z₁, Z₂, R₁, and R₂ are H, X is CO₂CH₃, and R is OCH₃; or Z₁ and Z₂ together form =O, R₁ and R₂ are Br, X is CO₂CH₃, and R is OH.

The present invention is also directed, in part, to methods for modulating the activity of an MLK protein comprising contacting the protein or a cell containing the protein with a compound having formula II below: 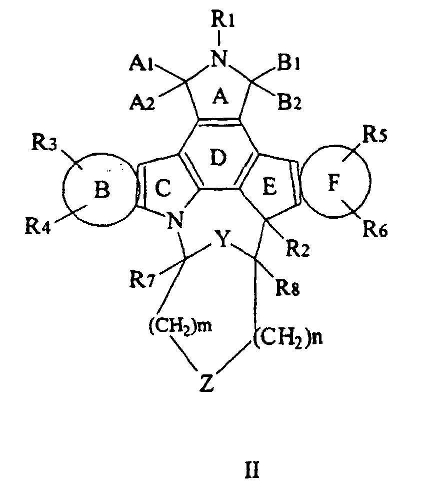 wherein:
ring B and ring F, independently, and each together with the carbon atoms to which they are attached, are selected from the group consisting of:
   a) an unsaturated 6-membered carbocyclic aromatic ring in which from 1 to 3 carbon atoms may be replaced by nitrogen atoms;
   b) an unsaturated 5-membered carbocyclic aromatic ring; and
   c) an unsaturated 5-membered carbocyclic aromatic ring in which either
      1) one carbon atom is replaced with an oxygen, nitrogen, or sulfur atom;
      2) two carbon atoms are replaced with a sulfur and a nitrogen atom, an oxygen and a nitrogen atom, or two nitrogen atoms; or
      3) three carbon atoms are replaced with three nitrogen atoms;
R¹ is selected from the group consisting of:
   a) H, substituted or unsubstituted alkyl having from 1 to 4 carbons, substituted or unsubstituted aryl, substituted or unsubstituted arylalkyl, substituted or unsubstituted heteroaryl, or substituted or unsubstituted heteroarylalkyl;
   b) -C(=O)R⁹, where R⁹ is selected from the group consisting of alkyl, aryl and heteroaryl;
   c) -OR¹⁰, where R¹⁰ is selected from the group consisting ofH and alkyl having from 1 to 4 carbons;
   d) -C(=O)NH₂, -NR¹¹R¹², -(CH₂)ₚNR¹¹R¹²,-(CH₂)ₚOR¹⁰, -(CH₂)ₚOR¹⁰ and -O(CH₂)ₚNR¹¹R¹², wherein p is from 1 to 4; and wherein either
      1) R¹¹ and R¹² are each independently selected from the group consisting ofH and alkyl having from 1 to 4 carbons; or
      2) R¹¹ and R¹² together form a linking group of the formula -(CH₂)₂-X¹-(CH₂)₂-, wherein X¹ is selected from the group consisting of -O-, -S-, and -CH₂-;
R² is selected from the group consisting ofH, alkyl having from 1 to 4 carbons, -OH, alkoxy having from 1 to 4 carbons, -OC(=O)R⁹, -OC(=O)NR¹¹R¹², -O(CH₂)ₚNR¹¹R¹², -O(CH₂)ₚOR¹⁰, substituted or unsubstituted arylalkyl having from 6 to 10 carbons, and substituted or unsubstituted heteroarylalkyl;
R³, R⁴, R⁵ and R⁶ are each independently selected from the group consisting of:
   a) H, aryl, heteroaryl, F, Cl, Br, I, -CN, CF₃, -NO₂,-OH, -OR⁹, -O(CH₂)ₚNR¹¹R¹², -OC(=O)R⁹,-OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹;
   b) -CH₂OR¹⁴, wherein R¹⁴ is the residue of an amino acid after the hydroxyl group of the carboxyl group is removed;
   c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, or -CH=NNR²R^{2A} wherein R^{2A} is the same as R²;
   d) -S(O)_{y}R² -(CH₂)ₚS(O)_{y}R⁹, -CH₂S(O)_{y}R¹⁴ wherein y is 0,1 or 2;
   e) alkyl having from 1 to 8 carbons, alkenyl having from 2 to 8 carbons, and alkynyl having 2 to 8 carbons, wherein
      1) each alkyl, alkenyl, or alkynyl group is unsubstituted; or
      2) each alkyl, alkenyl, or alkynyl group is substituted with 1 to 3 groups selected from the group consisting of aryl having from 6 to 10 carbons, heteroaryl, arylalkoxy, heterocycloalkoxy, hydroxyalkoxy, alkyloxy-alkoxy, hydroxyalkylthio, alkoxy-alkylthio, F, Cl, Br, I, -CN, -NO₂, -OH, -OR⁹, -X²(CH₂)ₚNR¹¹R¹², -X²(CH₂)ₚC(=O)NR¹¹R¹², -X²(CH₂)ₚOC(=O)NR¹¹R¹², -X²(CH₂)ₚCO₂R⁹,
         -X²(CH₂)ₚS(O)_{y}R⁹, -X²(CH₂)ₚNR¹⁰C(=O)NR¹¹R¹², -OC(=O)R⁹, -OCONHR², -O-tetrahydropyranyl, -NR¹¹R¹², -NR¹⁰C(=O)R⁹, -NR¹⁰CO₂R⁹, -NR¹⁰C(=O)NR¹¹R¹², -NHC(=NH)NH₂,
         -NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴, and a monosaccharide having from 5 to 7 carbons wherein each hydroxyl group of the monosaccharide is independently either unsubstituted or is replaced by H, alkyl having from 1 to 4 carbons, alkylcarbonyloxy having from 2 to 5 carbons, or alkoxy having from of 1 to 4 carbons;
X² is O, S, or NR¹⁰;
R⁷ and R⁸ are each independently selected from the group consisting of H, alkyl having from 1 to 4 carbons, alkoxy having from 1 to 4 carbons, substituted or unsubstituted arylalkyl having from 6 to 10 carbons, substituted or unsubstituted heteroarylalkyl, -(CH₂)ₚOR¹⁰, -(CH₂)ₚOC(=O)NR¹¹R¹², and -(CH₂)ₚNR¹¹R¹²; or R⁷ and R⁸ together form a linking group of the formula -CH₂-X³-CH₂-, wherein X³ is X² or a bond;
m and n are each independently 0, 1, or 2;
Y is selected from the group consisting of -O-, -S-, -N(R¹⁰)-, -N⁺(O⁻)(R¹⁰)-, -N(OR¹⁰)-, and -CH₂-;
Z is selected from the group consisting of a bond, -O-, -CH=CH-, -S-, -C(=O)-, -CH(OR¹⁰)-, -N(R¹⁰)-, -N(OR¹⁰)-, CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, and -CH(O(C=O)R⁹)CH(OC(=O)R^{9A})-, wherein R^{9A} is the same as R⁹;
R¹⁵ and R¹⁶ are independently selected from the group consisting of H, -OH, -C(=O)R¹⁰, -O(C=O)R⁹, hydroxyalkyl, and -CO₂R¹⁰;
R¹⁷ is selected from the group consisting of H, alkyl, aryl, and heteroaryl;
A¹ and A² are selected from the group consisting ofH, H; H, OR²; H, -SR²; H, -N(R²)₂; and a group wherein A¹ and A² together form a moiety selected from the group consisting of =O, =S, and =NR²;
B¹ and B² are selected from the group consisting of H, H; H, -OR²; H, -SR²; H, -N(R²)₂; and a group wherein B¹ and B² together form a moiety selected from the group consisting of =O, =S, and =NR²;
with the proviso that at least one of the pairs A¹ and A², or B¹ and B², form =O.

The present invention is also directed, in part, to methods for modulating the activity of an MLK protein comprising contacting the protein or a cell containing the protein with a compound having formula IV below: 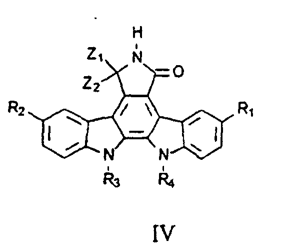 wherein:
Z₁ is H and Z₂ is H or Z₁ and Z₂ together form =O;
R₁ is H or Br;
R₂ is H;
R₃ is H, CH₂CH=CH₂, CH₂CH₂CH₂OH, or 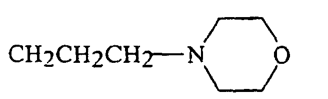 and
R₄ is H, CH₂CH=CH₂ or CH₂CH₂CH₂OH.

In preferred embodiments of the invention, R₁, R₂, R₄, Z₁, and Z₂ are H and R₃ is CH₂CH=CH₂. In other preferred embodiments of the invention, R₁ is Br and R₂, R₃ R₄, Z₁, and Z₂ are H; or R₁, R₂, Z₁, and Z₂ are H and R₃ and R₄ are CH₂CH=CH₂; or R₁, R₂, R₃, Z₁, and Z₂ are H and R₄ is CH₂CH=CH₂; or R₁, R₂, Z₁, and Z₂ are H, and R₃ and R₄ are CH₂CH₂CH₂OH; or R₁, R₂, R₄, Z₁, and Z₂ are H, and R₃ is

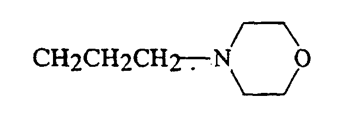

The present invention also provides methods for identifying compounds which may be useful in treating neurodegenerative disorders comprising contacting a cell or cell extract containing a multiple lineage kinase protein with the compound and determining whether the compound decreases activity of the multiple lineage kinase protein. The cells, and extracts therefrom, include those described above. Compounds which are found by the present methods (*i*.*e*., those compounds which inhibit or reduce the activity of a multiple lineage kinase protein) may be useful to treat neurodegenerative disorders. The protein is preferably selected from the group consisting of multiple lineage kinase 1, multiple lineage kinase 2, multiple lineage kinase 3, leucine zipper bearing kinase, dual leucine zipper bearing kinase, and multiple lineage kinase 6. The cell is contacted *in vitro*. Preferably, the protein activity is determined by measuring the activity or phosphorylation state of a substrate ofsaid protein. Preferably, the substrate is selected from the group consisting of JNK1, JNK2, JNK3, ERK1, ERK2, p38α, p38β, p38γ, p38δ, MEK1, MEK2, MKK3, MKK4 (SEK1), MEK5, MKK6, MKK7, jun, ATF2, ELK1, and the mammalian homolog of AEX-3, as well as general Ser/Thr substrates, such as, for example, myelin basic protein (MBP). The protein activity may also be determined by measuring the activity of a substrate o fthe protein, amount of a substrate of the protein, or mRNA encoding the substrate of the protein. Protein activity may also be determined by an *in vitro* kinase assay or binding assay. Cells are preferably primary embryonic motoneuron cells, cells which overexpress a multiple lineage kinase protein, or a neuronal cell, but can be any cell or extract therefrom. Preferably, compounds which directly bind the multiple lineage kinase protein are identified, as described above.

The present invention also provides methods for identifying compounds which may be useful in treating inflammation comprising contacting a cell or cell extract containing a multiple lineage kinase protein with the compound and determining whether the compound decreases activity of the multiple lineage kinase protein. The cells, and extracts therefrom, include those described above. Compounds which are found by the present methods (*i.e.*, those compounds which inhibit or reduce the activity of a multiple lineage kinase protein) may be useful to treat inflammation. The protein is preferably selected from the group consisting of multiple lineage kinase 1, multiple lineage kinase 2, multiple lineage kinase 3, leucine zipper bearing kinase, dual leucine zipper bearing kinase, and multiple lineage kinase 6. The cell is contacted *in vitro*. Preferably, the protein activity is determined by measuring the activity or phosphorylation state of a substrate of said protein. Preferably, the substrate is selected from the group consisting of JNK1, JNK2, JNK3, ERK1, ERK2, p38α, p38β, p38γ, p38δ, MEK1, MEK2, MKK3, MKK4 (SEK1), MEK5, MKK6, MKK7, jun, ATF2, ELK1, and the mammalian homolog of AEX-3, as well as general Ser/Thr substrates, such as, for example, myelin basic protein (MBP). The protein activity may also be determined by measuring the activity of a substrate of the protein, amount of a substrate of the protein, or mRNA encoding the substrate of the protein. Protein activity may also be determined by an *in vitro* kinase assay or binding assay.

Cells are preferably primary embryonic motoneuron cells, cells which overexpress a multiple lineage kinase protein, or a neuronal cell, but can be any cell or extract therefrom. Cells also include, but are not limited to, those involved in inflammation such as, for example, lymphocytes, macrophages and other white blood cells well known to those skilled in the art. Preferably, compounds which directly bind the multiple lineage kinase protein are identified.

The present invention also provides methods for treating a mammal having or suspected of having a neurodegenerative disorder comprising administering to the mammal a compound which inhibits or reduces multiple lineage kinase protein activity. A compound which inhibits or reduces multiple lineage kinase protein activity includes, but is not limited to, compounds having formula I, II, III, and IV. Preferred compounds include those described above with respect to the method for screening compounds which modulate the activity of a multiple lineage kinase protein and either promote cell survival or cell death. A preferred mammal is a human. An individual may be suspected of having a neurodegenerative disease if the individual has symptoms of a particular neurodegenerative disease, is in a high-risk group, or has a family history of a neurodegenerative disease.

The present invention also provides methods for treating a mammal having inflammation comprising administering to said mammal a compound which inhibits or reduces multiple lineage kinase protein activity. A compound which inhibits or reduces multiple lineage kinase protein activity includes, but is not limited to, compounds having formula I, II, III, and IV. Preferred compounds include those described above with respect to the method for screening compounds which modulate the activity of a multiple lineage kinase protein and either promote cell survival or cell death. A preferred mammal is a human.

The contacting with compounds having formulas I-IV can take place in buffers or media, which are well known to those skilled in the art. Alternately, the contacting can take place by administration of a pharmaceutical composition containing the test compound and a pharmaceutically acceptable salt, carrier, or diluent to a suitable animal or mammal, such as, for example, a mouse or other suitable animal known to those skilled in the art. In addition, varying numbers of cells and concentrations of compounds can be used. The cells which are contacted with the test compounds can be any mammalian cell. Preferably, the cell is a neuronal cell. Preferably, the cell is involved in a neurodegenerative disease, such as, for example, Alzheimer's disease, motor neuron disease, amyotrophic lateral sclerosis, Parkinson's disease, cerebrovascular disease, ischemic conditions, AIDS dementia, epilepsy, Huntington's disease, and concussive or penetrating injuries to the brain or spinal cord. Compounds having formula I, and methods of making the same, are described in U.S. Patent 5,705,511, which is incorporated herein by reference in its entirety. Compounds having formula III, and methods of making the same, are described in U.S. Patents 5,741,808, 5, 621,100, 5,621,101, 5,461,146, and 5,756,494, and WO 97/46567, each of which is incorporated herein by reference in its entirety. Compounds having formula IV, and methods of making the same, are described in U.S. Patents 5,741,808, 5, 621,100, 5,621,101, 5,461,146, and 5,756,494, and WO 97/46567, each of which is incorporated herein by reference in its entirety.

Compounds having formula II include diasteriomers and enantiomers around the carbon atoms to which the substituents R², R⁷, and R⁸ are attached.

Preferred bridged indenopyrrolocarbazoles are represented by formula II:

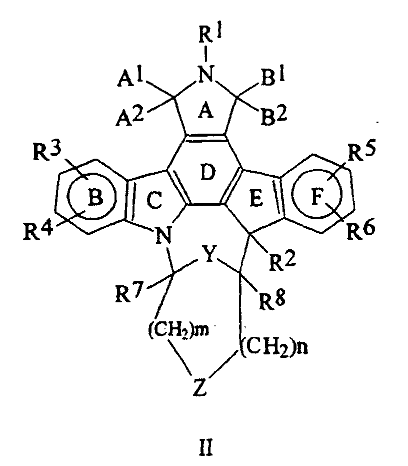

In some preferred embodiments of the compounds of formula II, R¹ is H. In further preferred embodiments, R², is H, hydroxyl, or substituted or unsubstituted alkyl.

In other preferred embodiments, R³, R⁴, R⁵, and R⁶ are independently H, substituted or unsubstituted alkyl, halogen, substituted or unsubstituted alkoxy, substituted or unsubstituted amino, or substituted or unsubstituted aryl. In further preferred embodiments, R⁷ and R⁸ are independently H, or substituted or unsubstituted alkyl.

In some preferred embodiments, Y is O. In further preferred embodiments Z is a bond, O, S, or substituted or unsubstituted N. In still further preferred embodiments, m and n are independently 1 or 2. In some especially preferred embodiments, Y is O, Z is a bond or O, and m and n are independently 1 or 2.

In further preferred embodiments, A¹A² and B¹B² are =O or H,H.

In some especially preferred embodiments, R¹, R⁴, R⁶, and R⁷ are each H, Y is =O, n is 1, A¹A² and B¹B² are =O or H,H, R² is H, OH or lower alkyl, R³ is H or substituted alkyl, R⁵ and R⁸ are each H or alkoxy, with methoxy being preferred, Z is a bond or O, and m is 1 or 2.

Some especially preferred embodiments of the compounds of formula II are compounds II-1, II-2, II-3, II-4a, II-4b, II-5, II-6, II-7a, II-7b, II-8, II-9, II-10, II-11, and II-12 set forth in Table 1, *infra*.

The compounds represented by formula II are hereinafter referred to as Compound (II).

As used herein, the term "carbocyclic" refers to cyclic groups in which the ring portion is composed solely of carbon atoms. The terms "heterocyclo" and "heterocyclic" refer to cyclic groups in which the ring portion includes at least one heteroatom such as O, N, or S.

As used herein, the term "alkyl" means a straight-chain, cyclic, or branched alkyl group having 1 to 8 carbon atoms, such as methyl, ethyl, propyl, isopropyl, butyl, isobutyl, sec-butyl, tert-butyl, pentyl, isoamyl, neopentyl, 1-ethylpropyl, hexyl, octyl, cyclopropyl, and cyclopentyl. The alkyl moiety of alkyl-containing groups, such as alkoxy, alkoxycarbonyl, and alkylaminocarbonyl groups, has the same meaning as alkyl defined above. Lower alkyl groups, which are preferred, are alkyl groups as defined above which contain I to 4 carbons. The term "alkenyl" is intended to include straight-chain or branched hydrocarbon chains having at least one carbon-carbon double bond. Examples of alkenyl groups include ethenyl and propenyl groups. As used herein, the term "alkynyl" is intended to include straight-chain or branched hydrocarbon chains having at least one carbon-carbon triple bond. Examples of alkynyl groups include ethynyl and propynyl groups.

The acyl moiety of acyl-containing groups such as acyloxy groups is intended to include a straight-chain or branched alkanoyl group having 1 to 6 carbon atoms, such as formyl, acetyl, propanoyl, butyryl, valeryl, pivaloyl or hexanoyl.

As used herein the term "aryl" means a group having 6 to 12 carbon atoms such as phenyl, biphenyl and naphthyl. Preferred aryl groups include unsubstituted or substituted phenyl and naphthyl groups. The term "heteroaryl" as used herein denotes an aryl group in which one or more ring carbon atom is replaced by a hetero (i.e., non-carbon) atom such as O, N or S. Preferred heteroaryl groups include pyridyl, pyrimidyl, pyrrolyl, furyl, thienyl, imidazolyl, triazolyl, tetrazolyl, quinolyl, isoquinolyl, benzoimidazolyl, thiazolyl, pyrazolyl, and benzothiazolyl groups.

The term "aralkyl" (or "arylalkyl") is intended to denotes a group having from 7 to 15 carbons, consisting of an alkyl group that bears an aryl group. Examples of aralkyl groups include benzyl, phenethyl, benzhydryl and naphthylmethyl groups.

Alkyl groups and alkyl moieties contained within substituent groups such as aralkyl, alkoxy, arylalkoxy, hydroxyalkoxy, alkoxy-alkoxy, hydroxy-alkylthio, alkoxy-alkylthio, alkylcarbonyloxy, hydroxyalkyl and acyloxy groups may be substituted or unsubstituted. A substituted alkyl group has 1 to 3 independently-selected substituents, preferably hydroxy, lower alkoxy, lower alkoxy-alkoxy, substituted or unsubstituted arylalkoxy-lower alkoxy, substituted or unsubstituted heteroarylalkoxy-lower alkoxy, substituted or unsubstituted arylalkoxy, substituted or unsubstituted heterocycloalkoxy, halogen, carboxyl, lower alkoxycarbonyl, nitro, amino, mono- or di-lower alkylamino, dioxolane, dioxane, dithiolane, dithione, furan, lactone, or lactam.

Substituted aryl, substituted heteroaryl and substituted aralkyl groups each have 1 to 3 independently-selected substituents that are preferably lower alkyl, hydroxy, lower alkoxy, carboxy, lower alkoxycarbonyl, nitro, amino, mono- or di-lower alkylamino, and halogen.

Heterocyclic groups formed with a nitrogen atom include pyrrolidinyl, piperidinyl, piperidino, morpholinyl, morpholino, thiomorpholino, N-methylpiperazinyl, indolyl, isoindolyl, imidazole, imidazoline, oxazoline, oxazole, triazole, thiazoline, thiazole, pyrazole, pyrazolone, and triazole groups. Heterocyclic groups formed with an oxygen atom includes furan, tetrahydrofuran, pyran, and tetrahydropyran groups.

"Hydroxyalkyl" groups are alkyl groups that have a hydroxyl group appended thereto. Halogens include fluorine, chlorine, bromine and iodine.

As used herein, the term "heteroarylalkyl" means an arylaklyl group that contains a heteroatom. The term "oxy" denotes the presence of an oxygen atom. Thus, "alkoxy" groups are alkyl groups that are attached through an oxygen atom, and "carbonyloxy" groups are carbonyl groups that are attached through an oxygen atom.

The term "heterocycloalkoxy" means an alkoxy group that has a heterocyclo group attached to the alkyl moiety thereof, and the term "arylalkoxy" means an alkoxy group that has an aryl group attached to the alkyl moiety thereof. The term "alkylcarbonyloxy" means an group of formula -O-C(=O)-alkyl.

As used herein, the term "alkyloxy-alkoxy" denotes an alkoxy group that contains an alkyloxy substituent attached to its alkyl moiety. The term "alkoxy-alkylthio" means an alkylthio group (i.e., a group of formula -S-alkyl) that contains an alkoxy substituent attached to its alkyl moiety. The term "hydroxy-alkylthio" means an alkylthio group (i.e., a group of formula -S-alkyl) that contains a hydroxy substituent attached to its alkyl moiety.

As used herein, the term "monosaccharide" has its accustomed meaning as a simple sugar.

As used herein, the term "amino acid" denotes a molecule containing both an amino group and a carboxyl group. Embodiments of amino acids include α-amino acids; i.e., carboxylic acids of general formula HOOC-CH(NH₂)-(side chain).

Side chains of amino acids include naturally occurring and non-naturally occurring moieties. Non-naturally occurring (i.e., unnatural) amino acid side chains are moieties that are used in place of naturally occurring amino acid side chains in, for example, amino acid analogs. *See,* for example, Lehninger, *Biochemistry*, Second Edition, Worth Publishers, Inc, 1975, pages 73-75, incorporated by reference herein.

Preferred α-amino acids include glycine, alanine, proline, glutamic acid, and lysine, having the D configuration, the L configuration, or as a racemate.

The sidechains of further representative α-amino acids are shown below in Table 1.

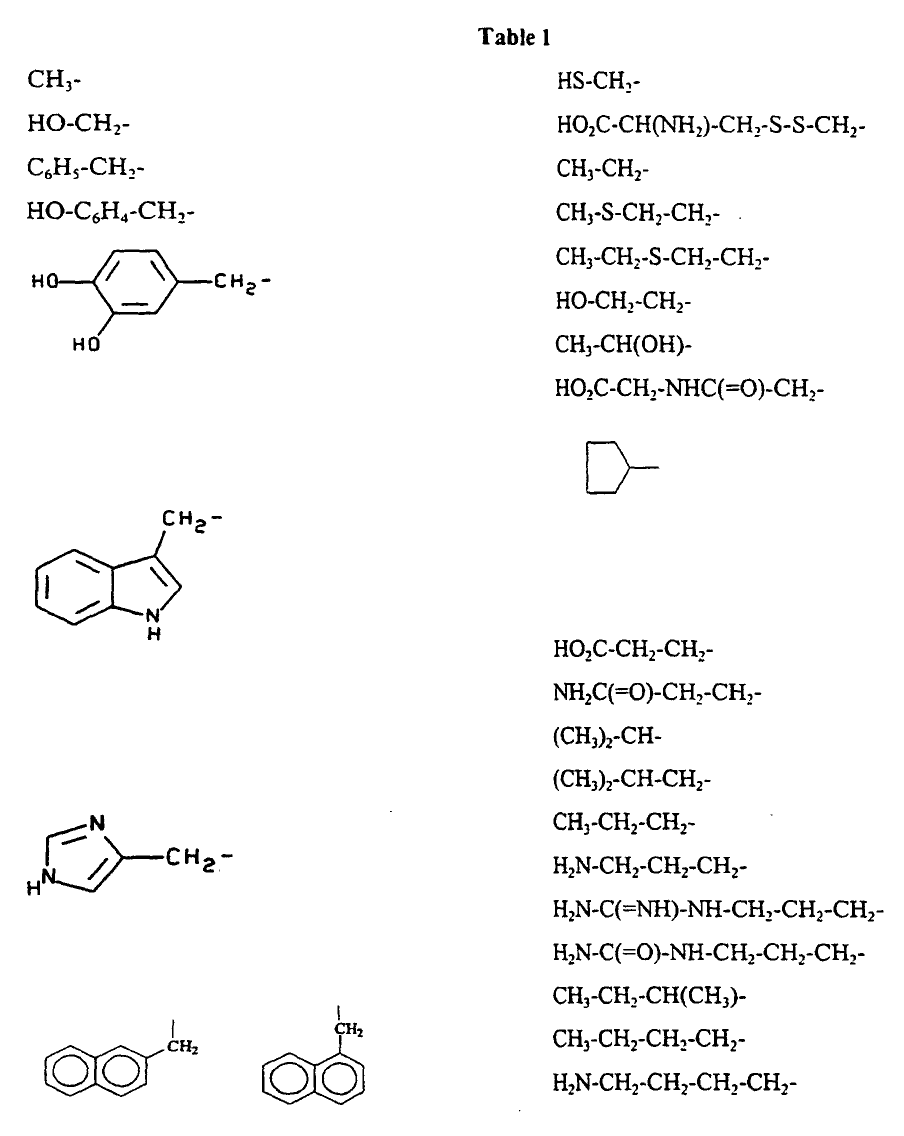

In some preferred embodiments, substituent groups for the compounds of formula II include the residue of an amino acid after removal of the hydroxyl moiety of the carboxyl group thereof; i.e., groups of formula -C(=O)-CH(NH₂)-(side chain).

Functional groups present on the compounds of formula II may contain protecting groups. For example, the amino acid sidechain substituents of the compounds of formula II can be substituted with protecting groups such as benzyloxycarbonyl or *t*-butoxycarbonyl groups. Protecting groups are known *per se* as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. One such protecting group is the benzyloxycarbonyl (Cbz; Z) group. Other preferred protecting groups according to the invention may be found in Greene, T.W. and Wuts, P.G.M., *"Protective Groups in Organic Synthesis"* 2d. Ed., Wiley & Sons, 1991.

The bridged indenopyrrolocarbazole compounds have evidenced important functional pharmacological activities which find utility in a variety of settings, including both research and therapeutic arenas. These derivatives are useful as therapeutic agents. The activities of the compounds show positive effects on the function and/or survival of trophic factor responsive cells. Effect on the function and/or survival of trophic factor responsive cells, e.g., cells of a neuronal lineage, has been demonstrated using any of the following assays: (1) cultured spinal cord choline acetyltransferase ("ChAT") assay; or (2) cultured basal forebrain neuron ChAT activity assay.

As used herein, the term "effect" when used to modify the terms "function" and "survival" means a positive or negative alteration or change. An effect which is positive can be referred to herein as an "enhancement" or "enhancing" and an effect which is negative can be referred to herein as "inhibition" or "inhibiting."

As used herein, the terms "enhance" or "enhancing" when used to modify the terms "function" or "survival" means that the presence of a bridged indenopyrrolocarbazole compound has a positive effect on the function and/or survival of a trophic factor responsive cell compared with a cell in the absence of the compound. For example, and not by way of limitation, with respect to the survival of, e.g., a cholinergic neuron, the compound would evidence enhancement of survival of a cholinergic neuronal population at risk of dying (due to, e.g., injury, a disease condition, a degenerative condition or natural progression) when compared to a cholinergic neuronal population not presented with such compound, if the treated population has a comparatively greater period of functionality than the non-treated population.

As used herein, "inhibit" and "inhibition" mean that a specified response of a designated material (e.g., enzymatic activity) is comparatively decreased in the presence of a bridged indenopyrrolocarbazole compound.

As used herein, the term "*trk*" refers to the family of high affinity neurotrophin receptors presently comprising *trkA, trk*B, and *trk*C, and other membrane associated proteins to which a neurotrophin can bind.

As used herein, inhibition of VEGFR implies utility in, for example, diseases where angiogenesis plays important roles, such as cancer of solid tumors, endometriosis, diabetic retinopathy, psoriasis, hemangioblastoma, as well as other ocular diseases and cancers.

Inhibition of trk implies utility in, for example, diseases of the prostate such as prostate cancer and benign prostate hyperplasia, and treatment of inflammatory pain.

Inhibition of Platelet Derived GrowthFactorReceptor(PDGFR) implies utility in, for example, various forms of neoplasia, rheumatoid arthritis, pulmonary fibrosis, myelofibrosis, abnormal wound healing, diseases with cardiovascular end points, such as atherosclerosis, restenosis, post-angioplasty restenosis, etc.

As used herein, the terms "cancer" and "cancerous" refer to any malignant proliferation of cells in a mammal. Examples include prostate, benign prostate hyperplasia, ovarian, breast, brain, lung, pancreatic, colorectal, gastric, stomach, solid tumors, head and neck, neuroblastoma, renal cell carcinoma, lymphoma, leukemia, other recognized malignancies of the hematopoietic systems, and other recognized cancers.

As used herein the terms "neuron," "cell of neuronal lineage" and "neuronal cell" include, but are not limited to, a heterogeneous population of neuronal types having singular or multiple transmitters and/or singular or multiple functions; preferably, these are cholinergic and sensory neurons. As used herein, the phrase "cholinergic neuron" means neurons of the Central Nervous System (CNS) and Peripheral Nervous System (PNS) whose neurotransmitter is acetylcholine; exemplary are basal forebrain, striatal, and spinal cord neurons. As used herein, the phrase "sensory neuron" includes neurons responsive to environmental cues (e.g., temperature, movement) from, e.g., skin, muscle and joints; exemplary is a neuron from the dorsal root ganglion.

A "trophic factor-responsive cell," as defined herein, is a cell which includes a receptor to which a trophic factor can specifically bind; examples include neurons (e.g., cholinergic and sensory neurons) and non-neuronal cells (e.g., monocytes and neoplastic cells).

The bridged indenopyrrolocarbazole compounds described herein find utility in both research and therapeutic settings in, for example, inhibition of enzymatic activity. For example, in a research environment, the compounds can be used in the development of assays and models for further enhancement of the understanding of the roles that inhibition of serine/threonine or tyrosine protein kinase (e.g., PKC, *trk* tyrosine kinase) play in the mechanistic aspects of the associated disorders and diseases. In a therapeutic setting, the compounds which inhibit these enzymatic activities can be used to inhibit the deleterious consequences of these enzymes with respect to disorders such as cancer.

As the Examp les below demonstrate, inhibition of enzymatic activity using the bridged indenopyrrolocarbazole compounds can be determined using, for example, the following assays:
1. *trkA* Tyrosine Kinase Activity inhibition assay;
2. Inhibition of NGF-stimulated trk phosphorylation in a whole cell preparation;
3. Vascular Endothelial Growth Factor Receptor (VEGFR) kinase inhibition assay;
4. PKC Activity inhibition assay;
5. PDGFR inhibition assay.

The disclosed bridged indenopyrrolocarbazole compounds can be used to enhance the function and/or survival of cells of neuronal lineage in a mammal, e.g., a human. In these contexts, the compounds can be utilized individually or with other fused pyrrolocarbazoles and/or indolocarbazoles, or in combination with other beneficial molecules which also evidence the ability to effect the function and/or survival of a designated cell.

A variety of neurological disorders are characterized by neuronal cells which are dying, injured, functionally compromised, undergoing axonal degeneration, at risk of dying, etc. These disorders include, but are not limited to: Alzheimer's disease; motor neuron disorders (e.g. amyotrophic lateral sclerosis); Parkinson's disease; cerebrovascular disorders (e.g., stroke, ischaemia); Huntington's disease; AIDS dementia; epilepsy; multiple sclerosis; peripheral neuropathies (e.g., those affecting DRG neurons in chemotherapy-associated peripheral neuropathy) including diabetic neuropathy; disorders induced by excitatory amino acids; and disorders associated with concussive or penetrating injuries of the brain or spinal cord.

ChAT catalyzes the synthesis of the neurotransmitter acetylcholine, and it is considered an enzymatic marker for a functional cholinergic neuron. A functional neuron is also capable of survival. Neuron survival is assayed by quantitation of the specific uptake and enzymatic conversion of a dye (e.g., calcein AM) by living neurons.

Because of their varied utilities, the compounds described herein, including those compounds identified by the methods described herein, find utility in a variety of settings. The compounds can be used in the development of *in vitro* models of neuronal cell survival, function, identification, or for the screening of other synthetic compounds which have activities similar to that ofthe compounds described herein, or compounds identified by the methods described herein. The compounds described herein, as well as those identified using the methods described herein, can be utilized in a research environment to investigate, define and determine molecular targets associated with functional responses. For example, by radiolabelling a bridged indenopyrrolocarbazole compound, or a compound identified by the methods described herein, associated with a specific cellular function (e.g., mitogenesis), the target entity to which the derivative binds can be identified, isolated, and purified for characterization.

The compounds, those described herein as well as those identified by using the methods described herein, are useful, *inter alia*, not only for enhancing trophic factor-induced activities of trophic responsive cells, e.g., cholinergic neurons, but also may function as survival promoting agents for other neuronal cell types, e.g., dopaminergic or glutamatergic. Growth factor may regulate survival of neurons by signaling cascades downstream of the small GTP binding proteins that include, but are not limited to, ras, rac, and cdc42 (Denhardt, *Biochem. J*., **1996,** *318,* 729). Specifically, activation of ras leads to phosphorylation and activation of extracellular receptor-activated kinase (ERK), which has been linked to biological growth and differentiation processes. Stimulation of rac/cdc42 leads to an increase in activation of JNK and p38, responses that are associated with stress, apoptosis, and inflammation. Although growth factor responses are primarily via the ERK pathway, affecting these latter processes may lead to alternative mechanisms of neuronal survival which may mimic growth factor enhancing survival properties (Xia et al., Science, 1995, 270, 1326). The compounds may also function as survival promoting agents for neuronal and non-neuronal cells by mechanisms related to, but also distinct from, growth factor mediated survival, for example, inhibition of the JNK and p38 pathways which may lead to survival by inhibition of apoptotic cell death processes.

The present compounds are useful in the treatment of disorders associated with decreased ChAT activity or the death, injury to spinal cord motoneurons, and also have utility in, for example, diseases associated with apoptotic cell death of the central and peripheral nervous system, immune system and in inflammatory diseases.

The compounds described herein may also find utility in the treatment of disease states involving malignant cell proliferation, such as many cancers.

The pharmaceutically acceptable salts of the compounds described herein, as well as those compounds identified by the present methods, include pharmaceutically acceptable acid addition salts, metal salts, ammonium salts, organic amine addition salts, and amino acid addition salts. Examples ofthe acid addition salts are inorganic acid addition salts such as hydrochloride, sulfate and phosphate, and organic acid addition salts such as acetate, maleate, fumarate, tartrate, citrate and lactate; examples ofthe metal salts are alkali metal salts such as lithium salt, sodium salt and potassium salt, alkaline earth metal salts such as magnesium salt and calcium salt, aluminum salt, and zinc salt; examples of the ammonium salts are ammonium salt and tetramethylammonium salt; examples of the organic amine addition salts are salts with morpholine and piperidine; and examples of the amino acid addition salts are salts with glycine, phenylalanine, glutamic acid and lysine.

Compounds provided herein, including those identified by the present methods, can be formulated into pharmaceutical compositions by admixture with pharmaceutically acceptable nontoxic excipients and carriers. Such compositions can be prepared for use in parenteral administration, particularly in the form of liquid solutions or suspensions; or oral administration, particularly in the form of tablets or capsules; or intranasally, particularly in the form of powders, nasal drops, or aerosols; or dermally, via, for example, trans-dermal patches.

The composition can be conveniently administered in unit dosage form and may be prepared by any of the methods well known in the pharmaceutical art, for example, as described in *Remington's Pharmaceutical Sciences* (Mack Pub. Co., Easton, PA, 1980). Formulations for parenteral administration may contain as common excipients sterile water or saline, polyalkylene glycols such as polyethylene glycol, oils and vegetable origin, hydrogenated naphthalenes and the like. In particular, biocompatible, biodegradable lactide polymer, lactide/glycolide copolymer, or polyoxyethylene-polyoxypropylene copolymers may be useful excipients to control the release of the active compounds. Other potentially useful parenteral delivery systems for these active compounds include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, a salicylate for rectal administration, or citric acid for vaginal administration. Formulations for trans-dermal patches are preferably lipophilic emulsions.

The compounds of this invention can be employed as the sole active agent in a pharmaceutical composition. Alternatively, they can be used in combination with other active ingredients, e.g., other growth factors which facilitate neuronal survival or axonal regeneration in diseases or disorders.

The compounds ofthe invention and pharmaceutically acceptable salts thereof can be administered orally or non-orally, e.g., as an ointment or an injection. The concentrations of the compounds of this invention in a therapeutic composition can vary. The concentration will depend upon factors such as the total dosage of the drug to be administered, the chemical characteristics (e.g., hydrophobicity) of the compounds employed, the route of administration, the age, body weight and symptoms of a patient, etc.. The compounds of this invention typically are provided in an aqueous physiological buffer solution containing about 0.1 to 10% w/v compound for parenteral administration. Typical dose ranges are from about 1 µg/kg to about 1 g/kg of body weight per day; a preferred dose range is from about 0.01 mg/kg to 100 mg/kg of body weight per day, and preferably about 0.1 to 20 mg/kg once to four times per day. A preferred dosage of drug to be administered is likely to depend on variables such as the type and extent of progression of the disease or disorder, the overall health status of the particular patient, the relative biological efficacy of the compound selected, and formulation of the compound excipient, and its route of administration.

The compounds of the invention, including test compound and compounds identified by the methods of the present invention, and pharmaceutically acceptable salts thereof can be administered alone, or in the form of various pharmaceutical compositions, according to the pharmacological activity and the purpose of administration. The pharmaceutical compositions in accordance with the present invention can be prepared by uniformly mixing an effective amount of a compound or a pharmaceutically acceptable salt thereof, as an active ingredient, with a pharmaceutically acceptable carrier. The carrier may take a wide range of forms according to the forms of composition suitable for administration. It is desired that such pharmaceutical compositions are prepared in a unit dose form suitable for oral or non-oral administration. The forms for non-oral administration include ointment and injection.

Tablets can be prepared using excipients such as lactose, glucose, sucrose, mannitol and methyl cellulose, disintegrating agents such as starch, sodium alginate, calcium carboxymethyl cellulose and crystalline cellulose, lubricants such as magnesium stearate and talc, binders such as gelatin, polyvinyl alcohol, polyvinyl pyrrolidone, hydroxypropyl cellulose and methyl cellulose, surfactants such as sucrose fatty acid ester and sorbitol fatty acid ester, and the like in a conventional manner. It is preferred that each tablet contains 15-300 mg of the active ingredient.

Granules can be prepared using excipients such as lactose and sucrose, disintegrating agents such as starch, binders such as gelatin, and the like in a conventional manner. Powders can be prepared using excipients such as lactose and mannitol, and the like in a conventional manner. Capsules can be prepared using gelatin, water, sucrose, gum arabic, sorbitol, glycerin, crystalline cellulose, magnesium stearate, talc, and the like in a conventional manner. It is preferred that each capsule contains 15-300 mg of the active ingredient.

Syrup preparations can be prepared using sugars such as sucrose, water, ethanol, and the like in a conventional manner.

Ointment can be prepared using ointment bases such as vaseline, liquid paraffin, lanolin and macrogol. emulsifiers such as sodium lauryl lactate, benzalkonium chloride, sorbitan mono-fatty acid ester, sodium carboxymethyl cellulose and gum arabic, and the like in a conventional manner.

Injectable preparations can be prepared using solvents such as water, physiological saline. vegetable oils (e.g., olive oil and peanut oil), ethyl oleate and propylene glycol, solubilizing agents such as sodium benzoate, sodium salicylate and urethane, isotonicity agents such as sodium chloride and glucose, preservatives such as phenol, cresol, p-hydroxybenzoic ester and chlorobutanol, antioxidants such as ascorbic acid and sodium pyrosulfite, and the like in a conventional manner.

The invention is further illustrated by way of the following examples which are intended to elucidate the invention. These examples are not intended, nor are they to be construed. as limiting the scope of the disclosure.

### EXAMPLES

### Example 1: General Description of the Synthetic Processes and Examples

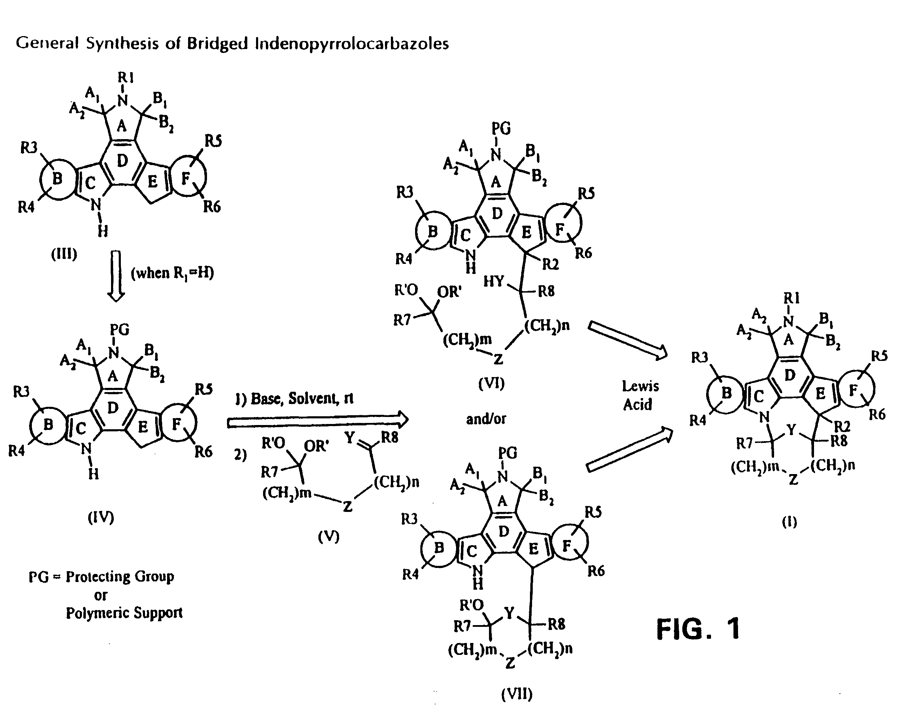

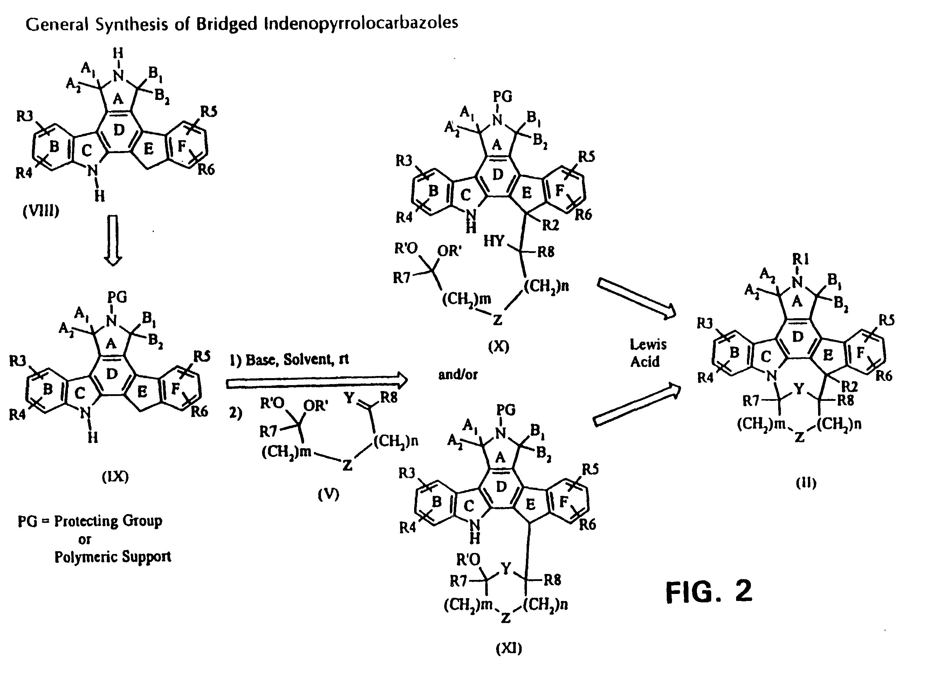

The general synthetic route employed to prepare the bridged indenopyrrolocarbazoles of this invention having formula II is shown in Figures 1 and 2. The general procedures for synthesis of the indenopyrrolocarbazoles (III)/(VIII) can be performed as described in U.S. Patent No. 5,705,511, the disclosure of which is hereby incorporated by reference in its entirety. When R¹ is H, the lactam nitrogen of the indenopyrrolocarbazoles (III)/(VIII) is protected with an appropriate protecting group leading to (IV)/(IX). The protected compounds are treated with an appropriate base in anhydrous organic solvent(s), which results in the generation of a dark red solution which is believed to be the carbanion. Reaction of the carbanion with a bi-functional reagent (V) results in an electrophilic addition to the C=Y bond of (V) leading to the initial intermediate (VI)/(X). Treatment of intermediate(s) (VI)(X) and /or (VII)/(XI) with either a sulphonic acid or a Lewis acid, e.g. boron trifluoride etherate, provides the bridged indenopyrrolocarbazoles (I)/(II).

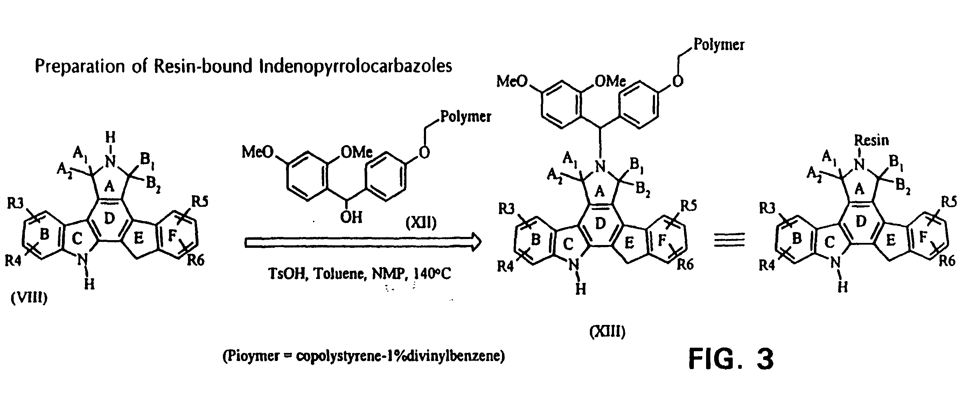

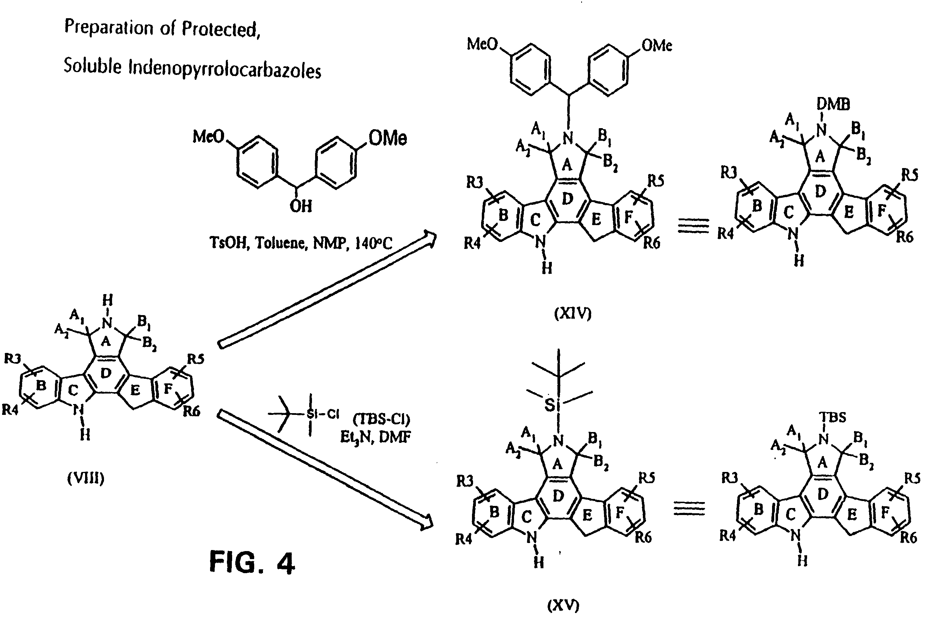

The lactam nitrogen protection strategy (shown in Figures 3 and 4) can be carried out by either an acid or a base-catalyzed process. The acid-catalyzed reaction can be carried out with a resin-bound reagent allowing immobilization of the indenopyrrolocarbazole (III)/(VIII) to a polymeric support, such as a polystyrene-based, Rink acid resin (XII) (Figure 3), providing (XIII). Alternatively, the acid-catalyzed reaction can be carried out with a soluble reagent to yield a compound (XIV) (Figure 4). The silyl-protected compound (XV) is produced under base catalysis (Figure 4).

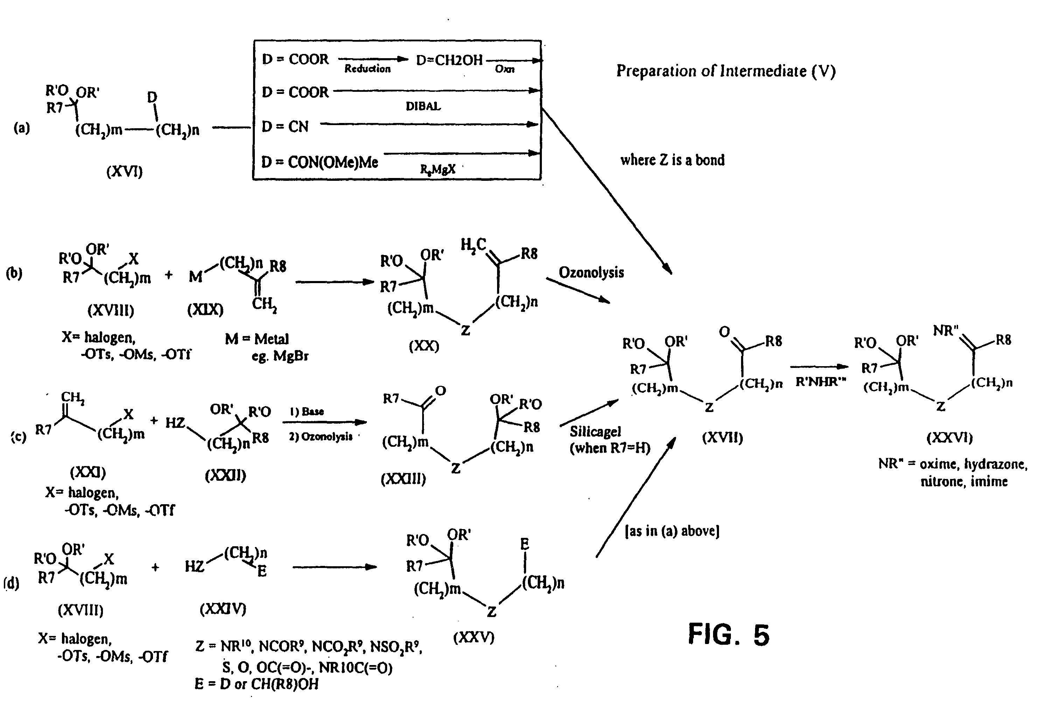

Figure 5 describes several methods for preparing intermediate (V). Procedure (a) describes the transformations of various acetals (XVI) to (XVII, Z=bond). For example, ester-acetal/ketal (XVI, D = COOR) is completely reduced to the corresponding alcohol and subsequently oxidized (e.g., Swem or Dess-Martin oxidation) to the aldehyde-acetal/ketal (XVII, R⁸ = H). Alternatively, ester-acetal/ketal (XVI, D = COOR) is partially reduced with DIBAL to afford aldehyde (XVII, R⁸ = H) directly. Similarly, reduction of nitrile-acetal (XVI, D = CN) with DIBAL gives aldehyde (XVII, R⁸ = H). Keto-acetals/ketal are prepared by addition of Grignard reagents to Weinreb amide-acetal/ketal (XVI, D = CON(OMe)Me).

Intermediate (XVII, Z=bond) can also be obtained by a two step procedure outlined in Procedure (b). Addition of organometallic reagent (XIX) to acetal/ketal (XVIII) gives alkene (XX) which upon ozonolysis followed by a reductive workup affords keto-acetal/ketal (XVII). Preparation of intermediate (XVII, Z = heteroatom) by a two step procedure is outlined in Procedure (c). Coupling acetal (XXII) with alkene (XXI) followed by ozonolysis (with a reductive workup) of the resulting alkene gives keto-acetal/ketal (XVII). Alternatively, intermediate (XVII, Z = heteroatom) is prepared by a two step procedure outlined in Procedure (d). Reaction of compound (XXIV) with acetal/ketal (XVIII) gives (XXV) which is transformed to keto-acetal/ketal (XVII) by the methods described in Procedure (a). Condensation of keto-acetal/ketal (XVII) with hydroxylamines, hydrazines, N-alkyl-N-alkoxyamines, and amines gives intermediate (XXVI) bearing an electrophilic C=N functionality.

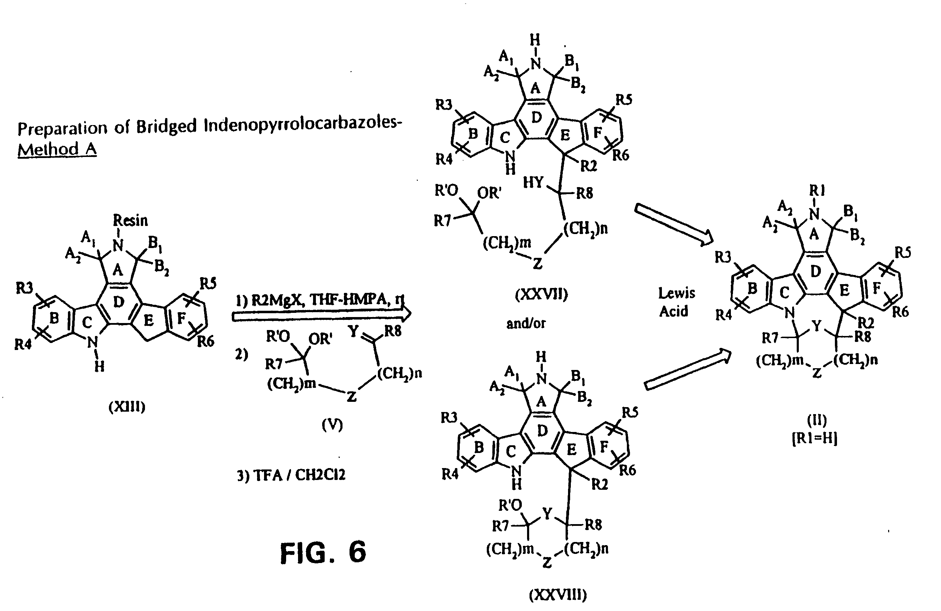

The resin-bound indenopyrrolocarbazole (XIII) (Figure 6, Method A) is treated with an excess of a Grignard reagent as a base, which results in the generation of a dark red solution of the carbanion. Subsequent reaction with (V) leads to products derived form electrophilic addition to the C=Y group. Aqueous workup and cleavage of the product(s) with dilute acid (1% TFA in methylene chloride) from the resin result in isolation of compound(s) (XXVII) and/or (XXVIII). Treatment of interrnediate(s) (XXVII) and/or (XXVIII) with either a sulphonic acid or a Lewis acid, e.g. boron trifluoride etherate, provides the bridged indenopyrrolocarbazoles (II).

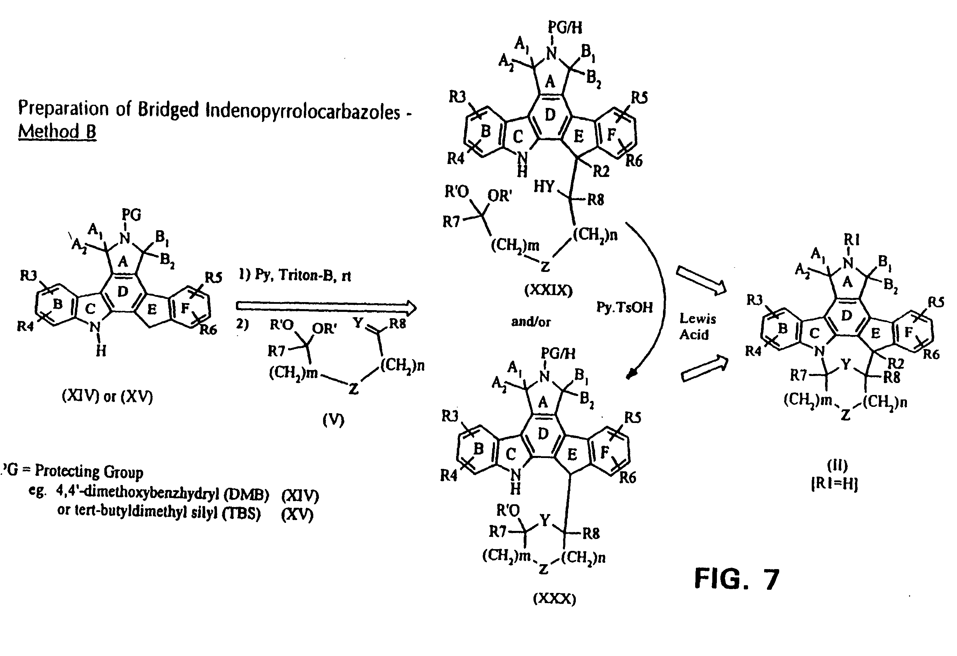

A similar strategy is employed for reaction of the soluble lactam protected intermediate, e.g. (XV) (Figure 7, Method B). However, in this case intermediate (XV) is treated with Triton B in pyridine as a base instead of the Grignard reagent. Intermediate(s) (XXIX) and/or (XXX) can be isolated with the lactam protecting group intact, which is amenable to chromatographic purification. As in method A, (Figure 6), treatment with a Lewis acid (such as boron trifluoride etherate) provides the bridged indenopyrrolocarbazoles (II), where R¹=H.

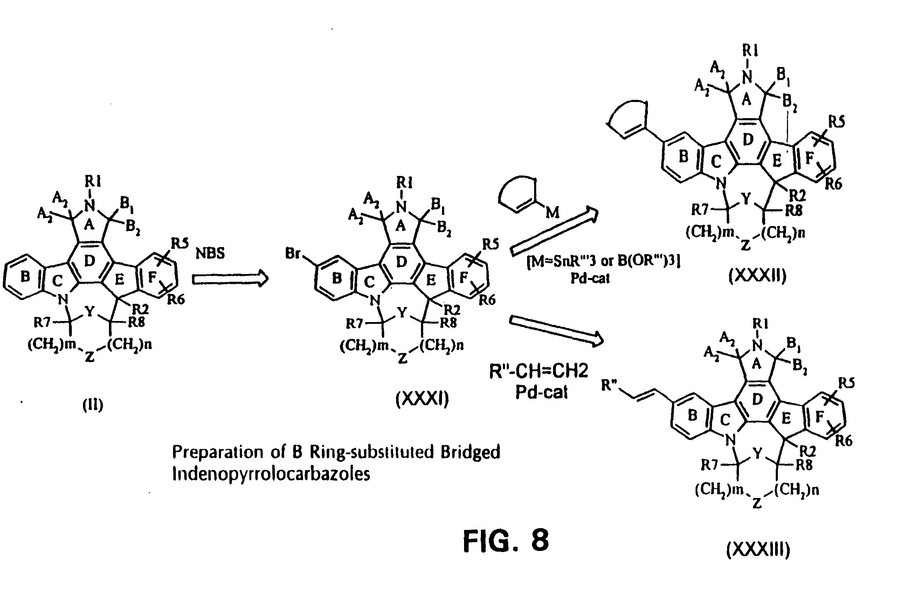

The introduction of groups R³, R⁴, R⁵ and R⁶ can be carried out as described in US Patents Nos. 5,705,511 and 4,923,986, the disclosures of which are incorporated by reference in their entirety. An R³ substituent can otherwise be introduced after the construction of the bridged indenopyrrolocarbazoles, as shown in Figure 8. The 3 position of the B ring is brominated with NBS providing compound (XXXI). A carbon fragment is subsequently introduced by employing palladium-catalyzed Stille, Suzuki, Heck, Kumada or Castro-Stephens reactions to provide compounds of the type (XXXII), (XXXIII), etc. In addition, compound (XXXI) can provide access to compounds where the bromine group is displaced with a heteroatom, e.g. an amine-based group by utilization of Buchwald's palladium catalyzed amination chemistry.

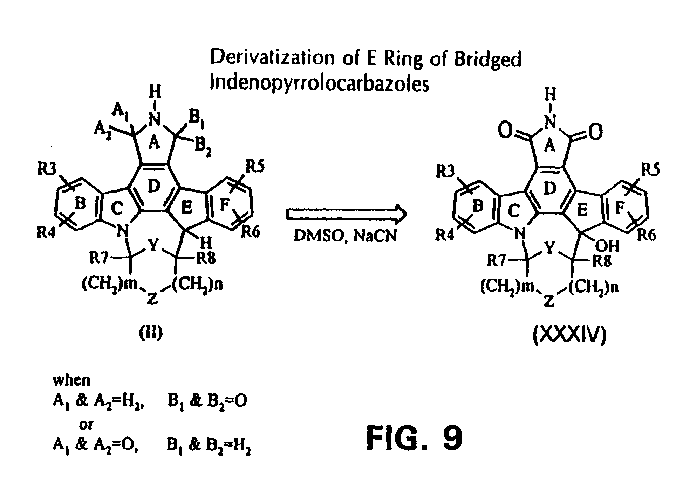

By an oxidative process, an oxygen linked group can be introduced at the indene carbon of the E ring, as shown in Figure 9, compound (XXXIV). This chemistry also results in oxidation of the methylene group of the lactam (A ring) providing an imide derivative, as shown.

### Example 2: Preparation of Rink Resin-bound intermediates: (XIII-A), (XIII-B) and (XIII-C), (Figure 3)

### Example 2-A

A three neck round bottom flask fitted with an overhead mechanical stirrer and a Dean-Stark trap was sequentially charged with Rink acid resin XII (10.00 g, 0.64 mmol/g), 1-methyl-2-pyrolidinone (80 mL), benzene (350 mL), VIII-A (A¹,A²=H₂, B¹,B²=O, R³=R⁴=R⁵=R⁶=H)) (3.00 g) and *p*-toluenesulfonic acid (1.00 g). The reaction mixture was warmed to reflux for 20 hours, and then filtered. The resin was washed with THF (5 x 175 mL) and the filtrate set aside. The resin was then sequentially washed with DMSO (4 x 100 mL), 2% aqueous NaHCO₃ (4 x 100 mL), water (4 x 100 mL), DMSO (2 x 200 mL), THF (4 x 100 mL) and ethyl acetate (4 x 100 mL). The resin was dried under vacuum (24 hours) to afford 11.70 (0.47 mmol/g) of resin bound VIII-A, (XIII-A),.

The original THF washings were evaporated, the residue was diluted with water (750 mL), and the resulting precipitate was filtered and sequentially washed with water, 2% aqueous NaHCO₃ (4 x 100 mL), and water (4 x 100 mL). After drying under vacuum, VIII-A (1.28 g) was recovered.

### Example 2-B

In a similar manner, VIII-B (A¹,A²=O, B¹,B²=H₂, R³=R⁴=R⁵=R⁶=H), (0.5 g) was coupled to Rink acid resin XII (1.52 g) to afford 1.58 g of resin bound VIII-B, (XIII-B).

### Example 2-C

In a similar manner, VIII-C (A¹,A²=H₂, B¹,B²=O, R³=R⁴=R⁵=H, R⁶=10-OMe), (1.02 g) was coupled to Rink acid resin XII (3.12 g) to afford 3.70 (0.46 mmol/g) of resin bound compound VIII-C, (XIII-C) along with recovered compound VIU-C (0.44 g).

### Example 3: Preparation of Compound (II-1), Compound (II-2), Compound (II-3), Compound (II-4a), Compound (II-4b), Compound (II-6) and Compound (II-8) (Method A, Figure 6)

### Example 3-A

To a suspension of (XIII-A), (1.25 g) in THF (24 mL) was added a 1.0 M solution of EtMgBr (6.25 mL in THF) and the reaction was stirred for 1 hour prior to the addition of HMPA (5.0 mL). After stirring for 10 minutes, diethoxybutyraldehyde (3.0 g) (which was prepared according to the literature procedure of Paquette, *et al*., *J. Am. Chem. Soc*., **1997,** *119*, 9662-71), was added, and the reaction was stirred for 20 hours. The reaction was quenched with 10% aqueous NH₄Cl (5 mL) and filtered. The resin was successively washed with 10% aqueous NH₄Cl (3 x 10 mL), water (3 x 10 mL), THF (3 x 10 mL), DMF (3 x 10 mL), water (3 x 10 mL), THF (3 x 10 mL), and ether (3 x 10 mL). The resin was dried under vacuum, taken up in methylene chloride (15 mL), and treated with trifluoroacetic acid (0.15 mL). After stirring for I hour, the reaction was filtered, and the filtrate was evaporated. The resulting residue was taken up in methylene chloride (20 mL) and treated with pyridinium tosylate (50 mg), and the resulting solution was stirred for 4 hours. At this time the reaction was washed with saturated aqueous NaHCO₃ and brine, and dried over MgSO₄.

After filtration and solvent evaporation, the residue was purified by preparative HPLC (Zorbax RX-8, 4 x 25 cm, eluted with 60% MeCN/water w/ 0.1% trifluoroacetic acid). The appropriate fractions were neutralized with NaHCO₃ and extracted into methylene chloride (3 x 50 mL) and dried over MgSO₄. After filtration and solvent evaporation, 70.2 mg of compound II-1 was obtained as a white powder which had the following characteristics: ¹³C NMR (DMSO-d₆) δ 171.8, 143.3, 142.4, 141.4, 140.1, 140.0, 136.6, 129.2, 127.9, 127.4, 127.1, 126.8, 124.1 (2C), 122.7, 121.6, 121.5, 118.3, 112.1, 88.1, 79.2, 56.6, 45.6, 33.4, 24.8; ¹H NMR (DMSO-d₆) d 9.21 (d, *J* = 7.5, 1H), 8.62 (s, 1H), 7.98 (d, *J* = 7.7, 1H), 7.86 (d, *J* = 8.3, 1H), 7.71 (d, *J* = 7.3, 1H), 7.49 (dd, *J =* 7.9, 7.4, 1H), 7.41 *(dd, J =* 7.5, 7.4, 1H), 7.36 - 7.27 (m, 2 H), 6.86 (d, *J* = 6.0, 1H), 5.63 - 5.58 (m, 1H), 4.91 (s, 2H), 4.53 (d, *J* = 3.3, 1H), 2.23 - 2.14 (m, 1H), 1.96 - 1.92 (m, 1H), 0.96- 0.88 (m, 1H), 0.60 - 0.57 (m, 1H); MS *m*/*z* (M+H) calcd 379, obsd 379.

Also isolated by preparative HPLC of this reaction product mixture was compound II-2 (0.5 mg) which had the following characteristics: ¹H NMR (DMSO-d₆) δ 9.17 (d, *J* = 8.1, 1H), 8.62 (s, 1H), 7.98 (d, *J* = 7.0, 1H), 7.85 (d, *J =* 6.8, 1H), 7.57 (d, *J =* 6.8, 1H), 7.49 (dd, *J* = 7.9, 7.4, 1H), 7.44 - 7.26 (m, 3H), 6.81 (d, *J* = 6.0, 1H), 5.43 - 5.33 (m, 1H), 4.43 (s, 2H), 2.23 - 2.14 (m, 1H), 1.96 - 1.92 (m, 1H), 1.45 - 1.55 (m, 2H), 0.96- 0.88 (m, 1H), 0.60 - 0.57 (m, 1H), 0.29 (t, *J =* 7.0, 3H); MS *m*/*z* (M+H) calcd 407, obsd 407.

### Example 3-B

In a similar manner, as described above for compound II-1, resin (XIII-A) (70.3 mg) was treated with 1,1-diethoxy-2-pentanone (0.75 mL) ) (which was prepared according to the literature procedure of Sworin, *et al*., *J. Org. Chem*., **1988,** *53,* 4894-6), to afford compound II-3 (3.5 mg) which was isolated by preparative TLC (silica gel, eluted with 50% EtOAc/toluene) and had the following properties: ¹H NMR (DMSO-d₆) δ 9.42 (d, *J* = 8.2, 1H), 8.58 (s, 1H), 7.95 (*d, J =* 7.4, 1H), 7.79 (*d, J =* 8.3, 1H), 7.71 (d, *J =* 7.1), 7.50 - 7.20 (m, 4H), 6.81 (d, *J* = 5.9, 1H), 4.90 (s, 2H), 4.46 (s, 1H), 2.35 - 2.20 (m, 1H), 1.98 (s, 3H), 1.75 - 1.60 (m, 1 H), 1.25 - 1.00 (m, 1H), 0.35 - 0.15 (m, I H); MS *m*/*z* (M+H) calcd 393, obsd 393.

### Example 3-C

In a similar manner, (XIII-A) (74.3 mg) was treated with 1,1-diethoxy-2-hexanone (which was prepared according to the literature procedure of Brenner, *J. Org. Chem*., **1961,** *26*, 22-7) (0.75 mL) to afford compound II-4a (2.10 mg) and compound II4b (1.06 mg) which were individually isolated by preparative HPLC (Zorbax RX-8, 4 x 25 cm, 65% MeCN/water w/ 0.1% trifluoroacetic acid). Compound II-4a had the following properties: ¹H NMR (DMSO-d₆,) δ 9.30 (d, *J=* 8.3, 1H), 8.55 (s, 1H), 7.97 (d, *J =* 7.2, 1H), 7.65 (d, *J =* 8.5, 1H), 7.59 (d, *J* = 7.5), 7.48 (dd, *J =* 7.8, 7.2, 1H) 7.39 - 7.15 (m, 3H), 6.31 (dd, *J =* 5.9, 5.5, 1H), 5.02 (s, 1H), 4.88 (s, 2H), 0.88 (s, 3H) other aliphatic signals lost under solvent peaks; MS *m*/*z* (M+H) calcd 407, obsd 407. Compound II-4b had the following properties: ¹H NMR (DMSO-d₆) d 9.43 (d, *J* = 8.1, 1H), 8.59 (s, 1H), 7.99 (d, *J* = 7.3, 1H), 7.75 - 7.65 (m, 2H), 7.49 (dd, *J* = 7.0, 6.4, 1H), 7.43 (dd, *J* = 8.2, 8.1, 1H), 7.36 - 7.25 (m, 2H), 6.75 (s, 1H), 4.91 (s, 2H), 4.50 (s, 1H), 1.95 (s, 3H) other aliphatic signals lost under solvent peaks; MS m/z (M+H) calcd 407, obsd 407.

### Example 3-D

In a similar manner, (XIII-C) (1.00 g) was treated with diethoxybutyraldehyde (3.65 g) to afford compound II-6 (87.8 mg) which was isolated by preparative HPLC (Zorbax RX-8, 2.5 x 25 cm, 65% MeCN/water w/ 0.1% trifluoroacetic acid) and had the following properties: ¹H NMR (DMSO-d₆) δ 9.09 (d, *J* = 8.6, 1H), 8.60 (s, 1H), 7.95 (d, *J =* 7.4, 1H), 7.84 (d, *J =* 8.3, 1H), 7.47 (dd, *J* = 7.2, 7.0, 1H), 7.35 (s, 1H), 7.29 (dd, *J* = 7.0, 7.0, 1H), 6.98 (dd, *J* = 8.6, 1.9, 1H), 6.83 (d, *J* = 6.0, 1H), 5.65 - 5.55 (m, 1H), 4.88 (s, 2H), 4.48 (d,*J* = 3.9, 1H), 3.82 (s, 3H), 2.25 - 2.10 (m, 1H), 2.08 - 1.85 (m, 1H), 0.96 - 0.75 (m, 1H), 0.65 - 0.50 (m, 1H); MS *m*/*z* (M+Na) calcd 431, obsd 431.

### Example 3-E

In a similar manner, resin (XIII-B) (153.2 mg) was treated with diethoxybutyraldehyde (1.5 mL) to afford compound II-8 (3.6 mg) which was isolated by preparative HPLC (Zorbax RX-8, 2.5 x 25 cm, 65% MeCN/water w/ 0.1% trifluoroacetic acid) and had the following properties: ¹H NMR (DMSO-d₆) δ 9.09 (d, *J* = 7.9, 1H), 8.81 (s, 1H), 7.81 - 7.73 (m, 3H), 7.48 - 7.35 (m, 3H), 7.24 (dd, *J* = 7.6, 7.5, 1H), 6.85 (d,*J* = 6.2, 1H), 5.63 - 5.59 (m, 1H), 4.86 (s, 2H), 4.61 (d, *J* = 3.6, 1H), 3.82 (s, 3H), 2.21 - 2.13 (m, 1H), 1.96 - 1.90 (m, 1H), 0.87 - 0. 79 (m, 1H), 0.61 - 0.56 (m, 1H); MS *m*/*z* (M+H) calcd 379, obsd 379.

### Example 4: Preparation of Compound II-7a and Compound II-7b (Method A, Figure 6)

### Example 4-A

### Preparation of (1,1-diethoxyethoxy)acetone

To a cold (0 °C) suspension of NaH (2.68 g, 60%) in THF (150 mL) was added a solution of 1,1-diethoxyethanol (which was prepared according to the literature procedure of Zirkle, *et*. *al*., *J*. *Org. Chem*., **1961**, *26*, 395-407) (9.00 g) in THF (20 mL), and the reaction mixture was stirred at room temperature for 1 hour before adding methallyl chloride (8.0 mL). The reaction mixture was heated to reflux overnight, cooled and filtered through a plug of celite. Solvent was removed by rotary evaporation, and the residue purified by column chromatography (silica, 20% ether/hexane) to give 1,1-diethoxyethyl methallyl ether (11.5, 90%). Ozonolysis of a chilled (-30 °C) solution of this ether (6.00 g) in EtOAc (80 mL) was carried out until no starting material was detectable by TLC (1 hour). At this time, the reaction was purged with oxygen, treated with Pd(OH)₂ (150 mg) and stirred under an atmosphere of hydrogen overnight. The catalyst was filtered away, and the filtrate was concentrated by rotary evaporation. The resulting residue was purified by column chromatography (silica, 20 % EtOAc/hexane) to afford the title compound (4.53 g, 82 %).

### Example 4-B

According to Method A (Figure 6), resin (XIII-A) (230.2 mg) was treated with EtMgBr (1.25 mL) followed by (1,1-diethoxyethoxy)acetone (Example 3-A) (1.2 mL). After workup and cleavage from the resin, a portion of the crude reaction product mixture (10.5 mg) was taken up in methylene chloride (20 mL) and treated with BF₃ etherate (20 uL). After stirring for 2.5 hours, the solution was washed with saturated aqueous NaHCO₃ and brine prior to drying over MgSO₄. After filtration and solvent removal, the resulting residue was purified by preparative HPLC (Zorbax RX-8, 4 x 25 cm, 65% MeCN/water w/ 0.1% trifluoroacetic acid) to afford compound II-7a (2.34 mg) and compound II-7b (1.34 mg). Compound (II-7a) had the following properties: ¹H NMR (CDCl₃) δ 9.35 - 9.20 (m, 1H), 7.87 (d, *J* = 7.6, 1H), 7.62 (d, *J* = 7.0, 1H), 7.60 - 7.45 (m, 1H), 7.49 (dd, *J* = 7.7, 7.5, 1H), 7.40 (d, *J* = 8.1, 1H), 7.37 - 7.26 (m, 3H), 6.22 (s, 1H), 5.20 - 4.85 (m, 1H), 4.47 (s, 1H), 3.67 (d, *J* = 12.7, 1H) 3.52 (d, *J* = 11.8, 1H), 3.40 (d, *J* = 12.7, 1H), 3.38 (d, *J* = 11.8, 1H), 1.91 (s, 3H); MS *m*/*z* (M+H) calcd 409, obsd 409. Compound II-7b had the following properties: ¹H NMR (CDCl₃) δ 9.58 - 9.22 (m, 1H), 7.82 (d, *J*= 7.4, 1H), 7.60-7.40 (m, 3H), 7.37 - 7.27 (m, 3H), 7.21 (d, *J* = 8.1, 1H), 5.81 (s, 1H), 5.21 (s, 1H), 5.10 - 4.80 (m, I H), 4.59 (d, *J =* 13.5, 1H), 4.38 (dd, *J* = 13.5, 5.3, 1H), 4.21 (d, *J* =13.1, 1H), 3.82 (d, *J* = 13.2, 1H), 1.13 (s, 3H); MS *m*/*z* (M+H) calcd 409, obsd 409.

### Example 5: Preparation of Compound II-5 (Figure 8)

To a solution of compound II-1 (8.1 mg) in THF (2 mL) was added NBS (4.6 mg), and the reaction was stirred overnight. Additional NBS (4.5 mg) was added, and the reaction stirred for 2.5 hours. Insoluble material was filtered away and the filtrate was concentrated by rotary evaporation. The resulting residue was purified by column chromatography (C-18, 65% MeCN/water w/ 0.1% trifluoroacetic acid). The appropriate fractions were neutralized with NaHCO₃ and extracted into methylene chloride (3 x 20 mL) and dried over MgSO₄. After filtration and solvent evaporation, compound II-5 (5.1 mg) was obtained as white powder which had the following characteristics: ¹H NMR (DMSO-d₆) δ 9.22 (d, *J =* 7.4, 1 H), 8.67 (s, 1H), 8.14 (s, 1H), 7.86 (d, *J* = 8.7, 1H), 7.72 (d, *J* = 7.0, 1 H), 7.63 (d, *J =* 7.8, 1H), 7.42 (dd, *J =* 7.5, 7.3, 1H), 7.35 (dd, *J =* 7.3, 7.2, 1H), 6.86 (d, *J* = 6.0, 1H), 5.63 - 5.58 (m, 1H), 4.94 (s, 2H), 4.54 (d, *J* = 3.1, 1H), 2.30 - 2.14 (m , 1H), 2.00 - 1.82 (m, 1H), 0.96- 0.88 (m, 1H), 0.62 - 0.50 (m, 1H); MS *mlz* (M+H) calcd 457/9 (1:1), obsd 457/9 (1:1).

### Example 6: Preparation of Intermediate XV (Figure 4)

To a solution of VIII-A [A¹,A²=H₂, B¹,B²=O, R³=R⁴=R⁵=R⁶=H)] (1.05 g) in DMF (25 mL) was added triethylamine (0.75 mL) and t-butyldimethylsilyl chloride (TBS-Cl) (0.65 g). After stirring for 3 hours, the reaction was quenched with saturated aqueous NaHCO₃ and extracted into EtOAc. The organic layer was washed with water and brine and dried over MgSO₄. After filtration and solvent evaporation, the resulting residue was triturated with ether to give compound XV (848 mg). The washings were evaporated to leave a residue that was purified by column chromatography (silica, 1% EtOAc/CH₂Cl₂) and gave additional product (502 mg, combined yield of 94%) that had the following spectral properties: ¹H NMR (DMSO-d₆) δ 11.94 (s, 1H), 9.32 (d, *J* = 7.6, 1H), 8.03 (d, *J* = 7.7, 1H), 7.64 (d, *J* = 7.2, 1H), 7.58 (d, *J =* 8.1, 1H), 7.44 (dd, *J* = 7.7, 7.6, 1H), 7.39 (dd, *J* = 7.7, 7.6, 1H), 7.32 (d, *J* = 7.3, 1H), 7.25 (dd, *J* = 7.6, 7.3, 1H), 5.00 (s, 2H), 4.14 (s, 2H), 0.99 (s, 9H), 0.46 (s, 6H); MS *m*/*z* (M+H) calcd 425, obsd 425.

### Example 7: Preparation of Compound II-1 via Method B (Figure 7)

A solution of Triton B in pyridine (0.45 M) was prepared by dissolving a 40% solution of Triton B in methanol (10 mL) in pyridine (10 mL). Solvent was removed under reduced pressure (20 mm Hg) to a final volume of ∼ 8 mL. The residue was diluted with pyridine to 50 mL, filtered and stored under nitrogen. A solution of XV (20.3 mg) in pyridine (2.0 mL) was flushed with argon and treated with 300 µL of Triton B (0.45 M in pyridine) and diethoxybutyraldehyde (50 µL). After stirring for 2 hours, the reaction was extracted into EtOAc, washed with 1N aqueous HCl, brine and dried over MgSO₄. After filtration and solvent evaporation, the adduct was taken up in CH₂Cl₂ (10 mL) and treated with BF₃ etherate (10 µL). After stirring for 2.0 h, the solution was washed with saturated aqueous NaHCO₃ and brine prior to drying over MgSO₄. Removal of solvent by rotary evaporation gave a residue that was purified by preparative HPLC (Zorbax RX-8, 2.5 x 25 cm, 65% MeCN/water w/ 0.1% trifluoroacetic acid). The appropriate fractions were neutralized with NaHCO₃ and extracted into methylene chloride (3 x 20 mL) and dried over MgSO₄. After filtration and solvent evaporation, II-1 (11.8 mg, 65% yield) was obtained whose ¹H NMR and MS spectra and HPLC retention time were identical to material prepared and isolated by method A, described in Example 3-A.

### Example 8: Preparation of Compound II-9 (Figure 8)

To a suspension ofbromo compound II-5 (6.2 mg) in 1-propanol (4.0 mL) was added 3-aminophenylboric acid (3.8 mg). After stirring for 0.25 hour, Pd(OAc)₂ (2.0 mg) Ph₃P (4.8 mg), Na₂CO₃ (2.8 mg), and water (2.0 mL) were sequentially added. The mixture was heated at reflux for 0.75 hour, cooled, extracted into CH₂Cl₂, and washed with water and brine. The organic layer was dried over MgSO₄, and solvent was removed by rotary evaporation to give a residue that was purified by preparative HPLC (Zorbax RX-8, 2.5 x 25 cm, 50% MeCN/water w/ 0.1% trifluoroacetic acid). The appropriate fractions were neutralized with NaHCO₃ and extracted into methylene chloride (3 x 20 mL) and dried over MgSO₄. After filtration and solvent evaporation, compound II-9 (3.1 mg, 49% yield) was obtained and had the following spectral properties: ¹H NMR (DMSO-d₆) δ 9.22 (d, *J* = 7.5, 1H), 8.66 (s, 1H), 8.00 - 7.25 (m, 8H), 7.12 (dd, *J* = 7.1, 7.0, 1H), 6.95 - 6.80 (m, 3H), 6.53 (d, *J =* 6.0, 1H), 5.63 - 5.58 (m, 1H), 4.99 (s, 2H), 4.55 (s, 1H), 2.25 - 2.10 (m, 1H), 1.95 - 1.90 (m, 1H), 0..98 - 0.88 (m, 1H), 0.65 - 0.57 (m, 1H); MS *m*/*z* (M+H) calcd 470, obsd 470.

### Example 9: Preparation of Compound II-10 (Figure 9)

To a solution of compound II-1 (5.0 mg) in DMSO (1 mL) was added NaCN (4.3 mg), and the mixture was warmed to 145 C for 1 hour. The mixture was cooled, extracted into EtOAc, and washed with water (3 x 20 mL) and brine. The organic layer was dried over MgSO4, filtered and evaporated to give a residue that was purified by preparative HPLC (Zorbax RX-8, 2.5 x 25 cm, 55% MeCN/water w/ 0.1% trifluoroacetic acid). The appropriate fractions were neutralized with NaHCO₃, extracted into methylene chloride (3 x 20 mL), and dried over MgSO₄. After filtration and solvent evaporation, compound II-10 (2.7 mg, 50% yield) was obtained and had the following spectral properties: ¹H NMR (DMSO-d₆) δ 11.4 (s, 1H), 8.86 (d, *J* = 7.9, 1H), 8.79 (d,*J* = 7.6, 1H), 7.90 (d, *J* = 8.3, 1H), 7.62 - 7.55(m, 2H), 7.49 (dd, *J =* 7.6, 7.4, 3H), 7.40 (dd, *J* = 7.4, 7.3 1H), 7.35 (dd, *J =* 7.5, 7.4, 1H), 6.86 (d, *J* = 6.0, 1H), 6.03(s, 1H), 5.40-5.30 (m, 1H), 2.25-2.14 (m, 1H), 2.03-1.90 (m, 1H), 1.10-0.98 (m, 1H), 0.82 - 0.77 (m, 1H).

### Example 10: Preparation of Compound II-11 (Method A, Figure 6)

According to the method A, resin (XIIIa) (150.2 mg) was reacted with EtMgBr (1.0 mL) followed by ethyl 2,5-dioxopentanoate (Schmidt, *et al*., *Synthesis*, **1993**, 809) (1.5 mL). After workup and cleavage from the resin, the crude reaction product mixture was taken up in methylene chloride (20 mL) and treated with BF₃ etherate (20 µL). After stirring for 2.5 hours, the solution was washed with saturated aqueous NaHCO₃ and brine prior to drying over MgSO₄. After filtration and solvent removal, the resulting residue was purified by preparative HPLC (Zorbax RX-8, 4 x 25 cm, 55%-75% gradient MeCN/water w/0.1% trifluoroacetic acid) to afford compound II-11 (6.4 mg) which had the following properties: ¹H NMR (DMSO-d₆) δ 9.36 (d, *J =* 7.7, 1H), 8.68 (s, 1H), 8.00 (d, *J =* 7.7, 1H), 7.83 (d, *J =* 8.3, 1H), 7.58-7.15 (m, 5H), 6.97 (d, *J* = 5.9, 1H), 4.93 (s, 2H), 4.82 (s, 1H), 4.48 (q, *J* = 7.1, 2H), 2.42 - 1.91 (m, 2H), 1.37 (t, 3H, *J* = 7.1), 1.25 - 0.63 (m, 2H).

### Example 11: Preparation of Compound II-12

A solution of compound II-11 (3.4 mg) in THF (2 mL) was treated with a 2 M solution of LiBH₄ (1.0 mL in THF) and the reaction was stirred for 1.5 h. The reaction was quenched by the addition of 1 N aqueous HCl (4 mL). After stirring for 20 minutes, 10% aqueous NaOH (15 mL) was added and the mixture was extracted into methylene chloride (3 x 10 mL). After drying over MgSO₄, the mixture was filtered and solvent evaporated to afford compound II-12 (0.32 mg) which had the following properties: ¹H NMR (DMSO-d₆) δ 9.35 *(d, J =* 7.7, 1H), 8.62 (s, 1H), 7.98 (d, *J =* 7.7, 1H), 7.83 (d, *J* = 8.2, 1H), 7.75 (d, *J* = 8.2, 1H), 7.50 - 7.25 (m, 4H), 6.84 *(d, J =* 7.7, 1H), 6.11 (s, 1H), 4.91 (s, 2H), 4.71 (s, 1H), 4.50 - 4.40 (m, 1H), 4.30 - 4.20 (m, 1H) 2.42 - 1.91 (m, 2H), 1.25 - 0.63 (m, 2H); MS *m*/*z* (M+H) calcd. 409, obsd. 409.

### Example 12: Enhancement of Spinal Cord ChAT Activity

ChAT is a specific biochemical marker for functional cholinergic neurons. Cholinergic neurons represent a major cholinergic input into the hippocampal formation, olfactory nucleus, interpeduncular nucleus, cortex, amygdala, and parts of the thalamus. In the spinal cord, the motoneurons are cholinergic neurons which contain ChAT (Phelps, *et al*., *J*. *Comp. Neurol*.,**1988,** *273*, 459-472). ChAT activity has been used to study the effects of neurotrophins (e.g., NGF or NT-3) on the survival and/or function of cholinergic neurons. The ChAT assay also serves as an indication of the regulation of ChAT levels within cholinergic neurons.

*Methods:* Fetal rat spinal cord cells were dissociated, and experiments were performed as described (Smith, *et al*., *J*. *Cell Biology,* **1985,** *101*, 1608-1621; Glicksman, *et al*., *J*. *Neurochem.,* **1993,** *61*, 210-221). Dissociated cells were prepared from spinal cords dissected from rats (embryonic day 14-15) by standard trypsin dissociation techniques (Smith et al., *supra*.). Cells were plated at 6 x 10⁵ cells/cm² on poly-1-omithine coated plastic tissue culture wells in serum-free N2 medium supplemented with 0.05% bovine serum albumin (BSA) (Bottenstein, *et al*., *Proc. Natl. Acad. Sci. USA,* **1979**, *76*, 514-517). Cultures were incubated at 37°C in a humidified atmosphere of 5% CO₂/95% air for 48 hours. ChAT activity was measured after 2 days *in vitro* using a modification of the Fonnum procedure (Fonnum, *Neurochem*., **1975,** *24*, 407-409) according to McManaman, *et al*. and Glicksman, *et al*. (McManaman, *et al*., *Develop. Biol.,* **1988**, *125,* 311-320; Glicksman, *et al*., *J. Neurochem*., *supra.*)*.*

Compounds having formula II described in the examples are listed in Table 2. Values for R¹, R⁴, R⁶, and R⁷ are H; Y is O; and n is 1.

**Table 2**

| **Compound No.** | **A**_{**1**}**A**_{**2**} | **B**_{**1**}**B**_{**2**} | **R**_{**2**} | **R**_{**3**} | **R**_{**5**} | **R**_{**8**} | **Z** | **m** |
|---|---|---|---|---|---|---|---|---|
| II-1 | O | H,H | H | H | H | H | bond | 1 |
| II-2 | O | H,H | Et | H | H | H | bond | I |
| II-3 | O | H,H | H | H | H | Me | bond | 1 |
| II-4a | O | H,H | H | H | H | Me | bond | 2 |
| II-4b | O | H,H | H | H | H | Me | bond | 2 |
| II-5 | O | H,H | H | Br | H | Me | bond | 1 |
| II-6 | O | H,H | H | H | 10-OMe | H | bond | 1 |
| II-7a | O | H,H | H | H | H | Me | O | 1 |
| II-7b | O | H,H | H | H | H | Me | O | 1 |
| II-8 | H,H | O | H | H | H | H | bond | 1 |
| II-9 | O | H,H | H | 3'-NH₂-Ph | H | H | bond | 1 |
| II-10 | O | O | O H | H | H | H | bond | 1 |
| II-11 | O | H,H | H | H | H | CO₂-Et | bond | 1 |
| II-12 | O | H,H | H | H | H | CH₂-OH | bond | 1 |

### Example 13: pCDNA3-EE-MLK3, pcDNA3-EE-MLK3(K144R)

MLK3 was cloned as described (Lee, *et al*., *Oncogene,* **1993,** *8*, 3403-3410; *Ezoe, et al*., *Oncogene*, **1994,** 9, 935-938). cDNA was prepared from 200 ng polyadenylated melanocyte mRNA and 5% of the reaction was used as template to amplify a repertoire of PTK cDNAs using mixtures of either two or four highly degenerate oligonucleotide primers derived from the consensus sequences of the conserved VIb and IX subdomains of known PTKs: PTK1, 5'-CGGATCCACMGIGAYYT-3' (SEQ ID NO:1); PTK2, 5'-GGAATTCCAWAGGACCASACRTC-3' (SEQ ID NO:2); PTK3, 5'-CGGATCCRTICAYMGIGAYYTIGCIGCIMGIAA-3' (SEQ ID NO:3); PTK4, 5'-GGAATTIAYIGGAWAIGWCCAIACRTCISW-3' (SEQ ID NO:4). Forty cycles of PCR were carried out using Taq DNA polymerase (AmpliTaq; Perkin-Elmer/Cetus) and an automated DNA thermal cycler; each cycle consisted of 40 s at 94°C, 2 min at 37°C and 3 min at 63°C. The products of eight PCRs were pooled, treated with DNA polymerase (Klenow), cleaved with BamH1 plus EcoR1 and electrophoresed in a 5% polyacrylamide gel. Ethidium bromide staining identified a predominant 200-230 bp band which was excised, eluted and cloned into M 13mp 18. In one experiment, part of the PCR amplified cDNA was not cleaved, but instead was cloned blunt into M 13mp18 cleaved with Sma1. Nucleotide sequences were determined by chain-termination sequencing method.

One cDNA, identified as PTK1, was used as a probe to screen human melanoma and melanocyte cDNA libraries. A clone, designated PTK1-3.2, included the entire open reading frame of 2541 nt, coding for a protein of 847 amino acids. This cDNA was cut with Nco1, blunted with DNA polymerase (Klenow), cut again with EcoR1 and ligated into the vector pCDNA3-EE cut with BamH1, blunted and then cut with EcoRl. The vector pCDNA3-EE was constructed by inserting into the HindIII/BamH1 site an oligo that codes for a start codon followed by the EE epitope, MEEEEYMPME (SEQ ID NO:5) (Grussenmeyer, *et al*., *Proc. Natl. Acad. Sci. USA,* **1985**, *82*, 7952-7954). The kinase-dead version of MLK3 was made by making the mutation K144R using PCR employing a previously published technique (Chen, *et al*., *Biotechniques,* **1994,** *17*, 657-659). The first, mutagenic oligo was 5'-GTGGCTGTGCGGGCAGCTCGCCAG-3' (SEQ ID NO:6) and the second oligo was 5'-GAGACCCTGGATCTCGCGCTT-3' (SEQ ID NO:7). Using MLK3 as a template, these oligos were used in PCR to generate a fragment of 806 bp and employed in a second PCR reaction using a T7 primer as the other ampiimer and MLK3 as the template to generate a fragment of 1285 bp. The fragment was separated by agarose gel electrophoresis, isolated, cloned into pGEM-5 (Promega) and sequenced. The fragment was excised with HindIII and HpaI, and inserted into pCDNA3-EE-MLK3 cut with HindIII and HpaI. An additional point mutation was detected at nucleotide 1342. To correct this, a Pf1M1 fragment (nt 1093-1418) was excised from the wild-type MLK3 and used to replace the identical fragment in the K144R mutated MLK3.

### Example 14: pFB-FLAG-MLK3

To obtain MLK3 protein, the cDNA was cloned into the baculoviral expression vector pFB-FLAG. MLK3 was excised from PTK1-3.2 by digestion with Nco 1, blunted with DNA polymerase (Klenow), cut again with Not 1 and ligated into pFB-FLAG digested with Stu1 and Not1. pFB-FLAG is derived from pFB (Life Technologies) and has the coding sequence for the FLAG epitope (Hopp, *et al*., *Biotechnology*, **1988,** *6*, 1205-1210) with a start codon, MDYKDDDDK (SEQ ID NO:8), added to the polylinker in the BamH1 site.

### Example 15: pFB-GST-MLK3(KD)

Baculoviral expression of the kinase domain of MLK3 was achieved by excising the MLK3 fragment from the pGEXKG-MLK3(KD) using EcoR1 and Xho1 and ligating it into a pFB vector cut with EcoR1 and Xho1 in which the coding sequence for glutathione S-transferase (GST) had been cloned upstream. This was achieved by obtaining the GST coding sequence and polylinker from the pGEXKG vector by PCR using the vector as a template (Guan, *et al*., *Anal. Bioch.,* **1991,** *192*, 262-267). The 5' oligo for PCR created a Bgl2 restriction site at the 5' end of the fragment. This isolated fragment was then digested with Bgl2 and HinD3 and ligated into pFB digested with BamH1 and HinD3.

### Example 16: pGEXKG-MLK3(KD)

A cDNA fragment that included both the MLK3 kinase domain and a portion of the leucine zipper (nt 736-1791) was obtained by PCR using the PTK1 cDNA. The isolated fragment was digested with the restriction enzymes EcoR 1 and Xho1, sites that were included in the PCR oligos, and cloned into pGEX-KG digested with EcoR1 and Xho1. This fragment in pGEX-KG was then shortened by PCR to include only the kinase domain (nt 736-1638).

### Example 17: pKH3-MLK2, pKH3-MLK2(KA)

MLK2 was cloned using degenerate PCR (Dorow, *et al*., *Eur*. *J*. *Biochem*., **1993,** *213,* 701-710; Dorow, *et al*., *Eur. J*. *Biochem.,* ***1995,*** *234*, 492-500). Segments of cDNAs encoding catalytic subdomains of protein kinases expressed in the epithelial tumor cell line Colo 16 were amplified from RNA by reverse transcriptase PCR. Degenerate PCR primers were based in sequences encoding conserved motifs in subdomains Vib and VIII of the epidermal-growth-factor receptor family kinase catalytic domains. Sequences of the primers were as follows: forward primer, 5'-CGGATCCGTG(A)CACC(A)GT (CG)G(A)ACC(T)T-3' (SEQ ID NO:9), reverse primer, 5'-GGAATTCACCA(G)TAA (G)CTCCAG(C)ACATC-3' (SEQ ID NO:10). Several PCR products were cloned into M13 and sequenced using a T7 Super-Base sequencing kit (Bresatec). One 216-bp PCR product was used as a probe to screen a human colon λgt11 cDNA library (Clontech, catalog #HL 10346)). The fragment was random-primed labeled, hybridization was performed at 65'C and the filters washed to a stringency of 0.2X NaCl/Citrate (150 mM sodium chloride, 15 mM sodium citrate, pH 7.0) and 0.5% SDS at 65°C. Filters were autoradiographed for 16h at -70°C on Kodak XAR-5 film. Four clones were isolated and the longest, 1.2 kb, was used to reprobe the same library using the same conditions. Four more clones were selected and one of these clones represented a 1034 bp fragment of MLK2. This clone was used to probe a human brain λgt10 library. Approximately 500,000 clones were screened and one 3454 bp clone was isolated, representing the entire coding region of MLK2.

MLK2 was cloned, from the ATG to the polyA tail, into the vector pKH3 between the BamH1 and EcoR1 sites in two steps as there is a BamH1 site in the middle of the MLK2 sequence. The vector pKH3 was constructed by inserting three copies of the HA epitope tag followed by a BamH1 site between the Xba1 and EcoR1 sites of the pRK7 polylinker. To make the mutagenized version, K125A, the MLK2 5' BamH1 fragment was cloned into the Promega pAlter vector and mutated as recommended by the manufacturer. The fragment was then cloned back into the MLK2 pKH3 vector.

### Example 18: pcDNA3-HA-JNK1

JNK1 cDNA was obtained as described (Coso, *et al*., *Cell*, **1995,** *81*,1137-1146). The cDNA was obtained by PCR using as a template human skeletal muscle cDNA (Invitrogen) and was cloned into the Bgl2 / Sal1 sites of pcDNA3-HA, a modified pcDNA3 expression plasmid encoding the HA epitope (Wilson, *et al*., *Cell*, **1984,** *37*, 767-778). This was then excised from pcDNA3, including the HA epitope, and ligated into pGEX-4T3 (Pharmacia). The JNK1 cDNA was excised from the pGEX-4T3 construct as a Bgl2 / Sal1 fragment and ligated into pcDNA3-HA, a vector with the HA epitope added in the HinD3/BamH1 site of pcDNA3.

### Example 19: pFLAG-DLK

DLK was cloned into the expression vector pcDNA3 with the FLAG epitope added as described (Holzman, *et al*., *J*. *Biol*. *Chem*., **1994,** *269*, 30808-30817). A fragment of the cDNA for DLK was isolated by degenerate oligonucleotide-based PCR cloning. Total RNA was extracted from embryonic day 13.5 kidneys (32 organs) and embryonic day 17.5 kidneys (16 organs) using a commercially prepared phenol/guanidine isothiocyanate reagent method according to the directions of the manufacturer (TRIzol Reagent, Life Technologies, Inc.). Following digestion with RNase-free DNase I, total RNA was reverse transcribed with RNase H-reverse transcriptase (Superscript, Life Technologies, Inc.) from an oligo(dT) synthetic oligonucleotide primer to single-stranded cDNA. Degenerate oligonucleotide primers corresponding to the protein tyrosine kinase catalytic subdomains Vib and IX originally designed by Wilks (Wilks, *Proc Natl Acad Sci USA.,* **1989,** *86*, 1603-1607) were modified to 5' *Eco*RI and *Hin*dIII sites, respectively (5'-ATAATTC(GT)GC(TAGC)GCCA(GA)GTC(TAGC)CGGTG-3' (SEQ ID NO:11), 5'-ATAAGCTTCC(TC)(AG)T(GC)AAGTGGA(TC)(GC)GC(AGC)CC(CT)GA-3') (SEQ ID NO:12). Forty PCR cycles were carried out for 1.5 min at 94°C, 2 min at 37°C, and 3 min at 63°C. Fresh reagents were added and an additional 40 cycles were completed before a final 10-min extension at 72°C. The resultant 200-210-bp DNA amplification product was gel isolated, subcloned into a prepared pGEM7zf(+) plasmid (Promega), and transformed into *Escherichia coli.* Miniprep plasmid DNA was prepared from transformed bacteria and a portion digested with *Eco*RI and *Hind*III restriction endonucleases; clones containing inserts were sequenced.

The 195-bp DLK cDNA fragment obtained from the degenerate PCR was radiolabeled and used to screen approximately 1 x 10⁶ recombinants of a Uni-ZAP II (Stratagene, La Jolla, CA), oligo(dT)-primed adult mouse brain cDNA library (Holzman, *et al*., *Mol Cell Biol,* **1990,** *10*, 5830-5838). Filters were hybridized in a buffer consisting of 50% formamide, 5 x SSC, 3 x Denhardt's solution, 0.25% SDS, 1 mg/ml polyadenylic acid, and 200 mg/ml salmon sperm DNA at 42°C. Filters were washed once at room temperature in 2 x SSC, 0.2% SDS and twice for 30 min at 65°C. Twenty five unique clones were identified; 10 clones were purified to homogeneity, *in vivo* excised according to the protocol of the manufacturer and restriction mapped. The two longest clones (3401 and 3397 bp, respectively, differing only at their 5' termini) were sequenced along both strands over their entire length.

The full-length *Not*I-*Xho*I DLK cDNA fragment (3401 bp) was subcloned into the cytomegalovirus promoter based eukaryotic expression vector pcDNA3 (Invitrogen, San Diego, CA) (construct designated pcDNA3-DLK). Next, a NH₄-Met FLAG epitope (DYKDDDDK) (SEQ ID NO:13) tagged construct (pFLAG-DLK) was made. The PCR was used to amplify cDNA fragments which encoded a 5' *Hind*III site, DLK's Kozak's consensus sequence including the initiation ATG, the FLAG epitope, and DLK cDNA open reading frame sequence extending from nucleotide 88 to an internal *EcoR*I site at nucleotide 758. (HPLC purified synthetic oligonucleotides used in equimolar quantities: 5'-ATAAAGCTTCCAGAGGCCATGGACTACAAGGACGACGATGACAAGGC-CTGCCTCCATGAAACCCGAACA-3' (SEQ ID NO:14) for the FLAG construct sense primer and 5'-GACAGGGCGGCCGGCTCT-3' (SEQ ID NO:15) for the antisense primer.) Gel purified *Hind*III and *Eco*RI-digested amplified fragments were subcloned into the *Hind*III-*Eco*R1 to prepared pcDNA3-DLK plasmid. Constructs were sequenced along both strands to assure Taq polymerase fidelity and maintenance of reading frame.

### Example 20: pcDNA3-MLKI

The 5' portion of MLK1 was obtained from the EST database (accession # AA160611 ). This clone was a fusion between MLK1 and anothercDNA of unknown identity. It contained previously unpublished 5' sequence of MLK1 along with part of the previously published kinase domain of MLK1 (Dorow, *et al*., *Eur. J. Biochem*., **1993,** *213*, 701-710). The MLK 1 cDNA sequence from the EST clone is as follows: GAATTCGGCA CGAGAGGACT CGCAGGTGTC CGGCGACGAG GGCTGGTGGA CCGGGCAGCT GAACCAGCGG GTGGGCATCT TCCCCAGCAA CTACGTGACC CCGCGCAGCG CCTTCTCCAG CCGCTGCCAG CCCGGCGGCG AGGACCCCAG TTGCTACCCG CCCATTCAGT TGTTAGAAAT TGATTTTGCG GAGCTCACCT TGGAAGAGAT TATTGGCATC GGGGGCTTTG GGAAGGTCTA TCGTGCTTTC TGGATAGGGG ATGAGGTTGC TGTGAAAGCA GCTCGCCACG ACCCTGATGA GGACATCAGC CAGACCATAG AGAATGTTCG CCAAGAGGCC AAGCTCTTCG CCATGCTGAA GCACCCCAAC ATCATTGCCC TAAGAGGGGT ATGTCTGAAG GAGCCCAACC TCTGCTTGGT CATGGAGTTT GCTCGTGGAG GACCTTTGAA TAGAGTGTTA TCTGGGAAAA GGATTCCCCC AGACATCCTG GTGAATTGGG CTGTGCAGAT TGCCAGAGGG ATGAACTACT TACATGATGA GGCAATTGTT CCCATCATCC ACCGCGACCT TAAGTCCAGC AAC (SEQ ID NO:16). This translates to: NSAREDSQVS GDEGWWTGQL NQRVGIFPSN YVTPRSAFSS RCQPGGEDPS CYPPIQLLEI DFAELTLEEI IGIGGFGKVY RAFWIGDEVA VKAARHDPDE DISQTIENVR QEAKLFAMLK HPNIIALRGV CLKEPNLCLV MEFARGGPLN RVLSGKRIPP DILVNWAVQI ARGMNYLHDE AIVPIIHRDL KSSN (SEQ ID NO:17).

The 3' portion of MLK1 was initially cloned by degenerate PCR as previously published (Dorow, *et al*., *Eur. J. Biochem*., **1993,** *213*, 701-710). The protocol for cloning the 3' portion of MLK1 was as described above for MLK2 with the following exceptions. Of the four clones obtained from rescreening the library with the 1.2 kb clone, three of the four clones represented MLK1. None of the clones included the entire kinase domain, which was obtained by PCR.

Phage from 1 ml aliquots of amplified libraries (normal human colonic epithelia and human T84 colonic carcinoma cell line cDNA in 1 Uni-ZAPXR (Stratagene, cat #937204) were lysed by suspending in 20 ml water and snap freezing. A 5 ml sample of the lysed phage was used as a PCR template in two reactions for each library. Primers representing the vectors were taken from nucleotide sequences flanking the cloning sites. In the case of the T84 colonic cell line library, the T3 and T7 sequencing primers (Promega) were used. In each reaction, one primer was from the 3' - 5' strand of the MLK1 gene, approximately 100bp from the 5' end of the known sequence. The second primer was one of the two vector primers. PCR reactions contained IX PCR buffer, 2.5 mM magnesium chloride, IU *Taq* polymerase (all from Bresatec), 0.2 mM dNTP and 0.4 mM each primer in a total of 50 mL. Reaction conditions were 60s at 95°C, 90s at 52°C, 90s at 72°C for 30 cycles with a 15 min extension time in the final cycle. PCR products were cloned and sequenced as a described above. The longest clone from the library screen and a PCR fragment that included additional MLK 1 sequence were ligated together to create a 1.08 kb MLK1 cDNA in pUC 18.

The MLK1 clone from the EST database was provided in the vectorpBluescript (Stratagene). The MLK1 cDNA from the colonic library was ligated into the EST clone by digestion of the former with EcoRI, blunted with Klenow, then cut with AflII. This isolated fragment was cloned into the MLK1 cDNA from the EST database cut with XhoI, blunted with Klenow, and cut with AflII. This new construct was then excised from pBluescript by digestion with Not 1 and Apa 1 and ligated into pcDNA3-EE also cut with Not 1 and Apa1. All cloning junctions were sequence verified.

### Example 21: E.coli expression of GST-MLK3_{KD}

pGEXKG-MLK3(KD) was transformed into *E*. *coli* strain BL21 by electroporation. Bacteria containing the plasmid were inoculated into a 15 liter Applikon fermenter in 10 liter volume of the following rich medium: 1.95 g/L K₂HPO₄, 0.9 g/L KH₂PO₄, 0.1 g/L ampicillin, 0.3 g/L (NH₄)₂SO₄, 0.92 g/L MgSO₄·7H₂O, 42.7 mg/L Na citrate, 21.8 mg/L FeSO₄·7H₂O, 0.5 mL Pichia trace metals (Higgins, *et al*., *Methods Molecular Biology,* **1998,** *103*, 149-177), 20 g/L casamino acids, 40 g/L glycerol, 25.5 mg/L CaCl₂. Bacteria were grown overnight at 800 rpm/68% dissolved oxygen/30°C until the culture reached an OD₆₀₀ = 4.4. Recombinant protein production was induced by the addition of 1 mM isopropyl-β-D-thiogalactoside, with continued fermentation at 25°C for up to 6 hr. Bacteria were then recovered by centrifugation and the cell paste stored frozen at -20°C until purification.

### Example 22: Purification of bacterial GST-MLK3_{KD}

Partially-purified GST-MLK3_{KD} was prepared by sonicating 100 gm of bacterial cell paste in 100 mM Tris-HCl, 150 mM NaCl, 1 mM EDTA, 5 mM dithiothreitol (DTT), pH 7.5 (buffer A). The solution was made 1% with Triton X-100, then stirred on ice for 1 hr. Supernatant solution after centrifugation for 45 min at 20,000 x g was mixed for 1 hr on ice with 10 mL glutathione Sepharose 4B resin (Pharmacia) equilibrated in buffer A. Pelleted resin was washed twice with 12.5x volume buffer A, then eluted with 20 mL 100 mM Tris-HCl, 150 mM NaCl, 5 mM DTT (buffer B), containing 20 mM glutathione, pH 7.5. Protein was dialyzed overnight against buffer B and stored in aliquots at -80°C.

### Example 23: Baculoviral expression of FLAG-MLK3 and GST-MLK3_{KD}

Recombinant baculoviruses expressing the FLAG-MLK3 and GST-MLK3_{KD} were produced from their respective transfer vectors, pFB-FLAG-MLK3 and pFB-GST-MLK3_{KD} using the BAC-TO-BAC system (Life Technologies) according to the instruction manual. Suspension cultures of Sf21 cells (Vaughn, *et al*., *In Vitro*, **1977,** *13*, 213-217) were grown at 27°C/120 rpm in supplemented Grace's medium (Hink, *Nature*, **1970**, *226*, 466-467) with 10% heat-inactivated fetal bovine serum (FBS). To produce recombinant FLAG-MLK3, Sf21 cells at a density of 1.5 x 10⁶ cells/mL supplemented Grace's medium containing 5% FBS were infected with a multiplicity of infection (MOI) of 3.1 and harvested at 39 hr after infection. To produce recombinant GST-MLK3_{KD}, Sf21 cells at a density of 1.5 x 10⁶ cells/mL supplemented Grace's medium containing 5% FBS were infected with an MOI of 2 and harvested at 41 hr after infection. In both cases, pelleted cells were resuspended in buffer comprised of 10 mM HEPES, 50 mM NaCl, 0.5 mM Pefabloc SC, 5 µM pepstatin, 10 µg/mL aprotinin, 10 µg/mL leupeptin, pH 7.4. Supernatant solution after centrifugation for 1 hr at 147,000 xg was readjusted to pH 7.4 with 3 M Tris base and then stored at -70°C prior to purification.

### Example 24: Purification of baculoviral GST-MLK3_{KD}

Partially-purified baculoviral GST-MLK3_{KD} was prepared by glutathione affinity chromatography. For 10 mL of cell extract (26.6 mg total protein), 1 mL of glutathione Sepharose 4B resin (Pharmacia) equilibrated in 10 mM HEPES, 150 mM NaCl, pH 7.4 (buffer C) was added and protein was allowed to bind for 45 min at 4°C. Resin was then washed in column format with 30 column volumes of buffer C, then eluted with 5 column volumes of buffer C containing 20 mM glutathione. Pooled final product was dialyzed overnight against buffer C and stored in aliquots at -70°C.

### Example 25: Purification of baculoviral FLAG-MLK3

Partially-purified baculoviral FLAG-MLK3 was prepared by antibody affinity chromatography. Protein from 15 mL of extract (19.5 mg total protein) with an additional 0.1M NaCl was bound onto a 0.25 mL column of M2 monoclonal FLAG peptide antibody coupled to agarose resin (Sigma) by repeated loading (three times total). Resin had been equilibrated with a 5 column volume wash of 50 mM Tris-HCl, 150 mM NaCl, pH 7.4 (TBS), a 3 column volume wash of 0.1M glycine, pH 3.5, followed by another 5 column volume wash with TBS, prior to chromatography. Recombinant protein was primarily eluted by 5 column volumes of 0.2 mM FLAG peptide (N-Asp-Tyr-Lys-Asp-Asp-Asp-Asp-Lys-C) (SEQ ID NO:18) in TBS. Protein was stored in aliquots at -80°C prior to assay.

### Example 26: Dominant Negative Mutant: A dominant negative mutant of the MLK family blocks death in differentiated PC12 cells following removal of Nerve Growth Factor

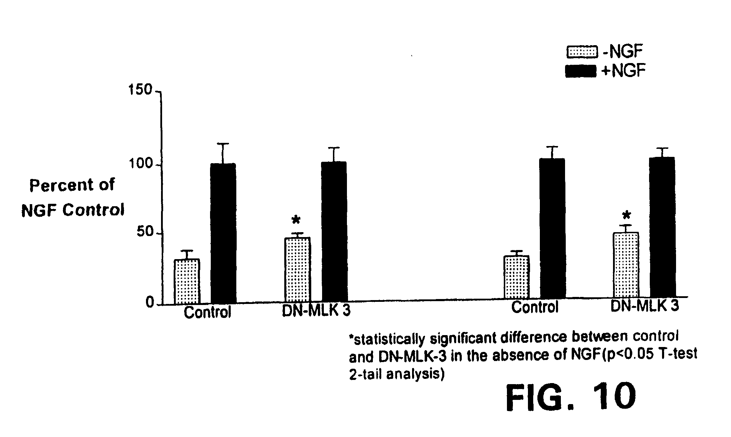

The PC-12 cell line derived from a rat pheochromocytoma tumor has been used extensively as a neuronal cell model for examining the molecular events leading to neuronal death (for review, see Troy, *et al*., *Adv*. *Neurology*, **1997,** 103-111). Nerve Growth Factor (NGF) induces PC-12 cells to differentiate into a sympathetic neuronal phenotype (Greene, *Cell Biol*., **1978,** *78*, 747-755). NGF differentiated PC-12 cells are dependent on NGF for survival and undergo a morphologically described apoptotic death upon removal ofNGF from the culture medium. A cell system was developed to determine the effect of members of the mixed lineage kinase family on PC-12 cell death following NGF withdrawal. PC-12 cells were transfected with cDNA coding for a dominant negative (DN) mutant of MLK-3 using Pfx lipid transfer system as recommended by the manufacturer (Invitrogen, Carlsbad, CA). A stable pool of transfectant expressing DN-MLK-3 was selected using G418 sulfate (Mediatech Inc., Hemdon, VA). Approximately 30% of cells in these pools express DN MLK3 as determined by immunohistochemistry. Pools of cells stably expressing the mutant kinase were plated on polyomithine/laminin (10 ug/ml each in phosphate buffered saline) coated tissue culture 96-well format plates at a density of 2 x 10⁴ cells/well and treated with 100 ng/ml of NGF for 7 days. Medium containing the NGF was removed, the cell monolayer washed with phosphate buffered saline and medium containing neutralizing NGF antibody (cat. #N6655; Sigma, St. Louis, MO) at a final dilution of 1:1000 was replaced for 1-5 days. Cell viability was quantified by a cell viability assay using the conversion of the tetrazolium salt, MTS, to a colored formazan which was read at an absorbance of 570 nm on a CytoFluor 2350 (Millipore, Bedford, MA) as recommended by manufacturer (Promega, Madison, WI). Stable pools expressing DN-MLK-3 were partially rescued from cell death caused by NGF withdrawal (Figure 10).

### Example 27: Assay for enzymatic activity of recombinant MLK protein

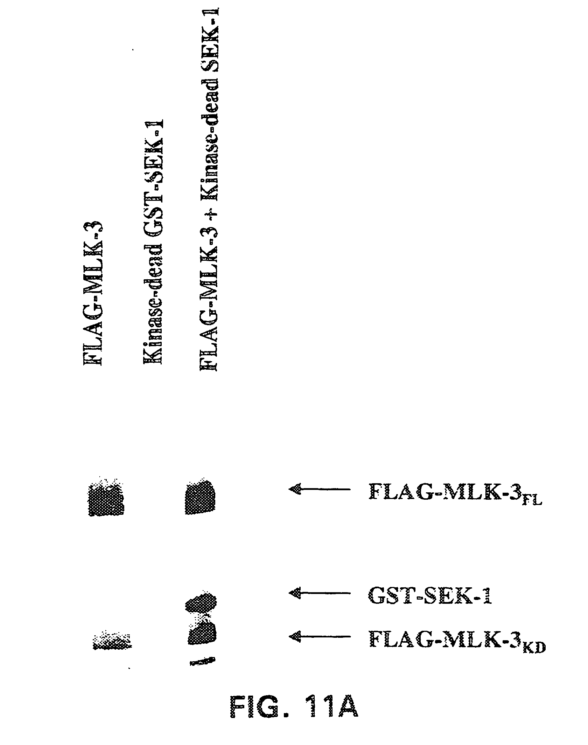

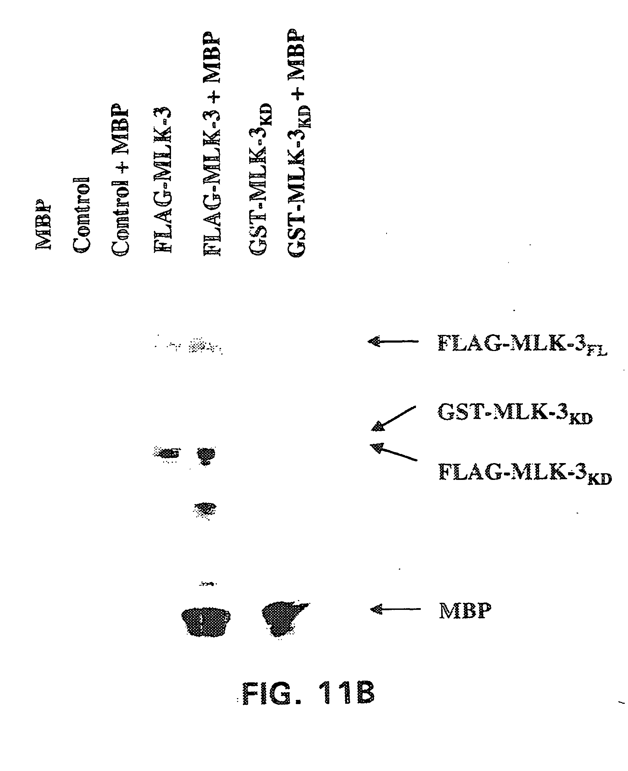

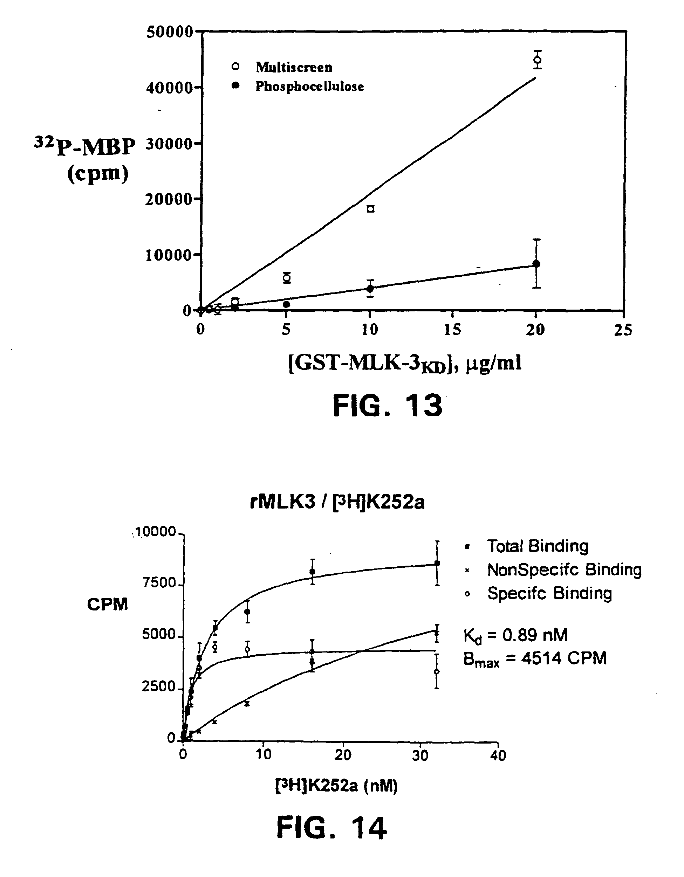

In order to demonstrate that the MLK protein expressed in either the baculovirus or bacterial expression system is enzymatically active, several assay formats may be utilized. The MLK protein may be a full-length construct or a kinase domain expressed in either a baculovirus or bacterial expression system. The assay may be antibody-based such as enzyme-linked immunosorbent assay (ELISA), time-resolved fluorescence (TRF), or fluorescence polarization (FP). The antibody may be monoclonal orpolyclonal with reactivity towards phosphoserine, phosphothreonine, orphospho-specific substrate. Alternatively, anon-antibody-based method may be used such as radioactive gel-based assay (see Figure 11), multiscreen trichloroacetic acid (TCA) precipitation assay (Figure 13), scintillation proximity assay (SPA), flashplate method, or phosphocellulose filter assay format (Figure 13). The assay may be designed to monitor direct phosphorylation of a substrate or a coupled assay system utilizing the downstream kinases in the signaling pathway. The substrate may be a specific substrate such as SEK-1or a relatively non-specific substrate such as myelin basic protein (MBP).

### Example 28: Kinase Assays:

### (1) Radioactive Gel-Based Kinase Assay

The kinase activity of MLK-3 was assayed by monitoring the incorporation of ³²P from [γ-³²P]-ATP into a substrate of MLK (e.g. kinase-dead SEK-1; myelin basic protein). The 50-µl assay mixture contained Buffer A (20 mM MOPS, pH 7.2, 25 mM β-glycerol phosphate, 5 mM EGTA, 1 mM sodium orthovanadate, 1 mM dithiothreitol), 15 mM MgCl₂, 100 µM ATP, 10 µCi [γ-³²P]-ATP, and 0.1 µg kinase-dead SEK-1 substrate (Stressgen, Inc; bound glutathione S-transferase-SEK-1 (GST-SEK-1) was released from glutathione-agarose beads with 10 mM glutathione, pH 8.0) or 25 µg MBP (Sigma Chemical Co.). Reaction was initiated by adding MLK protein (kinase domain or preparation containing both full-length and kinase domain) or control protein. The mixture was incubated for 30 min at 30°C. At the end of the reaction 2x reducing sample buffer was added. The mixture was boiled for 5 min, loaded onto either a 12% SDS-PAGE gel (using MBP as substrate) or 8% gel (SEK-1 as substrate). After electrophoresis, the gel was dried. Quantitation of phosphate incorporation into substrate, SEK-1, was performed on a Molecular Dynamics Phosphorimager (Sunnyvale, CA). Results of experiments designed to show the enzymatic activities of baculovirus-expressed MLK-3 (FLAG-tagged full-length or GST-tagged kinase domain) using kinase-dead GST-SEK-1 or MBP as substrate are shown in Figures 11A and 11B.

### (2) Western Blot Analysis

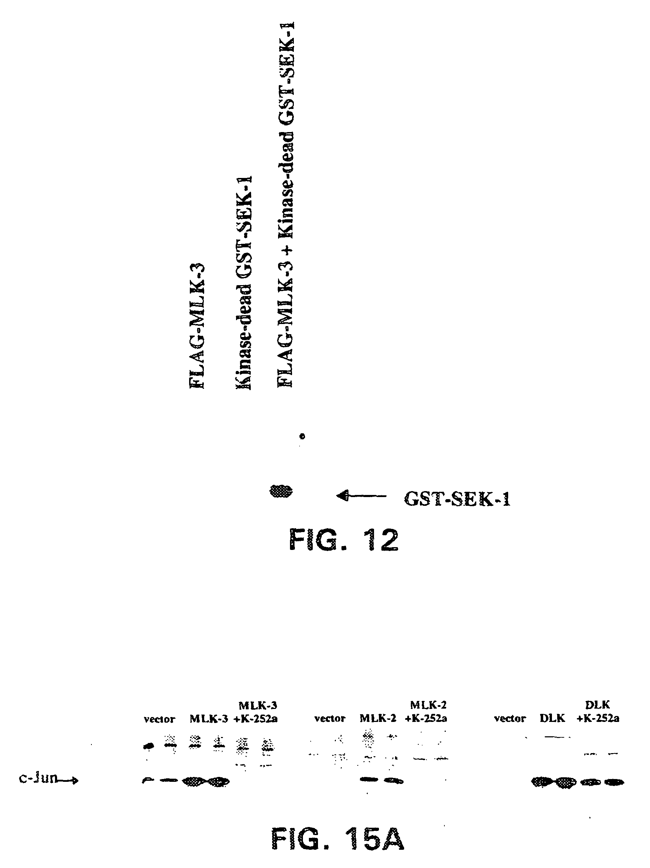

The kinase activity of baculovirus-expressed MLK-3 was examined by immunoblot analysis. The 20-µl assay mixture contained Buffer A, 15 mM MgCl₂, 100 µM ATP, and 0.1 µg kinase-dead SEK-1 substrate. The reaction was allowed to proceed for 30 min at 30°C, then quenched with 10 µl 4x reducing sample buffer. Proteins were separated on a 8% Tris-glycine gel and electrophoretically transferred to Immobilon PVDF membrane. The membrane was incubated with phospho-specific SEK-1 (Thr223) antibody (New England Biolabs, Inc.) followed by horseradish peroxidase-labeled goat anti-rabbit IgG (Bio-Rad). Detection of the immunoreactive bands was performed *via* enhanced chemiluminescence (Amersham). The phosphorylation of kinase-dead GST-SEK-1 by FLAG-MLK-3 protein (baculovirus preparation containing both full-length and kinase domain) is illustrated in Figure 12.

### (3) Multiscreen Trichloroacetic Acid (TCA) Precipitation Assay

The kinase activity of bacterially-expressed GST-MLK-3 kinase domain was assessed using the Millipore Multiscreen trichloroacetic (TCA) "in-plate" assay as described by Pitt, *et al*., *J. Biomol. Screening,* **1996,** *1*, 47-51). Assays were performed in 96-well Multiscreen Durapore plates (Millipore). Each 50-µl assay mixture contained 20 mM Hepes, pH 7.4, 20 mM MgCl₂, 20 mM MnCl₂, 2 mM DTT, 0.1 mM Na₃VO₄, 1 µCi [γ-P³²] ATP and 30 µg MBP substrate. The reaction was initiated by adding MLK protein and allowed to proceed for 15 min at 37°C. The reaction was stopped with 25 µl of 50% TCA. The plates were allowed to equilibrate for 30 min. at 4°C, then washed with ice cold 25% TCA. Scintillation cocktail was added to the plates, and the radioactivity was determined using Wallac MicroBeta 1450 PLUS scintillation counter. The protein dose response versus formation of ³²P-labeled MBP is shown in Figure 13.

### (4) Phosphocellulose Filter Assay

The kinase assay was performed in a 50-µl reaction mixture containing 20 mM Hepes, pH 7.4, 20 mM MgCl₂, 20 mM MnCl₂, 2 mM DTT, 0.1 mM Na₃VO₄, 1 µCi [γ-P³²] ATP and 30 µg MBP. The reaction was initiated by adding MLK protein and allowed to proceed for 15 min. at 37°C. The reaction was stopped with 75 µl of 75 mM phosphoric acid. An aliquot of the quenched solution was loaded directly on the phosphocellulose membrane (Pierce). Alternatively, the 96-well phosphocellulose multiscreen plate (Millipore) may be used. The membranes were washed with 75 mM H₃PO₄. The bound ³²P-labeled phosphorylated MBP was eluted in collection tubes by adding 1 M sodium hydroxide. The radioactivity was determined by Cerenkov counting in a Beckman scintillation counter (Somerset, NJ). The formation of phosphorylated MBP with increasing concentration of bacterially-expressed GST-MLK-3 kinase domain is shown in Figure 13.

### Example 29: Assay to determine binding of compounds to recombinant MLK Family

K-252a (Compound III-3; see, Table 4), an indolocarbazole metabolite of *Nocardia* species, binds to a variety of serine/threonine and tyrosine kinases (Angeles, et al., *Anal. Biochem* **,1996,** *236*, 49-55; Knight, *et al*., *Anal. Biochem.,* **1997,** *247*, 376-381). A tritiated K-252a ligand was used to assess binding to human recombinant full length MLK-3 from a crude preparation ofbaculovirus infected cells. [³H]K-252a was specifically labelled with tritium in the 3 and 9 positions through a contract with NEN Research products (Billerica, MA) and had a specific activity of 40 Ci/mmol. Binding reactions were performed in 1 ml in a 96-well plate. The reaction mixture contained 50 mM MOPS buffer, pH 7, 150mM NaCl, 5 mM MnCl₂, 1 mg/ml BSA, 1 % DMSO and 0.25 nM [³H]K252a. The samples were carried out in triplicate with a concentration of 5 ug/ml of crude baculovirus derived MLK-3. Non-specific binding was defined as binding in presence of unlabeled 1.2 uM K252a and was subtracted from total binding to give specific binding. At this dilution 12-15 % of the total counts were non-specifically bound to protein and 75-85 % of these counts were specifically bound to MLK-3 (Figure 14). All experiments were performed for 2 hrs at 4°C. [³H]K252a/MLK-3 complexes were collected on GF/C Whatman filters using a Brandel harvester, washed with cold MOPS/NaCl buffer and counted on a Wallac Micro Beta counter. A saturation binding experiment was performed to obtain a K_{d} for K252a. An example of the results from one of these experiments is shown (Figure 14). A K_{d} of 0.89 nM (Confidence Limits: 0.2 to 1.5 nM) was obtained.

### Example 30: Intact Cell Assays

### (A) Cos 7 Overexpression System

### Materials

K-252a and derivatives of this compound were provided by Kyowa-Hakko Kogyo Co. Ltd. (Tokyo, Japan) (Kaneko et al., 1997). Compounds were dissolved in cell culture grade dimethyl sulfoxide (DMSO) and stored in the dark at 4°C. All dilutions of compounds were made in Dulbecco's modified Eagle's medium (DMEM) containing 1 % bovine serum albumin. Hemagluttinin (HA) antibody was purchased from BAbCO (Richmond, CA). AP-1 (c-jun) substrate was purchased from Promega (Madison, WI). [γ-³²P]ATP (6000 Ci/mmol) was purchased from Amersham (Arlington Heights, IL).

### Cos7 Cell Culture

Green Monkey Kidney Cos7 cells were obtained from ATCC, Rockville, Maryland (CRL 1651) and maintained in DMEM containing 10 % bovine serum, 2 mM glutamine, 1 mM pyruvate, 50 U/ml penicillin/streptomycin at 37°C in 10% CO₂, 90 % air atmosphere. Cos7 cells were detached for passaging by adding 0.25 % trypsin.

### (1) Overexpression of MLK family members and JNK1 in Cos7 cells

Cos7 cells were plated at 80% confluency and transfected with 2 ug each of cDNA constructs using lipofectamine as recommended by the provider (Gibco BRL, Gaithersburg, MD). A full length cDNA of human MLK-3, MLK-2, or mouse DLK or a partial human MLK-1 as described above, and a full length Hemagluttinin A-tagged human JNK1, kindly provided by J. Silvio Gutkind (NIH, Bethesda, MD), were subcloned into the pcDNA3 vector (Invitrogen, San Diego, CA). After a 48 hr transfection, the cells were treated with 0.025% DMSO or 500 nM of the indicated compounds for 2 hr followed by lysis in 0.4 ml Triton buffer (1% Triton X-100, 50 mM sodium chloride, 10 mM Tris (pH 7.6), 0.1 % bovine serum albumin, 30 uM sodium pyrophosphate, 50 mM sodium fluoride, 20 ug/ml aprotinin, 1 mM phenylmethylsulfonylfluoride, 1 mM sodium vanadate). JNK activity from the lysate was assayed by an immunoprecipitation/kinase assay as described below.

### (2) Immunoprecipitation and Kinase Assay from Whole Cells

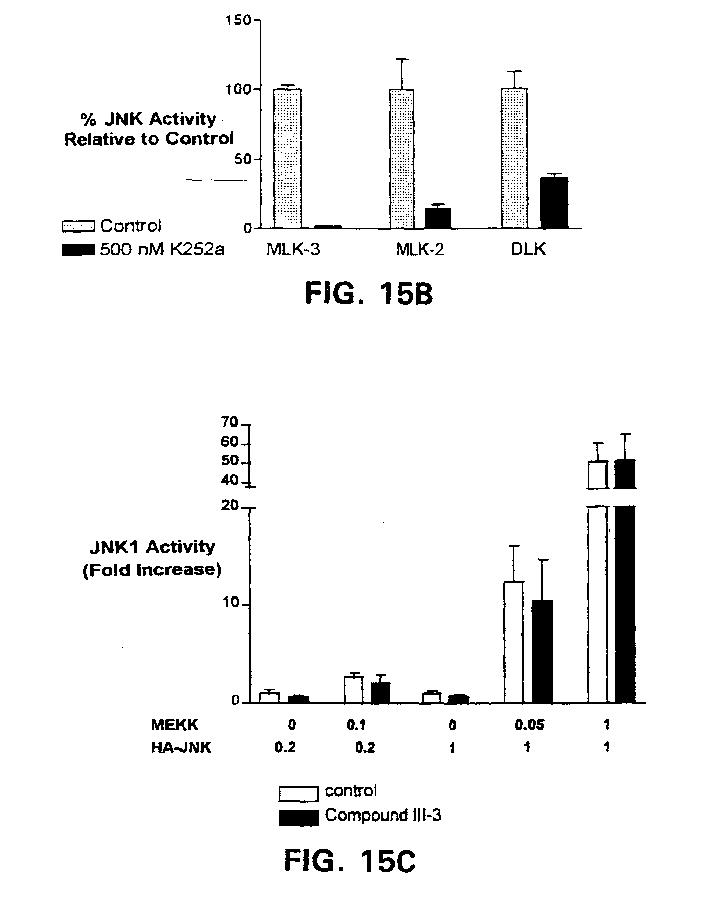

Lysate from Cos 7 cells was measured for protein concentration using the Micro BCA kit from Pierce (Rockford, IL) and equal amounts of protein were immunoprecipitated with the HA antibody for 1 hr at 4°C. Immunoprecipitates were pelleted by centrifugation in a microfuge centrifuge for 20 sec, resuspended in Triton buffer, washed by centrifugation 2 more times, followed by a final wash in Kinase buffer (20 mM Hepes pH 7.4,20 mM MgCl₂, 2 mM dithiothreitol, 0.1 mM sodium vanadate). The immunoprecipitate was resuspended in kinase buffer containing 1 µM ATP and 5 µCi [γ-³²P]ATP and substrate (1 µg/sample of AP-1 ) and incubated for 15 min at 30°C. The kinase reaction was stopped by addition of reducing sample buffer (Laemmli, Nature 1970:227;680-685). Samples were heated to 80°C for 5 min and loaded onto 10% SDS-polyacrylamide gels. Proteins were separated by electrophoresis. The gel was dried and quantitation of radioactivity in the AP-1 substrate was performed on a Molecular Dynamics Phosphorimager (Sunnyvale, Ca.). Results from experiments in which MLK-3, MLK-2 and DLK are co-expressed with HA-JNK1 and incubated in the absence or presence of K-252a are shown in Figures 15A and 15B. In contrast, a derivative of the parental K-252a compound named Compound III-3 (see Table 4), which is a more selective kinase inhibitor, did not interfere with the JNK pathway activated by another MAPKKK upstream of JNK, MEKK1 (Figure 15C).

### (B) Whole-Cell Reporter Assay For MLK activated JNK

Attempts at deriving clones constitutively expressing the MLK family have been unsuccessful, suggesting that overexpression of the MLK's may affect cell survival (Bergeron *et al*., *Biochem. Biophys. Res. Commun*., **1997,** *231*, 153-155; Nagata, *et al*., *EMBO J*., **1998,** *17*, 149-158). Therefore, in developing a whole cell assay for tracking MLK induced biochemical events, a cell line containing a genetically engineered inducible expression system of the kinase of interest may be required. For example, a PC-12 cell line transfected with a tetracycline-controlled transactivator. When cells are further transfected with a gene of interest driven by the inducible promoter *tetO*, expression of that gene is tightly controlled by tetracycline in the medium (Shockett, *et al*., *Proc. Natl*. *Acad*. *Sci. USA,* **1995,** *92*, 6522).

To quantitate the activation of MLK. one can measure the phosphorylation of downstream substrates such as MEK4. JNK or c-jun in multiple assay formats as described above. Another approach to quantitate the MLK activation in whole cells is to use a reporter enzyme activity such as the c-jun luciferase reporter system commercially available through the PathDetect™ system (Stratagene, LaJolla, CA). In this system, the tetracycline-inducible cell line is transfected with two plasmids. One plasmid constitutively expresses a fusion of the cJun NH₂-terminal transactivating domain with the yeast GAL4 DNA binding domain (cJun-DBD fusion protein). The other plasmid carries the coding sequences for firefly luciferase driven by five tandem repeats of the GAL4 binding site. Upon activation of MLK, the downstream substrate of JNK, cJun-DBD fusion protein, is phosphorylated, binds to the GAL4 binding sites, and induces luciferase gene transcription. Luciferase is easily assayed in cell lysates by addition of its substrate (Promega, Madison, WI) and measurement of chemiluminescence.

### Example 31: Association of Inhibition of MLK family members with Motoneuron And Cortical Survival

### Survival of Rat Spinal Cord Cultures Enriched for Motoneurons

Spinal cords were dissected from Sprague-Dawley rat fetuses (Charles River Laboratories, Wilmington. MA) of embryonic age (E) 14.5-15. Cells from only the ventral portion of the spinal cord were dissociated, and further enriched for motoneurons by centrifugation on a 6.5% step metrizamide gradient, as described previously (Henderson, *et al*., **1993),** and were analyzed for purity by staining with low affinity neurotrophin receptor antibody (IgG-192, Boehringer-Mannheim) (data not shown). Cells were seeded onto 96-well plates previously coated with poly-1-omithine and laminin (5 ug/ml each) at a density of 6 x 10⁴ cells/cm² in chemically defined serum-free N2 medium (Bottenstein, *et al*., ***1979***, *supra*). In order to separate attachment from survival effects, addition of compounds to cultures was made after an initial attachment period of 1-3 h. Neuronal survival was assessed after 4 d by using calcein AM (Molecular Probes, Eugene, OR) in a fluorometric viability assay (Bozyczko-Coyne, *et al*., **1993,** *supra*). Microscopic counts of neurons correlated directly with relative fluorescence values. In brief, culture medium was serially diluted in DPBS (Dulbeccos phosphate buffered saline) and a final concentration of 6 uM calcein AM stock was then added to each 96-well. The plates were incubated for 30 min at 37°C, followed by serial dilution washes in DPBS. The fluorescent signal was read using a plate-reading fluorimeter from Millipore (Cytofluor 2350) at excitation=485 nm and emission = 538 nm. For each plate, mean background derived from wells receiving calcein AM, but containing no cells, was subtracted from all values. Linearity of the fluorescence signal was verified for the concentration and incubation time for the range of cell densities in these experiments. An example of the percent survival above control of motoneurons in the presence of test compounds at 250 nM is shown in Table 3.

### Survival Of Cortical Neurons

Cerebral cortices were dissected from embryonic day 18 rat fetuses and enzymatically digested to obtain a single cell suspension. Cells were seeded at a density of 1.56 x 105/cm2 onto poly-ornithine/laminin coated 96 well tissue culture plates in serum-free Neural Basal Medium containing B27 supplements. Plates were coated with a solution of poly-ornithine/laminin (8ug/ml each) made in PBS for atleast 2hrs at 37oC. On *in vitro* days 5-7, cortical neurons were exposed to Ab25-35 (20uM) either in the presence or absence of test compounds. Ab25-35 (Sigma, St. Louis, MO) stock solutions (1mM) were prepared in deionized-distilled sterile H₂O. Relative neuronal survival was determined at 48hrs post-peptide addition using lactate dehydrogenase (LDH) release as an indicator of plasma membrane integrity/cell viability. LDH was measured using the Cytotoxicity Detection Kit (Boehringer-Mannheim, Indianapolis, IN) in accordance with the manufacturer's instructions. Data is expressed as percent inhibition of LDH released relative to cultures treated with Ab25-35 alone.

**Table 3**

| | **Cortical Neurons** | **Motoneurons** | | **Cos-7 Cells** | **Cos-7 Cells** | **Cos-7 Cells** | **Cos-7 Cells** |
|---|---|---|---|---|---|---|---|
| Formula | Survival % Control at 250 nM | survival % Control at 250 nM | % JNK Inhib. at 500 nM | DLK % JNK Inhib. @ 500nM | MLK-3 % JNK Inhib. @ 500nM | MLK-2 % JNK Inhib. @ 500nM | MLK-1 % JNK Inhib. @ 500nM |
| III¹ | 46,56 | 300 % | 65 % | 63, 73 | 99,98 | 89,67 | 97,96 |
| III² | 47,80 | 315 % | 88 % | 36,22, 42 | 94,94 | 69,44 | 92, 64 |
| I³ | 22,54 | 177 % | 88 % | 20, 25 | 94, 93 | 0 | 79, 29 |
| I⁴ | 29,39 | 165 % | 97 % | 58, 13, 52, 8 | 84,92, 90 | 0 | 63,38 |

| | | | | | | | |
|---|---|---|---|---|---|---|---|
| ¹Compound has formula III where Z₁, Z₂, R₁, and R₂ are H; X is CO₂CH₃; and R is OH. | | | | | | | |
| ²Compound has formula III where Z₁ and Z₂ are H; X is CO₂CH₃; R₁ and R₂ are CH₂SCH₂CH₃; and R is OH. | | | | | | | |
| ³Compound has formula I where A₁, A₂, R₁, R₃, R₅, and R₆ are H; B₁ and B₂ together represent O; R₂ is CH₂CH₂OAc; R₄ is CH₂CH₂(2-Pyridyl); and X is CH₂. | | | | | | | |
| ⁴Compound has formula I where A₁, A₂, R₁, R₃, R₅, and R₆ are H; B₁ and B₂ together represent O; R₂ is H; R₄ is CH₂CH₂(2-Pyrimidinyl); and X is CH₂. | | | | | | | |

### Example 32: Immunoprecipitation of Endogenous JNK Activity from motoneuron cultures in the Absence or Presence of Indolocarbazoles or Fused Pyrrolocarbazoles

Purified motorneurons were plated at a density of 6 x 10⁴ cells/cm² in 16 mm diameter wells. Cells were allowed to attach for 2 hours prior to treatment. Cells were treated with either 0.0125 % DMSO or 500 nM compound for 2 hrs in N2 defined medium. Cells were then rinsed with ice cold phosphate buffered saline followed by lysis in 0.4 ml Triton buffer as described above in example 30. Lysate from motoneuron cultures was normalized to cell number and immunoprecipitated with a JNK1 antibody (cat. # sc-474) purchased from Santa Cruz Biotechnology (Santa Cruz, CA). JNK activity from the immunoprecipitates was assayed in the presence of ³²P-ATP and c-jun substrate as described above. The profile of inhibitory activity of the 4 test compounds was compared in motoneurons and in Cos7 cells overexpressing either DLK, MLK-1, MLK-2 or MLK-3 (Table 3).

### Example 33: Correlation between inhibition of MLK3-induced JNK activity in Cos7 cells and cholineacetyl transferase activity in primary embryonic cultures

To determine whether inhibition of the JNK pathway regulated by these kinases correlated with neurotrophic compounds, we evaluated the effect of compounds on JNK activity in Cos7 cells overexpressing HA-JNK and MLK3. After a 48 hr transfection period, the cells were incubated with compounds at 500 nM for 2hr followed by cellular lysis. Lysate was immunoprecipitated and kinase activity measured as previously described. The results are reported as percent inhibition of control sample where control is JNK activity in the presence of DMSO. As can be seen in Table 4, most compounds which were active in spinal cord and/or basal forebrain ChAT activity were potent inhibitors of MLK-3 activation of JNK.

**Table 4**

| Effect of Indolo- and Indeno- carbazoles on JNK activity in Cos7 cells overexpressing MLK3 | | | |
|---|---|---|---|
| | **Cholineacetyltransferase Activity** | | **% Inhibition of JNK Activity (average)** |
| **Compound** | **Spinal Cord** | **Basal Forebrain** | **MLK3 in Cos7 cells** |
| III-1¹ | + | + | 84 |
| III-2² | + | + | 96 |
| III-3³ | + | + | 94 |
| I-1⁴ | + | + | 93 |
| I-2⁵ | + | + | 85 |
| I-3⁶ | + | + | 93.5 |
| I-4⁷ | - | + | 95 |
| I-5⁸ | - | + | 97 |
| I-6⁹ | - | + | 58 |
| I-7¹⁰ | - | + | 85.5 |
| III-4¹¹ | - | + | 66 |
| III-5¹² | - | + | 96 |
| III-7¹³ | - | + | 54 |
| I-8¹⁴ | + | - | 89 |
| III-8¹⁵ | + | - | 94 |
| III-9¹⁶ | + . | + | 98.5 |
| III-10¹⁷ | + | - | 78 |
| I-9¹⁸ | + | - | 88 |
| I-10¹⁹ | + | - | 94 |
| III-11²⁰ | - | - | 92.5 |
| I-11²¹ | - | + | 33 |
| I-12²² | - | - | 11 |
| I-13²³ | - | - | 1 |

| | | | |
|---|---|---|---|
| ¹ Compound having formula III where Z₁ and Z₂ are H; X is CO₂CH₃; R₁ is NHCONHC₂H₅; R₂ is CH₂CH₂(2-Pyridyl); and R is OH. | | | |
| ² Compound having formula III where Z₁ and Z₂ are H; X is CO₂CH₃; R₁ and R₂ are CH₂OCH₂OCH₂CH₃; and R is OH. | | | |
| ³ Compound having formula III where Z₁ and Z₂ are H; X is CO₂CH₃; R₁ and R₂ are CH₂SCH₂CH₃; and R is OH. | | | |
| ⁴ Compound having formula I where A₁, A₂, R₁, R₃, and R₄ are H; B₁ and B₂ together represent O; R₂ is CH₂CH₂OH; R₅ and R₆ are OCH₃; and X is CH₂. | | | |
| ⁵ Compound having formula I where A₁, A₂, R₁, R₃, R₅, and R₆ are H; B₁ and B₂ together represent O; R₂ is CH₂CH₂OAc; R₄ is Br; and X is CH₂. | | | |
| ⁶ Compound having formula I where A₁, A₂, R₁, R₃, R₅, and R₆ are H; B₁ and B₂ together represent O; R₂ is CH₂CH₂OAc; R₄ is CH₂CH₂(2-Pyridyl); and X is CH₂. | | | |
| ⁷ Compound having formula I where A₁, A₂, R₁, R₃, R₄, R₅, and R₆ are H; B₁ and B₂ together represent O; R₂ is CH₂CH₂OH; and X is CH₂. | | | |
| ⁸ Compound having formula I where A₁, A₂, R₁, R₃, R₄, R₅, and R₆ are H; B₁ and B₂ together represent O; R₂ is CH₂CH₂CH₂OH; and X is CH₂. | | | |
| ⁹ Compound having formula I where A₁, A₂, R₁, R₂, R₃, R₄, R₅, and R₆ are H; B₁ and B₂ together represent O; and X is S. | | | |
| ¹⁰ Compound having formula I where A₁, A₂, R₁, R₃, R₄, R₅, and R₆ are H; B₁ and B₂ together represent O; R₂ is CH₂CH₂CH₂NHCO(4-(OH)Ph); and X is CH₂. | | | |
| ¹¹ Compound having formula III where Z₁, Z₂, R₁, and R₂ are H; X is CO₂(CH₂)₄CH₃; and R is OH. | | | |
| ¹² Compound having formula III where Z₁, Z₂, and R₁, are H; R₂ is CH₂OH; X is CO₂CH₃; and R is OH. | | | |
| ¹³ Compound having formula III where Z₁ and Z₂ together form =O; R₁ and R₂ are Br; X is CO₂CH₃; and R is OH. | | | |
| ¹⁴ Compound having formula I where A₁, A₂, R₁, R₃, R₅, and R₆ are H; B₁ and B₂ together represent O; R₂ is H; R₄ is CH₂CH₂(2-Pyrimidinyl); and X is CH₂. | | | |
| ¹⁵ Compound having formula III where Z₁, and Z₂ are H; R₁ is Br; R₂ is I; X is CO₂CH₃; and R is OH. | | | |
| ¹⁶ Compound having formula III where Z₁, Z₂, R₁, and R₂ are H; X is CO₂CH₃; and R is OH. | | | |
| ¹⁷ Compound having formula III where Z₁, and Z₂ are H; R₁ and R₂ are CH₂CH₂SCH₃; X is CO₂CH₃; and R is OH. | | | |
| ¹⁸ Compound having formula I where A₁, A₂, R₁, R₂, R₃, R₅, and R₆ are H; B₁ and B₂ together represent O; R₄ is CH₂CH₂(2-Pyridazinyl); and X is CH₂. | | | |
| ¹⁹ Compound having formula I where A₁, A₂, R₁, R₃, R₅, and R₆ are H; B₁ and B₂ together represent O; R₂ is H; R₄ is CH₂CH₂(2-Pyridyl); and X is CH₂. | | | |
| ²⁰ Compound having formula III where Z₁, Z₂, R₁, and R₂ are H; X is CO₂CH₃; and R is OCH₃. | | | |
| ²¹ Compound having formula I where A₁, A₂, R₁, R₃, R₄, R₅, and R₆ are H; B₁ and B₂ together represent O; R₂ is (CH₂)₃-NH-C(-O)-3,5-dihydroxyphenyl; and X is CH₂. | | | |
| ²² Compound having formula I where A₁, A₂, R₁, R₃, R₄, R₅, and R₆ are H; B₁ and B₂ together represent O; R₂ is benzoyl; and X is CH₂. | | | |
| ²³ Compound having formula I where A₁, A₂, R₁, R₂, R₃, R₅, and R₆ are H; B₁ and B₂ together represent O; R₄ is CH=CH-C≡N; and X is CH₂. | | | |

### Example 34: Gel shift assay for MLK activation:

Activation of MLKs can lead in induction of *c-jun* transcription, resulting in increased c-Jun protein. The increased amount of c-Jun protein can be measured by a standard assay, identified as a gel shift assay. Garner, *et al*., *Nucleic Acids Res.,* **1981,** *9*, 3047-3060, which is incorporated herein by reference in its entirety. Radiolabeled double-stranded DNA oligomers, that code for a c-Jun DNA-binding site, are incubated with a nuclear cell extract followed by acrylamide gel electrophoresis and quantitation of the radiolabeled DNA shifted to a slower mobility. This represents the portion of DNA that is bound to the c-Jun protein and is directly proportional to the amount of c-Jun protein in the extract.

Activation of MLKs can also induce c-Jun phosphorylation. This can be detected using antibodies which specifically recognize the phosphorylated form of the protein in detection systems such as, for example, Western blots or ELISAs.

### Example 35: Survival of Chick Embryonic Neurons

### Materials

Leibovitz's L15 media, glucose, sodium bicarbonate, trypsin and antibiotics were from Gibco. Muscle extract was prepared as described (Henderson, *et al*., *Nature*, **1983,** *302*, 609-611, which is incorporated herein by reference in its entirety). All other reagents were from Sigma, unless otherwise indicated.

### Cell culture

Motoneurons (embryonic day 5.5) were isolated with an immunological method according to the procedure set forth in Bloch-Gallego, *et al*., *Development,* **1991,** *111*, 221-232, which is incorporated herein by reference in its entirety, with modifications as described in Weng, *et al*., *NeuroReport*, **1996,** *7*, 1077-1081, which is incorporated herein by reference in its entirety. Purified motoneurons were seeded onto 35 mm tissue culture dishes (Nunc) pre-coated with poly-DL-omithine and laminin (1 µg/ml, Upstate Biotech). The culture medium was L15 with sodium bicarbonate (22.5 mM), glucose (20 mM), progesterone (2x10⁻⁸ M), sodium selenite (3x10⁻⁸M), conalbumin (0.1 mg/ml), insulin (5 µg/ml), penicillin-streptomycin, and 10% heat-inactivated horse serum. Muscle extract was supplemented at 30 µg/ml. Compound III-3 was prepared as a 4mM stock solution in DMSO and stored protected from light at 4°C. The final concentration of DMSO in treated and control cultures was 0.125%.

Paravertebral sympathetic ganglia (SG; embryonal day 12 (E12)), dorsal root ganglia (DRG;E9), and ciliary ganglia (CG;E8) were dissected from chick embryos at the indicated embryonic day as described in *Lindsay, et al*., *Dev. Biol*., **1985,** *112*, 319-328, which is incorporated herein by reference in its entirety. After trypsinization and dissociation, the nerve cell suspensions were plated onto polyomithine-laminin-coated culture dishes in Ham's F14 culture medium, supplemented with 10% horse serum. Immediately after plating, survival factors were added at the following concentrations: Nerve growth factor (NGF), 20 ng/ml; ciliary neutrophic factor (CNTF), 10 ng/ml. The cultures were maintained at 37° and 5% CO₂ in a humidified environment.

### Cell counting

Neurons were plated in 35 mm culture dishes with grids (Nunc). Selected areas of each dish comprising together about 10% of the surface scanned for the presence of phase bright cells immediately after plating and again after 48 h to assess survival percentages. Cell survival was confirmed by vital staining with trypan blue (not shown).

### Intact DRG

Ganglia were placed in 96 well plates previously coated with poly-1-omithine and laminin (5 µg each/ml phosphate buffered saline) in serum-free N2 medium (Bottenstein, *et al*., *Proc*. *Natl*. *Acad. Sci. USA*, **1979,** *76*, 514-517, which is incorporated herein by reference in its entirety) containing 0.05% bovine serum albumin (BSA) and maintained for 48 h at 37° and 5% CO₂ in a humidified environment. Treated ganglia received either 250 nM Compound III-3 or 20 ng/ml NGF 2 h after plating.

### Compound III-3 supports the survival of chick embryonic peripheral neurons in a concentration-dependent fashion

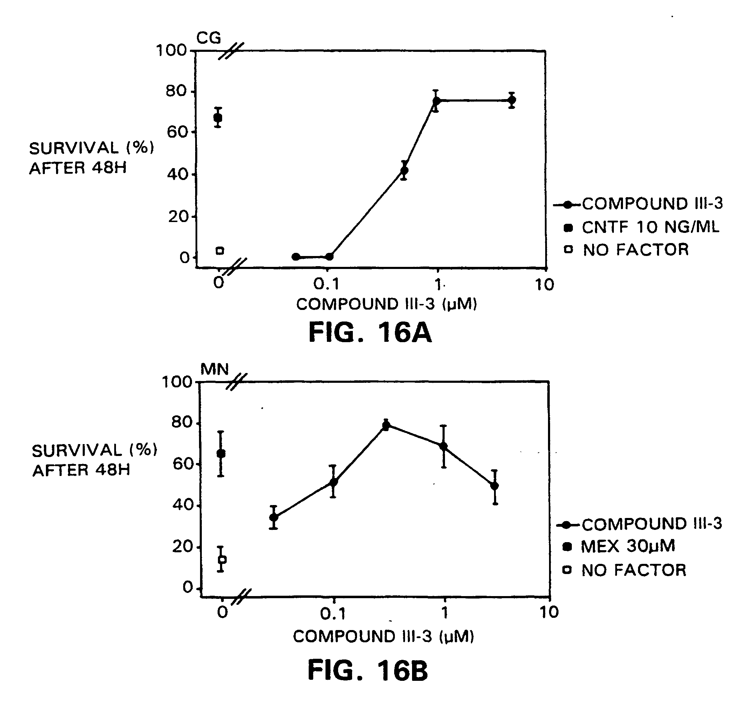

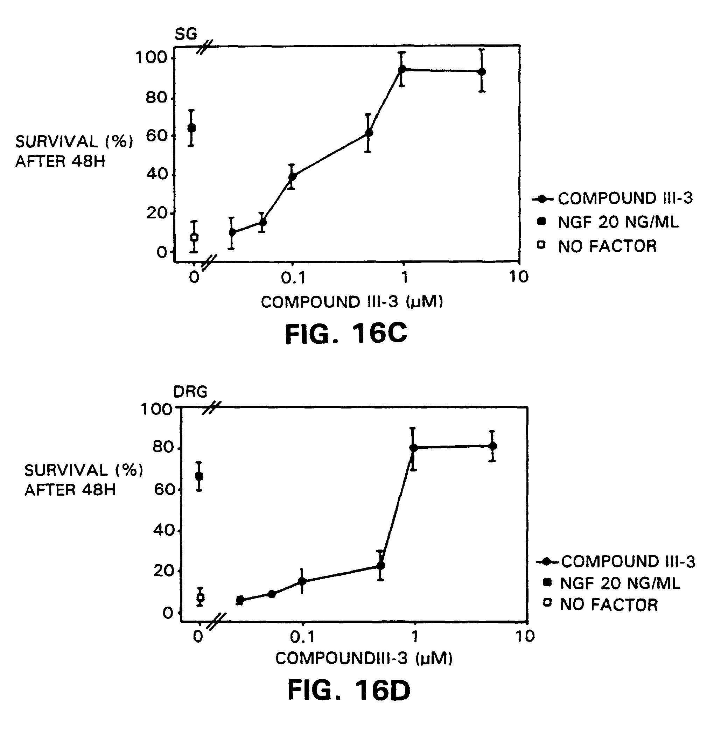

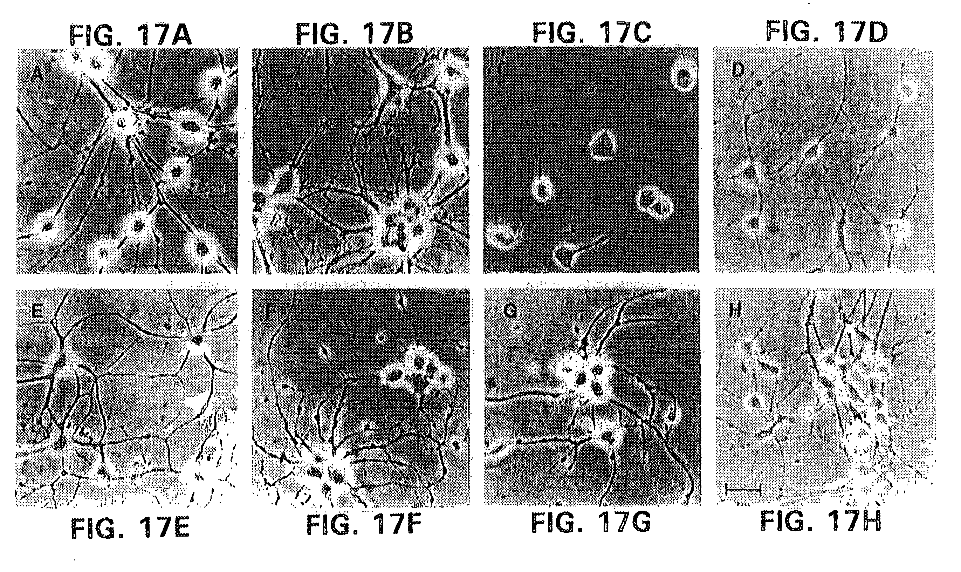

Withdrawal of NGF from dissociated cultures of E9 dorsal root ganglion sensory neurons (DRG) and E 12 sympathetic ganglion neurons (SG) cause them to undergo PCD within 48 h. This was prevented by addition of Compound III-3 to the culture medium at the time of NGF withdrawal. AT 1 µM, Compound III-3 kept 94% of SG neurons and 890% of DRG neurons alive after 48 h (NGF-treated control: SG 65%, DRG 66%). Similarly, Compound III-3 promoted the survival of 76% of CNTF-dependent ciliary ganglion (CG) neurons after 24 h (CNTF-treated control 67%). In the presence of 10% serum, the survival effects of Compound III-3 were concentration-dependent, with a plateau reached around 1 µM for all three neuronal populations (Fig. 16A-C). The surviving neurons showed extensive neurite outgrowth with thicker and more curved neurites as compared to control cultures. (Fig. 17 E-H). After four days, the survival promoting activity was still intact: DRG: Compound III-3 52%, growth factor-treated control 41 %; SG:Compound III-3 83%, NGF-treated control 55%; CG:Compound III-3 58%, CNTF-treated control 50%). Under optimal conditions, the cultures could be maintained with Compound III-3 for one week and longer (not shown).

### Compound III-3 supports the survival of chick embryonic motoneurons in a concentration-dependent fashion

Cultured chick motoneurons can survive and extend processes in the presence of muscle extract, whereas they die rapidly in its absence. In our experiments, after 48 h, 65% of the motoneurons survived in the presence of muscle extract, in contrast to 14% of untreated controls. In serum-free conditions, the survival effect of Compound III-3 was maximal at 300 nM, and was somewhat higher (79%) than that induced by muscle extract. The concentration-dependency of the survival effect of Compound III-3 in this system is different than in peripheral neurons, since Compound III-3 concentrations above 300 nM showed a progressively reduced effect (Fig. 16A). This might indicate a particular sensitivity of motoneurons to some aspect of Compound III-3 activity. Morphologically, motoneurons rescued with Compound III-3 exhibited phase bright cell bodies and were able to extend long neurites, which appeared slightly thicker than those induced by muscle extract (Fig. 17). After four days in culture, 56% of the motoneurons were alive with Compound III-, compared with 42% with muscle extract. At 300 nM, Compound III-treated neurons survived in vitro for at least a week (not shown).

### Compound III-3 promotes neurite outgrowth from intact dorsal root ganglia

Results from the above experiments demonstrate that Compound III-3 not only promotes survival of embryonic neurons from the peripheral and central nervous systems, but also resulted in robust neurite outgrowth. Many of these extensions appeared to be thicker than those elicted in the presence of growth factors (compare Fig. 17 A-D to Fig. 17 E-H). This effect was also observed in the neuritic outgrowth elicited from intact embryonic dorsal root ganglia cultured in the presence of 250 nM Compound III-3 (Fig. 18C). Neurites grew in response to both NGF (Fig. 18B) and Compound III-3; those elicited by NGF were much finer and more branched than those grown in the presence of Compound III-3 which appeared thick and possibly fasciculated.

### Example 36: In Vivo Treatment

### Developmentally Regulated Motor Neuronal Death in the Chick Embryo

The present example is described in detail in Glicksman, *et al*., *J. Neurobiol.,* **1998,** *35*, 361-370, which is incorporated herein by reference in its entirety. On E6, a window in the shell of chick eggs (Spafas, Preston, CT) was made and either vehicle (5% Solutol ™ HS 15, polyethylene glycol 660 hydroxysterate; BASF Aktiengesellschaft, Ludwigshafen, Germany (in phosphate-buffered saline, pH 7.2)) or the specified dose of Compound III-3 in the vehicle was applied directly onto the vascularized chorioallantoic membrane-once daily from E6 to E9 as described in Oppenheim, *et al*., *Science*, **1991,** *251*, 1616-1617, which is incorporated herein by reference in its entirety). Embryos were sacrificed at E10 and their spinal cords were removed, fixed in Carnoy's solution (10% glacial acetic acid, 60% absolute ethanol, 30% chloroform), processed for serial paraffin sections, and stained with thionin. Every 20th section of lumbar segments 1-8 was counted according to previously described criteria (Clarke, *et al*., *Methods In Cell Biology: Cell Death,* **1995,** Schwart & Osborne, Eds., Academic Press, New York, pp.277-321, which is incorporated herein by reference in its entirety).

### Developmentally Regulated Motor Neuronal Death in the Neonatal Rat

Untimed pregnant Sprague-Dawley rats were obtained from Harlan Laboratories (Indianapolis, IN). Female rat pups were injected daily, subcutaneously (SC), over the target perineal muscles, with Compound III-3 in 5% Solutol ™ HS 15 or vehicle along starting on the day of birth (P1) and continuing for 5 days (P5). On P10 or P60, pups were decapitated, blood was collected in heparinized capillary tubes, and the region of the spinal cord containing the sexually dimorphic spinal nucleus of the bulbocavemosus (SNB) and the perineal area containing the bulbocavernosus (BC) and levator ani (LA) muscles were dissected after perfusion of the animals with saline/formalin. The region of the spinal cord containing the SNB was postfixed, embedded in Paraplast, sectioned at 10µm, and stained with Cresylecht violet (Nordeen, *et al*., *Science,* **1985,** *229*, 671-673, which is incorporated herein by reference in its entirety). Motor neurons were counted at X500 in serial sections from the lumbar 5 to the sacral 1 region of the spinal cord as described previously (Nordeen, *et al*., *supra*). The microscopic enumeration was made on coded sections by an observer blinded to the treatment groups. Motor neuron counts were corrected for cell size and section thickness (Konisgsmark, *Contemporary Research Methods in Neuroanatomy*, Nauta & Ebbesson, Eds., **1970,** Springer-Verlag, New York, pp.315-340, which is incorporated herein by reference in its entirety) and statistical analysis was by one-way analysis ofvariance (ANOVA). Perineal musculature was postfixed, decalcified, embedded in Paraplast, sectioned at I 0µm and stained with Milligan's Trichrome. Using bright-field microscopy (X250), BC and LA muscles in normal females and Compound III-3-treated females (405 animals/group) were positively identified by both their location and the presence of striated fibers. The outline of muscle tissue was traced from alternate sections using a projection microscope (62.5), and the cross-sectional area was measured using a digitizing pad and a computer-based morphometry system (Sigmascan, Jandel Scientific). Muscle volume was calculated by talking the total cross-sectional area and multiplying it by the section thickness, and corrected for the percentage of the structure sampled.

Collected blood was centrifuged for 5 min at room temperature; then, plasma was removed and frozen at -20°C. Serum testosterone levels (6-7 animals/group) were measured by radioimmunoassay following the procedures set forth in Wingfield, *et al*., *Steroids*, **1975,** *26*, 311-327, which is incorporated herein by reference in its entirety.

### Axotomy-Induced Motor Neuronal Dedifferentiation in the Adult Rat

The left hypoglossal nerve was transected in the neck of adult female Sprague-Dawley rats (120-180 g) under Neumbutol anesthesia, and 50 µl of Compound III-3 or its vehicle (5% Solutol™ HS 15) were applied to a piece of Gelfoam ™ (AJ Buck, Owings Mills, MD), then wrapped around the proximal end of the transected nerve. After 7 days, the animals were anesthetized and perfused with 4% paraformaldehyde in Sorenson's buffer, 0.07 M phosphate, pH 7.2. The brain stem was removed and 40- µm-thick serial coronal sections were cut on a cryostat (Chiu, *et al*., *NeuroReport,* **1994,** *5*, 693-696, which is incorporated herein by reference in its entirety). Every fifth section was processed for ChAT immunohistochemistry as previously described (Chiu, *et al*., *J. Comp. Neurol.,* **1993,** *328*, 351-363, which is incorporated herein by reference in its entirety) using a 1:350 dilution of an anti-ChAT monoclonal antibody obtained from Chemicon. Cells that stained clearly above background were counted in stained sections; the number of enumerated cells was expressed as the ratio of the number of ChAT-immunoreactive cells on the axotomized side of the hypoglossal nucleus versus the number of immunoreactive cells on the control (uninjured) side.

Compound III-3 rescued rat embryo motor neurons from apoptotic death *in vitro* and inhibited a signaling pathway resulting in JNK1 activation in these cells (Maroney, *et al*., *J. Neurosci.,* **1998,** *18,* 104-111, which is incorporated herein by reference in its entirety). To determine potential activity *in vivo,* Compound III-3 was assessed in two models of developmentally regulated programmed motor neuronal death and in a model of axotomy-induced dedifferentiation in adult motor neurons. In chicks, approximately 50% of the spinal cord motor neurons undergo PCD during ES-10 (Hamburger, *et al*., *J. Neurosci*., **1982,** *1*, 38-55;-Purves, *et al*., *Body and Brain: A Trophic Theory of Neural Connections,* **1988,** Harvard University Press, Cambridge, MA, both of which are incorporated herein by reference in its entirety). Application of Compound III-3 to the chorioallantoic membrane during this period prevented motor neuronal death in a dose-dependent manner (Fig. 19). Forty percent of the motor neurons that would normally die were rescued a the two highest doses tested (2.3 and 7 µg/day), while 25% of the motor neurons were rescued at lower doses (1.2 and 1.8 µg/day) (Fig. 19).

During early perinatal life of female rats (late embryonic stage until postnatal day (PN) 4), more than 50% of the motor neurons in the SNB are eliminated via PCD (Breedlove, *J*. *Neurobiol.,* **1986,** *17,* 157-176, which is incorporated herein by reference in its entirety). In males, motor neurons in this nucleus innervate striated penile muscles involved I copulatory reflexes. Testicular secretion of androgenic steroids reduces SNB motor neuronal death in males and prevents much of the atrophy of the BC and LA muscles innervated by the neurons. Administration of testosterone to female pups resulted in a fully masculine number of SNB motor neurons (Nordeen, *et al*., *supra*) and prevented BC and LA muscle atrophy (Waiman, *et al*., *Endocrinology,* **1941,** *29*, 955-978, which is incorporated herein by reference in its entirety). Daily sc administration of Compound III-3 (PN 1-5) to female rats significantly attenuated motor neuronal death (Fig. 20A). Rescue of the SNB motor neuronal death by Compound III-3 occurred at two doses (0.5 and 1 mg/kg per day). At the maximally effective doses of 0.5 and 1 mg/kg per day, administration of Compound III-3 resulted in a 70% enhancement in motor-neuronal survival which equaled the effect of testosterone (Fig. 20A). Compound III-3 did not alter plasma testosterone levels of treated females. Radio immune measurement of plasma testosterone levels in the 1-mg/kg per day group resulted in no significant difference when compared to the vehicle control group (0.016 ± 0.008 ng/mL and 0.029 ± 0.015 ng/ML standard error of the mean (S.E.M.), respectively).

To determine whether the Compound III-3 treatment was effective in long-term maintenance of motor neuron survival, females were treated with Compound III-3 (0.5 and 1 mg/kg per day) for the same time period PN (1-5). One half of the animals in the vehicle and both treatment groups were sacrificed on PN10. The remaining animals were then maintained without additional Compound III-3 treatment until sacrifice at PN60. As previously observed (Fig. 20A), Compound III-3 treatment resulted in a 70% enhancement in motor neuronal survival (Fig. 20B). Furthermore, 100% of these rescued motor neurons were identifiable morphologically 55 days after the last treatment with Compound III-3 (Fig. 20B). Compound III-3 inhibition of motor neuronal death during the neonatal period permitted motor neuronal survival into adulthood.

Despite the clear demonstration and devastating effects of motor neuronal loss in adult human diseases such as amyotrophic lateral sclerosis adult motor neurons in most animal models of motor neuronal injury are resistant to death. However, axonal injury does result in morphological (Oppenheim, *et al*., *supra*) as well as biochemical changes (Oppenheim, *et al*., *supra*; Rende, *et al*., *J*. *Comp*. *Neurol*., **1992**, *319,* 285-298, which is incorporated herein by reference in its entirety; Chiu, *et al*., *J*. *Comp. Neurol*., **1993,** *328,* 351-363, which is incorporated herein by reference in its entirety) in adult motor neurons that may mimic degenerative change preceding death in diseased in degenerating motor neurons. One example of this type of change results form axotomy of the hypoglossal nerve that innervates the tongue. Unilateral transection of this nerve in the adult rat resulted in the loss of 95% of the ChAT-immunoreactive hypoglossal motor neurons in the ipsilateral nucleus after 7 days (Chiu, *et al*., *NeuroReport,* **1994**, *5*, 693-696, which is incorporated herein by reference in its entirety). The loss in ChAT immunoreactivity was not permanent. Four weeks following axotomy, 100% of the motor neurons had recovered control levels of ChAT immunoreactivity (Borke, *et al*., *J*. *Neurocytol*., **1993**, *22*, 141-153, which is incorporated herein by reference in its entirety). ChAT immunoreactivity in the contralateral hypoglossal motor neurons was not affected (Chiu, *et al*., *supra*) (Fig. 21 and Table 5).

When applied in Gelfoam ™ to the proximal end of the hypoglossal nerve, Compound III-3 dose-dependently attenuated the decrease in ChAT immunoreactivity in ipsilateral hypoglossal motor neurons assessed 7 days postaxotomy. The maximally effective dose (50 µg) resulted in 40% more ChAT-immunoreactive motor neurons compared to the axotomized, untreated control (Fig. 21 B and Table 5). There was a bell-shaped dose dependence with both lower and higher doses resulting in survival greater than the untreated control, but less than that achieved at 50 µg. As was true with the SNB model, there was no associated weight loss, mortality, or gross tissue damage in these animals at any doses tested.

In three separate models of motor neuron degeneration *in vivo*, Compound III-3 demonstrated neuroprotective activity: developmentally-regulated PCD of lumbar spinal cord motor neurons in embryos (Fig. 19), androgen-sensitive death of postnatal SNB motor neurons (Fig. 20), and axotomy-induced loss of a functional marker, ChAT, in adult hypoglossal motor neurons (Fig. 21 and Table 5). Compound III-3 was efficacious when administered peripherally by sc injection, applied locally to the cut end of a nerve, or directly overlaid on the chick embryo chorioallantoic membrane. In contrast to the parent molecule K-252a, Compound III-3 was approximately fivefold more potent in mediating survival in motor neuron-enriched cultures (data not shown) and did not exhibit inhibitory activity against trkA tyrosine kinase and several serine threonine kinases (Maroney *et al*., *supra*; Kaneko, *et al*., *J. Med. Chem*., **1997,** *40,* 1863-1869, which is incorporated herein by reference in its entirety).

**Table 5**

| **Effext of Compound III-3 On Choline Acetyltransferase Immunoreactivity** **In Axotomized Hypoglossal Motor Neurons** | | | | |
|---|---|---|---|---|
| | | ChAT-Positive Motor Neurons | | |
| Treatment | n | Experimental/Control | % | Average/Group |
| vehicle | 2 | 20/544 | 3.68 | 4.01 |
| | | 19/437 | 4.35 | |
| 3.6 µg III-3 | 2 | 55/420 | 13.10 | 12.84* |
| | | 72/572 | 12.59 | |
| 25 µg III-3 | 2 | 95/597 | 15.91 | 19.01* |
| | | 142/642 | 22.12 | |
| 50 µg III-3 | 2 | 188/484 | 38.84 | 41.34* |
| | | 278/637 | 43.85 | |
| 100 µg III-3 | 4 | 465/920 | 50.54 | 32.61* |
| | | 235/784 | 29.98 | |
| | | 178/770 | 23.12 | |
| | | 182/679 | 26.80 | |
| 200 µg III-3 | 2 | 99/461 | 21.48 | 24.96* |
| | | 159/559 | 28.44 | |
| sham operated | 2 | 350/335 | 104.48 | 101.24 |
| | | 292/298 | 98.00 | |

| | | | | |
|---|---|---|---|---|
| Compound III-3 or vehicle were added in gel foam to the proximal end of the hypoglossal nerve immediately following its transection. After 7 days, animals were sacrificed and serially sectioned through the hypoglossal nucleus, and every fifth section was immunostained with anti-ChAT antibodies. Counts of ChAT-positive neurons were made in the ipsilateral (experimental) and contralateral (control) sides of the nucleus *p<0.05, statistically significant compared to control vehicle-treated animals. | | | | |

### An Inhibitor of the MLK-3 pathway demonstrates in vivo efficacy and blocks phosphorylation events downstream of MLK-3 In The MPTP Model

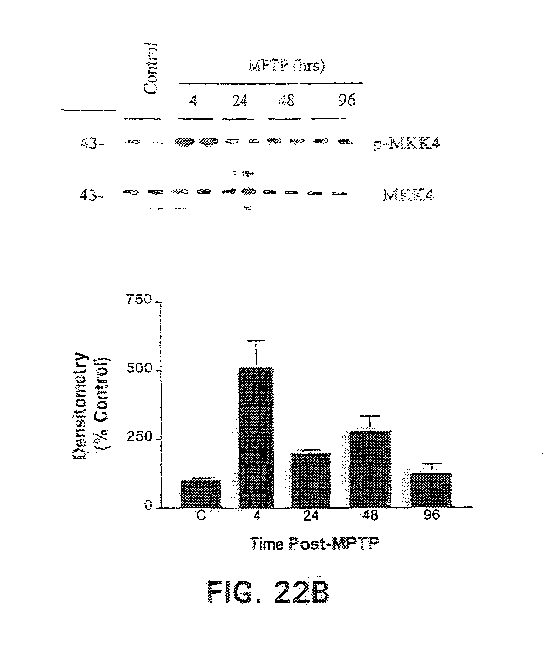

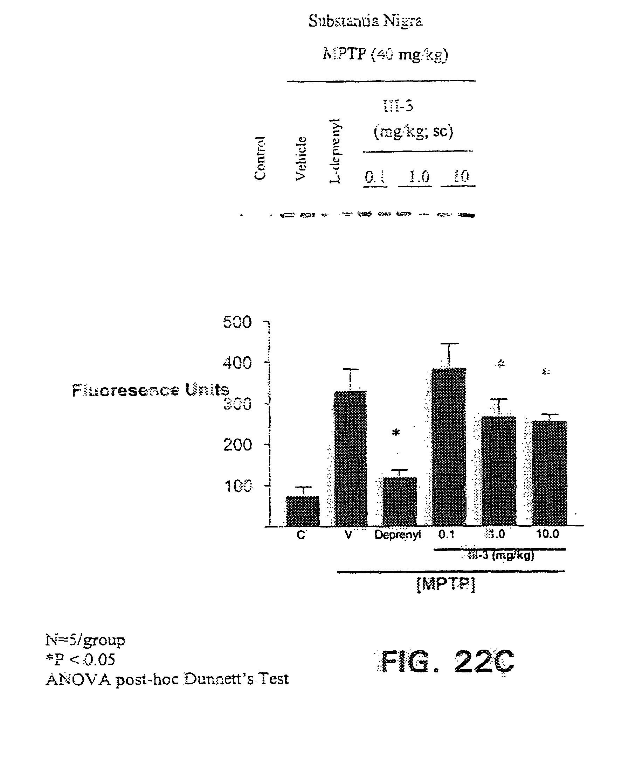

MPTP was administered at a dose (40 mg/kg) that produces loss of striatal dopaminergic terminals and cell bodies in the substantia nigra. Tyrosine hydroxylase was used as a marker for dopamenergic nerve terminals in the substantia nigra. Systemically admininstered Compound III-3 attenuated the loss of substantia nigra tyrosine hydroxylase immunoreactive neurons after MPTP lesion (Fig. 22a; Saporito et al., 1999). Since Compound III-3 is a known inhibitor of MLK3, activation of a downstream substrate of MLK3 was measured in MPTP- treated mice. Levels of phosphorylated MKK4 were measured using a phospho-MKK4 specific antibody (New England Biolabs, Beverly, MA) that recognizes the monophosphorylated form of MKK4 by either immunoblot (Fig. 22b) or ELISA (Fig. 22c). MPTP administration elevated levels of phosphorylated MKK4 in the substantia nigra by up to 5 fold over control levels (Fig. 22b). Peak elevations occurred 4 hrs after administration ofMPTP and coincided with peak CNS levels of MPP⁺. MPTP-mediated MKK4 phosphorylation was attenuated by pretreatment with 1-deprenyl, indicating that these phosphorylation events were mediated by MPP⁺ (Fig. 22c). Moreover, MKK4 phosphorylation was partially inhibited with Compound III-3 pretreatment at a dose (1 mg/kg) that produces protection against MPTP-induced nigrostriatal dopaminergic loss (Fig. 22c). These data demonstrate that MPTP (MPP⁺) activates MKK4, a downstream substrate of MLK3. Moreover, these data demonstrate that a known inhibitor of MLK3, inhibits activation of this kinase pathway *in vivo.*

### Example 37: Inflammation

### The induction of IL-1 and TNF-a by LPS in THP-1 cells and the effect of indolocarbazoles and pyrrolocarbazoles on their induction

Cells of the immune system were chosen since many kinases are involved in the regulation of numerous immunological functions, e.g., the induction of the synthesis of cytokines and the induction of a cytokine's biological response. A recent report (Hambleton, *et al*., *Proc. Natl. Acad. Sci. USA*, **1996,** *93,* 2774-2778, which is incorporated herein by reference in its entirety) showed that the treatment of monocyte-derived cell lines with LPS caused a rapid activation of JNK activity. When monocytes come in contact with bacterial endotoxins such as lipopolysaccharide (LPS) they produce the inflammatory cytokines, IL-1 and TNF-α. Inhibition of production of these two cytokines may be a useful treatment of certain inflammatory disorders of the immune system. These cytokines can be easily measured by commercial ELISA kits. We designed experiments to determine (1) if indolo- and fused pyrrolocarbazoles can inhibit the synthesis of IL-1 and TNF-α in our monocyte cell line THP-1, (2) if JNK is activated by LPS in THP-1 cells, and (3) if the activation of JNK by LPS can be inhibited by indolo- and fused pyrrolocarbazoles.

### Experimental Procedures

THP-1 cells were grown in RPMI 1640 medium supplemented with 10% fetal bovine serum. LPS (E.coli serotype 0111.B4, TCA extracted) was purchased from Sigma and dissolved in PBS. ELISA kits for assaying IL-1 and TNF-α were purchased from Boerhinger-Mannheim and assays on THP-1 culture medium were performed as directed by the manufacturer. Standard curves according to directions were obtained with each assay.

Experiments were performed in 12 well culture plates with either 1 or 2 ml of THP-1 cells at 4 X 10⁵ cells/ml. IL-1 and TNF-α were induced by the addition of LPS to the culture medium and the medium collected at various times thereafter for cytokine assay. Cells were removed by centrifugation and the supernatants frozen at -70°C until assay. To minimize costs experiments were performed in duplicate cultures and the duplicate supernatants were pooled after centrifugation. Each pooled supernatant was assayed in duplicate. Stock solutions of indolo- and fused pyrrolocarbazoles in 100% DMSO were diluted to the desired concentrations in either medium containing 10% fetal bovine serum or in medium containing 0.5 mg/ml BSA. Unless otherwise stated, compounds were added to the THP-1 cells 1 hr prior to the addition of LPS.

Assays for JNK activity were performed after immunoprecipitating the JNK protein from an extract of lysed THP-1 cells. Pelleted THP-1 cells were lysed on ice for 15 min in 500 µl of Frac buffer (10 mM Tris-Hcl, pH 7.5,50 mM NaCl, 30 µM sodium pyrophosphate, 1 mg/ml BSA, 1% Triton-X-100). The extract was centrifuged for 10 min at 14K and 5 µl of JNK antibody (Santa Cruz) was added to the supernatant. The extract was rotated for 60 min at 4°C, 75 µl of washed protein A Sepharose (20% w/v in Frac) added and the extract rotated another 30 min to bind the antibody complex to the protein A Sepharose. The protein A Sepharose was washed twice with Frac buffer, once with 20 mM Hepes, pH 7.6, 20 mM MgCl₂, 2 mM DTT, then incubated for 15 min at 30° C in 30 µl of kinase buffer (20 mM hepes, 20 mM MgCl₂, 2 MM DTT, 1 µg recombinant c-jun, and 2 µM ATP-γ-³²P, 2 µCi. The reaction was terminated by the addition of 10 µl of 4X SDS gel loading buffer, heated for 3 min at 80° C, and the proteins were analyzed on a 10% SDS gel. The gel was dried, exposed to a Phosphorimager plate, and the radioactive bands were analyzed on a Phosphorimager.

Results from initial experiments indicated that LPS at 2 µg/ml gave the maximum yield of IL-1 and this concentration of LPS was used in all experiments thereafter. The minimum time after addition of LPS for maximum yield of the cytokines was determined by taking aliquots of medium for assay at various times after the addition of LPS. The first experiment indicated that both IL-1 and TNF-α attained maximum yield at less than 5 hr after the addition of LPS. Since the earliest collection time was 2.4 hr in the first experiment, a second experiment was performed with medium collections starting at 15 min after the addition of LPS. The results of this experiment where only TNF-α was assayed showed that it attained maximum yield at 3 hr after the addition of LPS. No significant TNF-α was found in the medium until 90 min after LPS addition.

The rapid attainment of maximum yield indicated a very tight regulation of the synthesis of the 2 cytokines - rapid synthesis and rapid down regulation. Cultures of cells were treated for 30 min prior to the addition of LPS with either Actinomycin D, a RNA synthesis inhibitor, or cycloheximide, a protein synthesis inhibitor. Medium was collected 3 hr after the addition of LPS and TNF-α was assayed. Both new RNA and new protein synthesis are required for TNF-α induction since no TNF-α was found in the medium of cells treated with either inhibitor. The next experiments were performed to determine if Compound III-3 would inhibit the induction of IL-1 and TNF-α. Compound III-3 inhibited the induction of both IL-1 and TNF-α with IC50 values of 267 nM and 139 nM respectively. The results of these experiments were obtained with cells in medium containing 10% fetal bovine serum. Since the assays with spinal cord tissue and basal forebrain tissue for the neurotrophic activity of compounds are performed in serum-free medium (500 µg/ml BSA) it was of interest to determine the IC50 values for the inhibition of IL-1 and TNF-α in serum-free medium. When THP-1 cells were treated with Compound III-3 in serum-free medium (500 µg/ml BSA) the IC50 was reduced 10 fold from 269 nM to 23 nM. Unless otherwise stated all experiments performed hereafter were performed in serum-free medium. The inhibition by Compound III-3 of the induction of IL-1 and TNF-α in THP-1 cells suggests that Compound III-3 might be useful as a therapeutic in treating pathological conditions caused by the production of above normal quantities of these cytokines. Septic shock is such a condition. Septic shock is caused by the growth of gram negative bacteria in the circulation which in turn release large amounts of the endotoxin, LPS. The LPS then stimulates primarily the monocytes and macrophages to produce large quantities of IL-1 and TNF-α which then cause massive tissue damage and in many cases death.

Several compounds were tested for their ability to inhibit TNF-α and compared with the ability to inhibit JNK. Results are shown in Table 6.

**Table 6**

| | THP-1 Cells | | Overexpressed MLK3 in Cos 7 Cells |
|---|---|---|---|
| Compound | TNF-α IC50 nM | JNK % inh. 500 nM | JNK % inh. 500 nM |
| III-1 | 49.5 | 93.5 | 83.8 |
| III-3 | 29 | 93 | 94 |
| I-2 | >5000 | 78.5 | 85 |
| 1-3 | 366 | 80.5 | 93.7 |
| I-4 | 75.5 | 79.5 | 95 |
| 1-5 | 514 | 89 | 97.2 |
| 1-6 | 817.5 | 77.5 | 57.8 |
| I-7 | 1009 | 74 | 85.5 |
| III-4 | 462.5 | 81 | 66 |
| III-5 | 4 | 84.5 | 96 |
| III-7 | 590.5 | 11.5 | 54 |
| III-8 | 11.5 | 51 | 94 |
| III-10 | 4298 | 48 | 78 |
| I-10 | 4500 | 62 | 94 |
| III-11 | 686 | 51 | 92.5 |

### Effect of Compound III-3 on the induction of Il-2 in Jurkat cells

Experiments were performed to determine if Compound III-3 inhibited the induction of I1-2 in Jurkat cells.

### Experimental Procedures

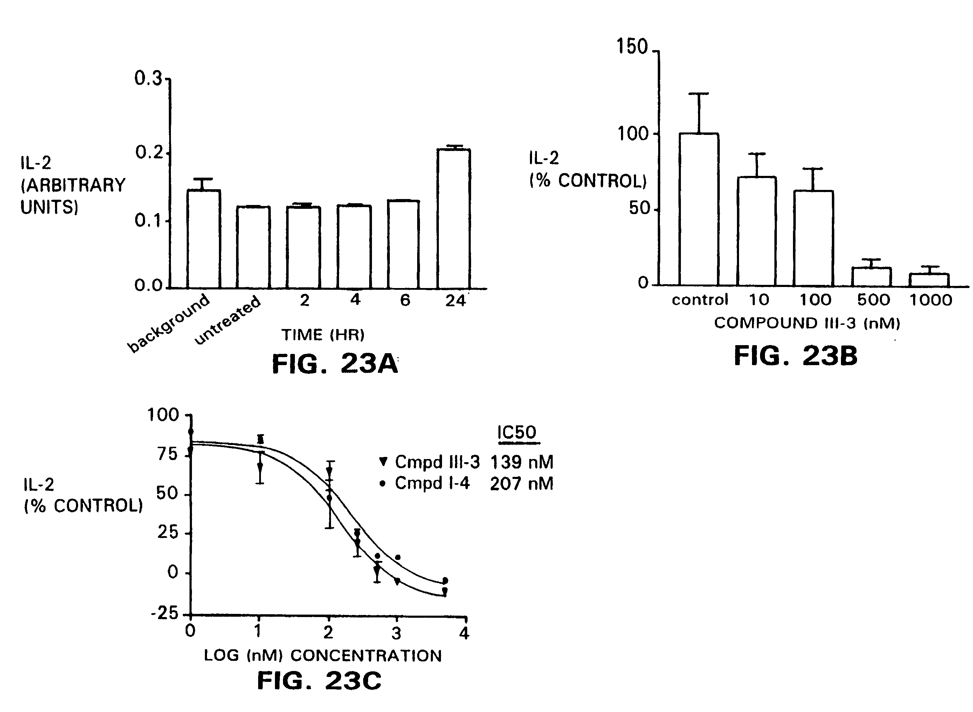

Jurkat cells were grown in RPMI 1640 medium supplemented with 10% fetal bovine serum. TNF-α was from Promega and anti CD3 and anti CD28 antibodies were from Pharmigen. Jurkat experiments were done in 200 µl in a 96 well plate. IL-2 was measured with an ELISA kit purchased from Boehringer Mannheim. The antibodies to CD3 and CD28 were allowed to bind to the plastic of the 96 well plate (18 hr in PBS) prior to addition of the Jurkat Cells. Cells were treated with compounds 1 hr prior to adding to the antibody coated plate. Antibodies to CD3 and CD28 were used to activate the T cell receptor and induce IL-2. I1-2 was released from the Jurkat cells between 6 hr and 24 hr after initiation of induction (Fig. 23A). No IL-2 was made constitutively (Fig. 23A CNT). The effect of Compound III-3 (1 hr treatment with Compound III-3 prior to induction) on IL-2 induction was next assessed (Fig. 23B). A Compound III-3 concentration of 500 nM inhibited IL-2 induction by greater than 80% (Fig. 23B). A more extensive dose response experiment was performed with Compound III-3 and with Compound I-4 which yielded IC₅₀ values of 139 nM for Compound III-3 and 207 nM for Compound I-4 (Fig. 23C).

## Claims

1. A method for identifying a compound which modulates activity of a multiple lineage kinase protein and promotes cell survival or cell death comprising the steps of:
(a) contacting *in vitro* said cell containing said multiple lineage kinase protein with said compound;
(b) determining whether said compound increases or decreases activity of said multiple lineage kinase protein; and
(c) determining whether said compound promotes cell survival or cell death.

2. The method of claim 1 for identifying a compound which promotes cell survival comprising the step of determining whether said compound decreases activity of said multiple lineage kinase protein.

3. The method of claim 1 for identifying a compound which promotes cell death comprising the step of determining whether said compound increases activity of said multiple lineage kinase protein.

4. The method according to claim 2, wherein said compound may be useful in the treatment of a neurodegenerative disorder or inflammation.

5. The method of claim 2 or claim 4 wherein said protein is selected from the group consisting of multiple lineage kinase 1, multiple lineage kinase 2, multiple lineage kinase 3, leucine zipper bearing kinase, dual leucine zipper bearing kinase, and multiple lineage kinase 6.

6. The method of claim 3 wherein said protein is selected from the group consisting of multiple lineage kinase 1, multiple lineage kinase 2, multiple lineage kinase 3, leucine zipper bearing kinase, dual leucine zipper bearing kinase, and multiple lineage kinase 6.

7. The method of claim 5 or claim 6 wherein said protein activity is determined by an *in vitro* kinase assay or binding assay.

8. The method of claim 5 or claim 6 wherein said cell is a neuronal cell.

9. The method of claim 5 wherein said protein activity is determined by measuring the activity or phosphorylation state of a substrate of said protein.

10. The method of claim 6 wherein said protein activity is determined by measuring the activity of a substrate of said protein.

11. The method of claims 9 or 10 wherein said substrate is selected from the group consisting of JNK1, JNK2, JNK3, ERK1, ERK2, p38α, p38β, p38γ, p38δ, MEK1, MEK2, MKK3, MKK4 (SEK1), MEK5, MKK6, MKK7, jun, ATF2, ELK1, and the mammalian homolog of AEX-3.

12. The method of claim 5 wherein said protein activity is determined by measuring the activity of a substrate of said protein, amount of a substrate of said protein, or mRNA encoding said substrate of said protein.

13. The method of claim 6 wherein said protein activity is determined by measuring the activity of a substrate of said protein, amount of said protein, or mRNA encoding said protein.

14. The method of claim 5 or claim 6 wherein said promotion of cell survival is determined by using cells at risk of dying and comparing the amount of living cells which were contacted with said compound with the amount of living cells which were not contacted with said compound.

15. The method of claim 5 or 14 wherein said cells are primary embryonic motoneuron cells.

16. The method of claim 5 or 14 wherein said cells overexpress said multiple lineage kinase protein.

17. The method of claim 5 wherein said promotion of cell survival is determined by observing a decrease in apoptosis.

18. The method of claim 6 wherein said promotion of cell death is determined by observing an increase in apoptosis.

19. The method of any one of claims 5 or 6 wherein said cell is involved in a neurodegenerative disease.

20. The method of claim 2 wherein said cell is involved in inflammation.

21. The use of a compound having the formula: 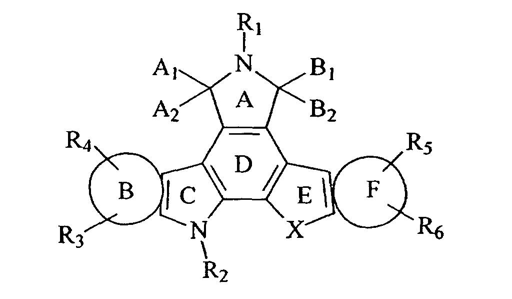 which inhibits a multiple lineage kinase protein in the manufacture of a medicament for use in the treatment of a neurodegenerative disorder or inflammation,
wherein
ring B and ring F, independently, each together with the carbon atoms to which they are attached, form either
an unsaturated 6-membered carbocyclic aromatic ring in which from one to three carbon atom(s) may be replaced by nitrogen atom(s); or
an unsaturated 5-membered carbocyclic aromatic ring in which either
one carbon atom is replaced with an oxygen, nitrogen, or sulfur atom;
or
two carbon atoms are replaced with a sulfur and nitrogen atom, or an oxygen and nitrogen atom;
A¹ and A² together represent O, and B¹ and B² together represent O;
R¹ is H, alkyl of 1 - 4 carbons (inclusive), aryl, arylalkyl, heteroaryl, and heteroarylalkyl; COR⁹, where R⁹ is alkyl of 1 - 4 carbons (inclusive), or aryl, preferably phenyl or naphthyl; -OR¹⁰, where R¹⁰ is H or alkyl of 1 - 4 carbons (inclusive); -CONH₂, -NR⁷R⁸, -(CH₂)ₙNR⁷R⁸, where n is an integer of 1 - 4 (inclusive); or -O(CH₂)ₙNR⁷R⁸; and either
R⁷ and R⁸ independently are H or alkyl of 1 - 4 carbons (inclusive); or
R⁷ and R⁸ are combined together to form a linking group of the general formula -(CH₂)₂-X¹-(CH₂)₂-, where X¹ is O, S or CH₂;
R² is H, -SO₂R⁹; -CO₂R⁹, -COR⁹, alkyl of 1 - 8 carbons (inclusive), preferably an alkyl of 1 - 4 carbons (inclusive), alkenyl of 1 - 8 carbons (inclusive), preferably an alkenyl of 1 - 4 carbons (inclusive), or alkynyl of 1 - 8 carbons (inclusive), preferably an alkynyl of 1 - 4 carbons (inclusive); or a monosaccharide of 5 - 7 carbons (inclusive) where each hydroxyl group of the monosaccharide independently is either unsubstituted or is replaced by H, alkyl of 1 - 4 carbons (inclusive), alkylcarbonyloxy of 2 - 5 carbons (inclusive) or alkoxy of 1 - 4 carbons (inclusive); and either
each alkyl of 1 - 8 carbons (inclusive), alkenyl of 1 - 8 carbons (inclusive), or alkynyl of I - 8 carbons (inclusive) is unsubstituted; or
each alkyl of 1 - 8 carbons (inclusive), alkenyl of I - 8 carbons (inclusive), or alkynyl of 1 - 8 carbons (inclusive) independently is substituted with 1 - 3 aryl of 6 - 10 carbons (inclusive), preferably phenyl or naphthyl; heteroaryl, F, Cl, Br, I, -CN, -NO₂, OH, -OR⁹, -O(CH₂)ₙNR⁷R⁸, -OCOR⁹, -OCONHR⁹, O-tetrahydropyranyl, NH₂, -NR⁷R⁸, -NR¹⁰COR⁹, -NR¹⁰CO₂R⁹, -NR¹⁰CONR⁷R⁸, -NHC(=NH)NH₂, -NR¹⁰SO₂R⁹, -S(O)_{y}R¹¹, where R¹¹ is H or alkyl of 1 - 4 carbons, aryl of 6 - 10 carbons, preferably phenyl or naphthyl, or heteroaryl and y is 1 or 2; -SR¹¹, -CO₂R⁹, -CONR⁷R⁸, -CHO, COR⁹, -CH₂OR⁷, -CH=NNR¹¹R¹², -CH=NOR¹¹, -CH=NR⁹, -CH=NNHCH(N=NH)NH₂, -SO₂NR¹²R¹³, -PO(OR¹¹)₂, or OR¹⁴ where R¹⁴ is the residue of an amino acid after the hydroxyl group of the carboxyl group is removed;
and either
R¹² and R¹³ independently are H, alkyl of 1 - 4 carbons (inclusive), aryl of 6 - 10 carbons, preferably phenyl or naphthyl, or heteroaryl; or
R¹² and R¹³ are combined together to form a linking group, preferably -(CH₂)₂-X¹-(CH₂)₂;
each R³, R⁴, R⁵ and R⁶, independently is H, aryl, preferably an aryl of 6 - 10 carbons (inclusive), more preferably phenyl or naphthyl; heteroaryl; F, Cl, Br, I, -CN, CF₃, -NO₂, OH, -OR⁹, -O(CH₂)ₙNR⁷R⁸, -OCOR⁹, -OCONHR⁹, NH₂, -CH₂OH, -CH₂OR¹⁴, -NR⁷R⁸, -NR¹⁰COR⁹, -NR¹⁰CONR⁷R⁸ -SR¹¹, -S(O)_{y}R¹¹ where y is 1 or 2; -CO₂R⁹, -COR⁹, -CONR⁷R⁸, -CHO, -CH=NOR¹¹, -CH=NR⁹, -CH=NNR¹¹R¹², -(CH₂)ₙSR⁹, where n is an integer of 1 - 4 (inclusive), -(CH₂)ₙS(O)_{y}R⁹, -CH₂SR¹⁵ where R¹⁵ is alkyl of 1 - 4 carbons (inclusive); -CH₂S(O)_{y}R¹⁴, -(CH₂)ₙNR⁷R⁸, -(CH₂)ₙNHR¹⁴, alkyl of 1 - 8 carbons (inclusive), preferably alkyl of 1 - 4 carbons (inclusive); alkenyl of 1 - 8 carbons (inclusive), preferably alkenyl of 1 - 4 carbons (inclusive); alkynyl of 1 - 8 carbons (inclusive), preferably alkynyl of 1 - 4 carbons (inclusive); and either
each alkyl of 1 - 8 carbons (inclusive), alkenyl of 1 - 8 carbons (inclusive) or alkynyl of 1 - 8 carbons (inclusive) is unsubstituted; or
each alkyl of 1 - 8 carbons (inclusive), alkenyl of 1 - 8 carbons (inclusive) or alkynyl of 1 - 8 carbons (inclusive) is substituted as described in (d)(2) of claim 58;
X is either
an unsubstituted alkylene of 1 - 3 carbons (inclusive); or
X is an alkylene of 1 - 3 carbons (inclusive) substituted with one R² group, preferably OR¹⁰, -SR¹⁰, R¹⁵, where R¹⁵ is an alkyl of 1 - 4 carbons (inclusive); phenyl, naphthyl, arylalkyl of 7 - 14 carbons (inclusive), preferably benzyl; or
X is -CH=CH-, -CH(OH)-CH(OH)-, -O-, -S-, -S(=O)-, -S(=O)₂-, -CR¹⁰)₂-, -C(=O)-, -C(=NOR¹¹)-, -C(OR¹¹)(R¹¹)-, -C(=O)CHR¹⁵)-, -CHR¹⁵)C(=O)-, -C(=NOR¹¹)CHR¹⁵)-, -CHR¹⁵)C(=NOR¹¹)-, -CH₂Z-, -Z-CH₂-, -CH₂ZCH₂-,
where Z is, C(OR¹¹)(R¹¹), O, S, C(=O), C(=NOR¹¹), or NR¹¹;
or
A¹ and A² together are each independently H, H; H, -OR¹¹; H, -SR¹¹; H, -NR¹¹R¹²; or together represent =S or =NR¹¹; B¹ and B² together represent O; and each R¹, R², R³, R⁴, R⁵, R⁶ and X are as defined in (c), (d), (e), and (f) of claim 58;
or
A¹ and A² together represent O, and B¹ and B² together are each independently H, H; H, -OR¹¹, H, -SR¹¹, H, -NR¹¹R¹², or together represent =S or =NR¹¹; and each R¹, R², R³, R⁴, R⁵, R⁶ and X are as defined in (c), (d), (e), and (f) of claim 58.

22. The use of claim 21 wherein A₁, A₂, R₁, R₃, and R₄ are H; B₁ and B₂ together represent O; R₂ is CH₂CH₂OH; R₅ and R₆ are OCH₃; and X is CH₂.

23. The use of claim 21 wherein A₁, A₂, R₁, R₃, R₅, and R₆ are H; B₁ and B₂ together represent O; R₂ is CH₂CH₂OAc; R₄ is Br; and X is CH₂.

24. The use of claim 21 wherein A₁, A₂, R₁, R₃, R₅, and R₆ are H; B₁ and B₂ together represent O; R₂ is CH₂CH₂OAc; R₄ is CH₂CH₂(2-Pyr); and X is CH₂.

25. The use of claim 21 wherein A₁, A₂, R₁, R₃, R₅, and R₆ are H; B₁ and B₂ together represent O; R₂ is H; R₄ is CH₂CH₂(2-Pyrimidinyl); and X is CH₂.

26. The use of claim 21 wherein A₁, A₂, R₁, R₃, R₅, and R₆ are H; B₁ and B₂ together represent O; R₂ is H; R₄ is CH₂CH₂(2-Pyr); and X is CH₂.

27. The use of claim 21 wherein A₁, A₂, R₁, R₂, R₃, R₅, and R₆ are H; B₁ and B₂ together represent O; R₄ is CH₂CH₂(2-Pyridazinyl); and X is CH₂.

28. The use of claim 21 wherein A₁, A₂, R₁, R₃, R₄, R₅, and R₆ are H; B₁ and B₂ together represent O; R₂ is CH₂CH₂OH; and X is CH₂.

29. The use of claim 21 wherein A₁, A₂, R₁, R₃, R₄, R₅, and R₆ are H; B₁ and B₂ together represent O; R₂ is CH₂CH₂CH₂OH; and X is CH₂.

30. The use of claim 21 wherein A₁, A₂, R₁, R₂, R₃, R₄ R₅, and R₆ are H; B₁ and B₂ together represent O; and X is S.

31. The use of claim 21 wherein A₁, A₂, R₁, R₃, R₄, R₅, and R₆ are H; B₁ and B₂ together represent O; R₂ is CH₂CH₂CH₂NHCO(4-(OH)Ph); and X is CH₂.

32. The use of claim 21 wherein A₁, A₂, R₁, R₃, R₄, R₅, and R₆ are H; B₁ and B₂ together represent O; R₂ is CH₂CH₂OH; and X is CH₂.

33. The use of a compound having the formula: 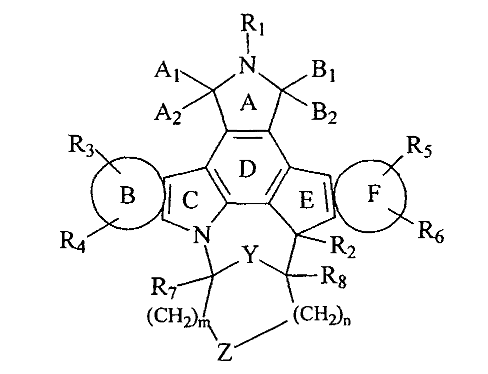 which inhibits a multiple lineage kinase protein in the manufacture of a medicament for use in the treatment of a neurodegenerative disorder or inflammation,
wherein:
ring B and ring F, independently, and each together with the carbon atoms to which they are attached, are selected from the group consisting of:
(a) an unsaturated 6-membered carbocyclic aromatic ring in which from 1 to 3 carbon atoms may be replaced by nitrogen atoms;
(b) an unsaturated 5-membered carbocyclic aromatic ring; and
(c) an unsaturated 5-membered carbocyclic aromatic ring in which either
(1) one carbon atom is replace with an oxygen, nitrogen, or sulfur atom;
(2) two carbon atoms are replaced with a sulfur and a nitrogen atom, an oxygen and a nitrogen atom, or two nitrogen atoms; or
(3) three carbon atoms are replaced with three nitrogen atoms;
R¹ is selected from the group consisting of:
(a) H, substituted or unsubstituted alkyl having from 1 to 4 carbons, substituted or unsubstituted aryl, substituted or unsubstituted arylalkyl, substituted or unsubstituted heteroaryl, or substituted or unsubstituted heteroarylalkyl;
(b) -C(=O)R⁹, where R⁹ is selected from the group consisting of alkyl, aryl and heteroaryl;
(c) -OR¹⁰, where R¹⁰ is selected from the group consisting of H and alkyl having from 1 to 4 carbons;
(d) -C(=O)NH₂, -NR¹¹R¹², -(CH₂)ₚNR¹¹R¹², -(CH₂)ₚOR¹⁰, -O(CH₂)ₚOR¹⁰ and -O(CH₂)ₚNR¹¹R¹², wherein p is from 1 to 4; and wherein either
(1) R¹¹ and R¹² are each independently selected from the group consisting of H and alkyl having from 1 to 4 carbons; or
(2) R¹¹ and R¹² together form a linking group of the formula -(CH₂)₂-X¹(CH₂)₂-, wherein X¹ is selected from the group consisting of -O-, -S-, -CH₂-;
R² is selected from the group consisting of H, alkyl having from 1 to 4 carbons, -OH, alkoxy having from 1 to 4 carbons, -OC(=O)R⁹, -OC(=O)NR¹¹R¹², -O(CH₂)ₚ NR¹¹R¹², -O(CH₂)ₚOR¹⁰, substituted or unsubstituted arylalkyl having from 6 to 10 carbons, and substituted or unsubstituted heteroarylalkyl;
R³, R⁴, R⁵ and R⁶ are each independently selected from the group consisting of:
(a) H, aryl, heteroaryl, F, Cl, Br, I, -CN, CF₃, -NO₂, -OH, -OR⁹, -O(CH₂)ₚNR¹¹R¹², -OC(=O)R⁹, -OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹;
(b) -CH₂OR¹⁴, wherein R¹⁴ is the residue of an amino acid after the hydroxyl group of the carboxyl group is removed;
(c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, or -CH=NNR²R^{2A} wherein R^{2A} is the same as R²;
(d) -S(O)_{y}R², -(CH₂)ₚS(O)_{y}R⁹, -CH₂S(O)_{y}R¹⁴ wherein y is 0, 1 or 2;
(e) alkyl having from 1 to 8 carbons, alkenyl having from 2 to 8 carbons, and alkynyl having 2 to 8 carbons, wherein
(1) each alkyl, alkenyl, or alkynyl group is unsubstituted; or
(2) each alkyl, alkenyl, or alkynyl group is substituted with 1 to 3 groups selected from the group consisting of aryl having from 6 to 10 carbons, heteroaryl, arylalkoxy, heterocycloalkoxy, hydroxyalkoxy, alkyloxy-alkoxy, hydroxyalkylthio, alkoxy-alkylthio, F, Cl, Br, I, -CN, -NO₂, -OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², -O-tetrahydropyranyl, -NR¹¹R¹², -NR¹⁰C(=O)R⁹, -NR¹⁰CO₂R⁹, -NR¹⁰C(=0)NR¹¹R¹², -NHC(=NH)NH₂, NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴, and a monosaccharide having from 5 to 7 carbons wherein each hydroxyl group of the monosaccharide is independently either unsubstituted or is replaced by H, alkyl having from 1 to 4 carbons, alkylcarbonyloxy having from 2 to 5 carbons, or alkoxy having from of 1 to 4 carbons;
X² is O, S or NR¹⁰;
R⁷ and R⁸ are each independently selected from the group consisting of H, alkyl having from I to 4 carbons, alkoxy having from 1 to 4 carbons, substituted or unsubstituted arylalkyl having from 6 to 10 carbons, substituted or unsubstituted heteroarylalkyl, -(CH₂)ₚOR¹⁰, -(CH₂)ₚOC(=O)NR¹¹R¹², and -(CH₂)ₚNR¹¹R¹²; or R⁷ and R⁸ together form a linking group of the formula -CH₂-X³-CH₂-, wherein X³ is X² or a bond;
m and n are each independently 0, 1, or 2;
Y is selected from the group consisting of -O-, -S-, -N(R¹⁰)-, -N⁺(O⁻)(R¹⁰)-, -N(OR¹⁰)-, and -CH₂-;
Z is selected from the group consisting of a bond, -O-, -CH=CH-, -S-, -C(=0)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, and -CH(O(C=O)R⁹)CH(OC(=O)R^{9A})-, wherein R^{9A} is the same as R⁹;
R¹⁵ and R¹⁶ are independently selected from the group consisting of H, -OH, -C(=O)R¹⁰, -O(C=O)R⁹, hydroxyalkyl, and -CO₂R¹⁰;
R¹⁷ is selected from the group consisting of H, alkyl, aryl, and heteroaryl;
A¹ and A² are selected from the group consisting of H, H; H, OR²; H, -SR²; H, -N(R²)₂; and a group wherein A¹ and A² together form a moiety selected from the group consisting of =O, =S, and =NR²;
B¹ and B² are selected from the group consisting of H, H; H, -OR²; H, -SR²; H, -N(R²)₂; and a group wherein B¹ and B² together form a moiety selected from the group consisting of =O, =S, and =NR²;
with the proviso that at least one of the pairs A¹ and A², or B¹ and B², form =O.

34. The use of a compound having the formula: 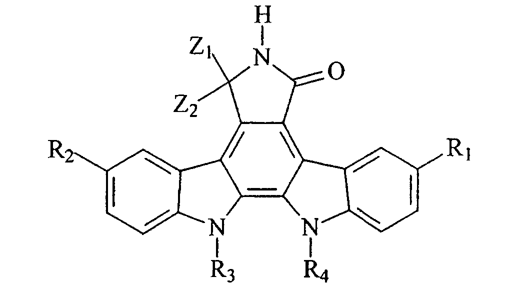 which inhibits a multiple lineage kinase protein in the manufacture of a medicament for use in the treatment of a neurodegenerative disorder or inflammation,
wherein
Z₁ is H and Z₂ is H or Z₁ and Z₂ together form =O;
R₁ is H or Br;
R₂ is H;
R₃ is H, CH₂CH=CH₂, CH₂CH₂CH₂OH, or 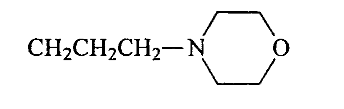 and
R₄ is H, CH₂CH=CH₂ or CH₂CH₂CH₂OH.

35. The use of claim 34 wherein R₁, R₂, R₄, Z₁, and Z₂ are H and R₃ is CH₂CH=CH₂.

36. The use of claim 34 wherein R₁ is Br and R₂, R₃, R₄, Z₁, and Z₂ are H.

37. The use of claim 34 wherein R₁, R₂, Z₁, and Z₂ are H and R₃ and R₄ are CH₂CH=CH₂.

38. The use of claim 34 wherein R₁, R₂, R₃, Z₁, and Z₂ are H and R₄ is CH₂CH=CH₂.

39. The use of claim 34 wherein R₁, R₂, Z₁, and Z₂ are H and R₃ and R₄ are CH₂CH=CH₂; or R₁, R₂, R₄, Z₁, and Z₂ are H, and R₃ is

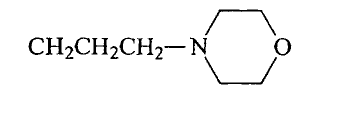

40. The use of a compound having the formula: 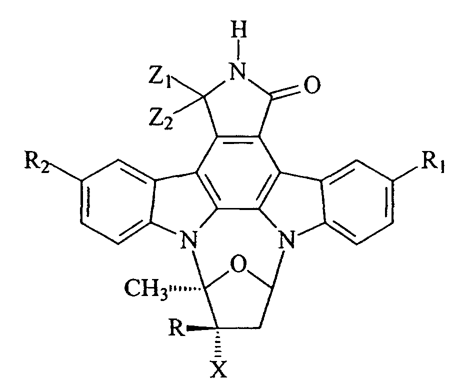 which inhibits a multiple lineage kinase protein in the manufacture of a medicament for use in the treatment of a neurodegenerative disorder or inflammation,
wherein
Z₁ is H and Z₂ is H or Z₁ and Z₂ together form =O;
R₁ is selected from the group consisting of H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-triazine)], CH₂CH₂SCH₃;
and 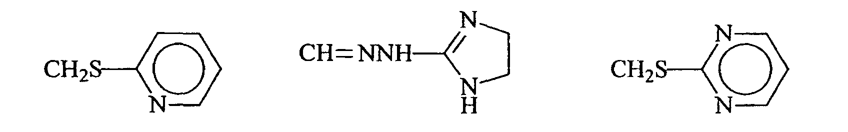 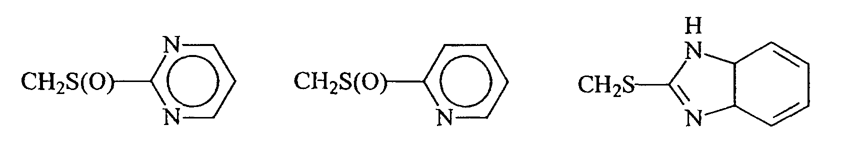 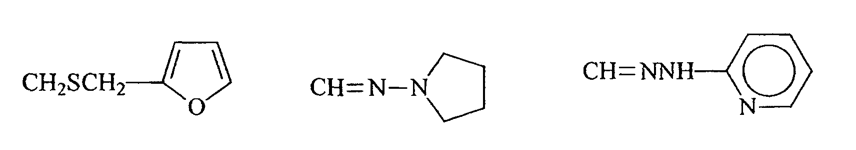 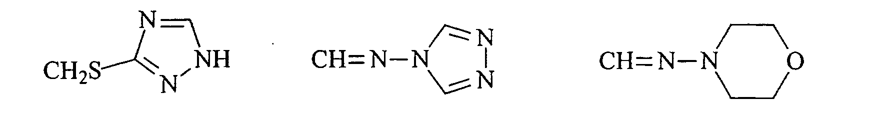 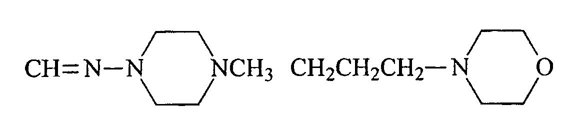 R₂ is selected from the group consisting of H, Br, Cl, I, CH₂S(CH₂)₂N(CH₃)₂, NHCONHC₂H₅, CH₂SC₂H₅, CH₂OCH₂OCH₂CH₃, CH₂S(3-(1.2,4-triazine)), CH₂CH₂SCH₃, and CH₂OH;
X is selected from the group consisting of H, CH₂OH, CH₂NH-SerineH, CO₂CH₃, CONHC₆H₅, CH₂NHCO₂C₆H₅, CH₂NHCO₂CH₃, CH₂N₃, CONHC₂H₅, CH₂NH-Glycine, CON(CH₃)₂, -CH₂NHCO₂-, CONH₂, CONHC₃H₇, CH₂NH-Serine, CH₂SOCH₃, CH=NOH, CH₂NH-Proline, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-hexyl, CONHCH₃, CO₂(CH₂)₄CH₃; 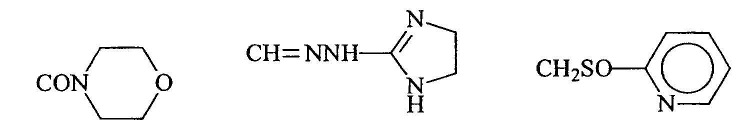 and
R is selected from the group consisting of OH, and OCH₃.

41. The use of claim 40 wherein Z₁ and Z₂ are H; X is CO₂CH₃; R₁ is NHCONHC₂H₅; R₂ is CH₂CH₂(2-Pyridyl); and R is OH.

42. The use of claim 40 wherein Z₁ and Z₂ are H; X is CO₂CH₃; R₁ and R₂ are CH₂OCH₂OCH₂CH₃; and R is OH.

43. The use of claim 40 wherein Z₁ and Z₂ are H; X is CO₂CH₃; R₁ and R₂ are CH₂SCH₂CH₃; and R is OH.

44. The use of claim 40 wherein Z₁, Z₂, R₁, and R₂ are H; X is CO₂CH₃; and R is OH.

45. The use of claim 40 wherein Z₁, Z₂, R₁, and R₂ are H; X is CO₂(CH₂)₄CH₃; and R is OH.

46. The use of claim 40 wherein Z₁, Z₂, and R₁ are H; R₂ is CH₂OH; X is CO₂CH₃; and R is OH.

47. The use of claim 40 wherein Z₁ and Z₂ are H; R₁ and R₂ are H₂S(3-(1,2,4-triazine)); X is CO₂CH₃; and R is OH.

48. The use of claim 40 wherein Z₁ and Z₂ are H; R₁ is Br; R₂ is I; X is CO₂CH₃; and R is OH.

49. The use of claim 40 wherein Z₁ and Z₂ are H; R₁ and R₂ are CH₂CH₂SCH₃; X is CO₂CH₃; and R is OH.

50. The use of claim 40 wherein Z₁, Z₂, R₁, and R₂ are H; X is CO₂CH₃; and R is OCH₃.

51. The use of claim 40 wherein Z₁ and Z₂ together form =O; R₁ and R₂ are Br; X is CO₂CH₃; and R is OH.

52. A method of modulating the activity of a multiple linage kinase protein comprising contacting said protein or a cell containing said protein with a compound having the formula 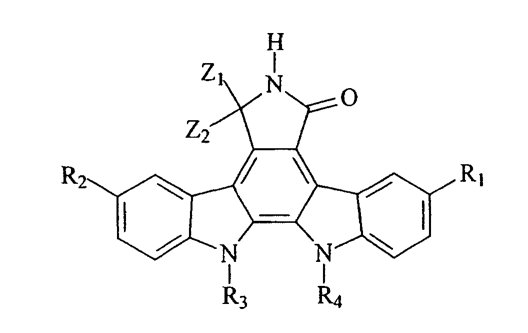 wherein
Z₁ is H and Z₂ is H or Z₁ and Z₂ together form =O;
R₁ is H or Br;
R₂ is H;
R₃ is H, CH₂CH=CH₂, CH₂CH₂CH₂OH, or 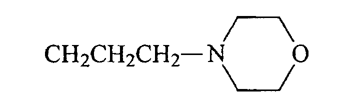 and
R₄ is H, CH₂CH=CH₂ or CH₂CH₂CH₂OH.

53. The method of claim 52 wherein R₁, R₂, R₄, Z₁, and Z₂ are H and R₃ is CH₂CH=CH₂.

54. The method of claim 52 wherein R₁ is Br and R₂, R₃, R₄, Z₁, and Z₂ are H.

55. The method of claim 52 wherein R₁, R₂, Z₁, and Z₂ are H and R₃ and R₄ are CH₂CH=CH₂.

56. The method of claim 52 wherein R₁, R₂, R₃, Z₁, and Z₂ are H and R₄ is CH₂CH=CH₂.

57. The method of claim 52 wherein R₁, R₂, Z₁, and Z₂ are H and R₃ and R₄ are CH₂CH=CH₂; or R₁, R₂, R₄, Z₁, and Z₂ are H, and R₃ is

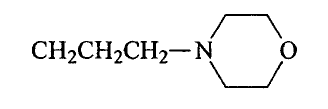

58. A method of modulating the activity of a multiple lineage kinase protein comprising contacting said protein or cell containing said protein with a compound having the formula 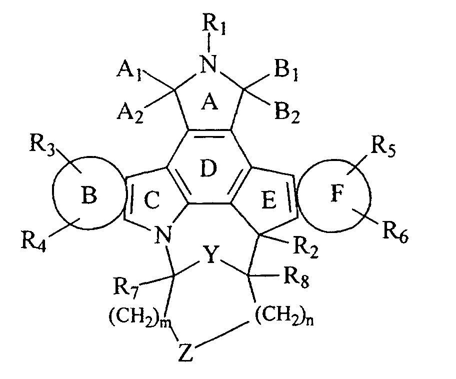 wherein:
ring B and ring F, independently, and each together with the carbon atoms to which they are attached, are selected from the group consisting of:
(a) an unsaturated 6-membered carbocyclic aromatic ring in which from I to 3 carbon atoms may be replaced by nitrogen atoms;
(b) an unsaturated 5-membered carbocyclic aromatic ring; and
(c) an unsaturated 5-membered carbocyclic aromatic ring in which either
(1) one carbon atom is replace with an oxygen, nitrogen, or sulfur atom;
(2) two carbon atoms are replaced with a sulfur and a nitrogen atom, an oxygen and a nitrogen atom, or two nitrogen atoms; or
(3) three carbon atoms are replaced with three nitrogen atoms;
R¹ is selected from the group consisting of:
(a) H, substituted or unsubstituted alkyl having from 1 to 4 carbons, substituted or unsubstituted aryl, substituted or unsubstituted arylalkyl, substituted or unsubstituted heteroaryl, or substituted or unsubstituted heteroarylalkyl;
(b) -C(=O)R⁹, where R⁹ is selected from the group consisting of alkyl, aryl and heteroaryl;
(c) -OR¹⁰, where R¹⁰ is selected from the group consisting of H and alkyl having from 1 to 4 carbons;
(d) -C(=O)NH₂, -NR¹¹R¹², -(CH₂)ₚNR¹¹R¹², -(CH₂)ₚOR¹⁰, -O(CH₂)ₚOR¹⁰ and -O(CH₂)ₚNR¹¹R¹², wherein p is from 1 to 4; and wherein either
(1) R¹¹ and R¹² are each independently selected from the group consisting of H and alkyl having from I to 4 carbons; or
(2) R¹¹ and R¹² together form a linking group of the formula -(CH₂)₂-X¹-(CH₂)₂-, wherein X¹ is selected from the group consisting of -O-, -S-, -CH₂-;
R² is selected from the group consisting of H, alkyl having from 1 to 4 carbons, -OH, alkoxy having from 1 to 4 carbons, -OC(=O)R⁹, -OC(=O)NR¹¹R¹², -O(CH₂)ₚ NR¹¹R¹², -O(CH₂)ₚOR¹⁰, substituted or unsubstituted arylalkyl having from 6 to 10 carbons, and substituted or unsubstituted heteroarylalkyl;
R³, R⁴, R⁵ and R⁶ are each independently selected from the group consisting of:
(a) H, aryl, heteroaryl, F, Cl, Br, I, -CN, CF₃, -NO₂, -OH, -OR⁹, -O(CH₂)ₚNR¹¹R¹², -OC(=O)R⁹, -OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹;
(b) -CH₂OR¹⁴, wherein R¹⁴ is the residue of an amino acid after the hydroxyl group of the carboxyl group is removed;
(c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, or -CH=NNR²R^{2A} wherein R^{2A} is the same as R²;
(d) -S(O)_{y}R², -(CH₂)ₚS(O)_{y}R⁹, -CH₂S(O)_{y}R¹⁴ wherein y is 0, 1 or 2;
(e) alkyl having from 1 to 8 carbons, alkenyl having from 2 to 8 carbons, and alkynyl having 2 to 8 carbons, wherein
(1) each alkyl, alkenyl, or alkynyl group is unsubstituted; or
(2) each alkyl, alkenyl, or alkynyl group is substituted with 1 to 3 groups selected from the group consisting of aryl having from 6 to 10 carbons, heteroaryl, arylalkoxy, heterocycloalkoxy, hydroxyalkoxy, alkyloxy-alkoxy, hydroxyalkylthio, alkoxy-alkylthio, F, Cl, Br, I, -CN, -NO₂, -OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², -O-tetrahydropyranyl, -NR¹¹R¹², -NR¹⁰C(=O)R⁹, -NR¹⁰CO₂R⁹, -NR¹⁰C(=0)NR¹¹R¹², -NHC(=NH)NH₂, NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴, and a monosaccharide having from 5 to 7 carbons wherein each hydroxyl group of the monosaccharide is independently either unsubstituted or is replaced by H, alkyl having from 1 to 4 carbons, alkylcarbonyloxy having from 2 to 5 carbons, or alkoxy having from of 1 to 4 carbons;
X² is O, S or NR¹⁰;
R⁷ and R⁸ are each independently selected from the group consisting of H, alkyl having from 1 to 4 carbons, alkoxy having from 1 to 4 carbons, substituted or unsubstituted arylalkyl having from 6 to 10 carbons, substituted or unsubstituted heteroarylalkyl, -(CH₂)ₚOR¹⁰, -(CH₂)ₚOC(=O)NR¹¹R¹², and -(CH₂)ₚNR¹¹R¹²; or R⁷ and R⁸ together form a linking group of the formula -CH₂-X³-CH₂-, wherein X³ is X² or a bond;
m and n are each independently 0, 1, or 2;
Y is selected from the group consisting of -O-, -S-, -N(R¹⁰)-, -N⁺(O⁻)(R¹⁰)-, -N(OR¹⁰)-, and -CH₂-;
Z is selected from the group consisting of a bond, -O-, -CH=CH-, -S-, -C(=O)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, and -CH(O(C=O)R⁹)CH(OC(=O)R^{9A})-, wherein R^{9A} is the same as R⁹;
R¹⁵ and R¹⁶ are independently selected from the group consisting of H, -OH, -C(=O)R¹⁰, -O(C=O)R⁹, hydroxyalkyl, and -CO₂R¹⁰;
R¹⁷ is selected from the group consisting of H, alkyl, aryl, and heteroaryl;
A¹ and A² are selected from the group consisting of H, H; H, OR²; H, -SR²; H, -N(R²)₂; and a group wherein A¹ and A² together form a moiety selected from the group consisting of =O, =S, and =NR²;
B¹ and B² are selected from the group consisting of H, H; H, -OR²; H, -SR²; H, -N(R²)₂; and a group wherein B¹ and B² together form a moiety selected from the group consisting of =O, =S, and =NR²;
with the proviso that at least one of the pairs A¹ and A², or B¹ and B², form =O.

59. A method of modulating the activity of a multiple lineage kinase protein comprising contacting said protein or a cell containing said protein with a compound having the formula 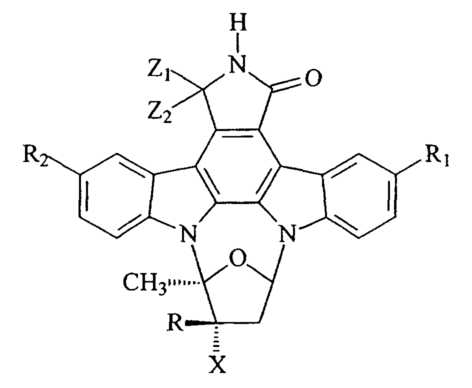 wherein
Z₁ is H and Z₂ is H or Z₁ and Z₂ together form =O;
R₁ is selected from the group consisting of H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-triazine)], CH₂CH₂SCH₃;
and 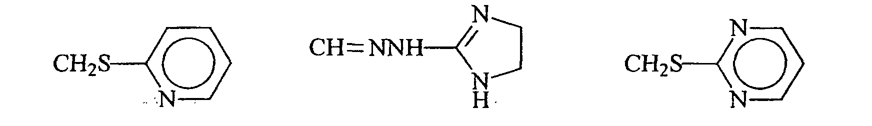 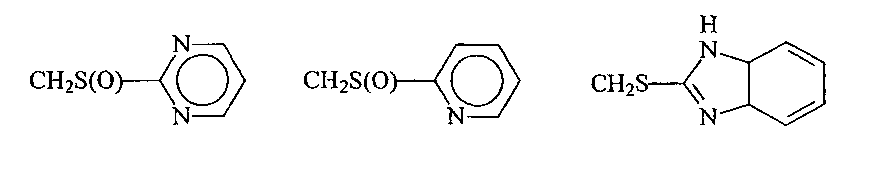 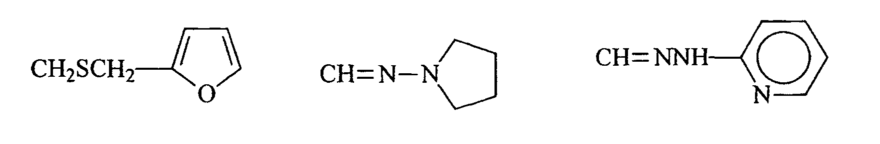 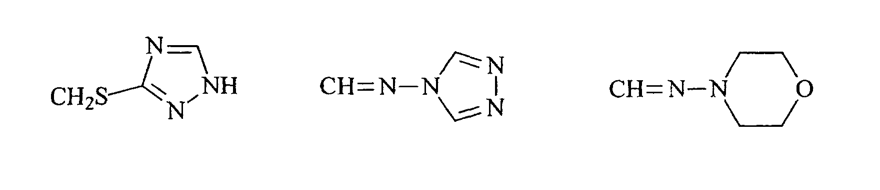 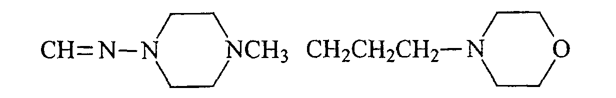 R₂ is selected from the group consisting of H, Br, Cl, I, CH₂S(CH₂)₂N(CH₃)₂, NHCONHC₂H₅, CH₂SC₂H₅, CH₂OCH₂OCH₂CH₃, CH₂S(3-(1.2,4-triazine)), CH₂CH₂SCH₃, and CH₂OH;
X is selected from the group consisting of H, CH₂OH, CH₂NH-SerineH, CO₂CH₃, CONHC₆H₅, CH₂NHCO₂C₆H₅, CH₂NHCO₂CH₃, CH₂N₃, CONHC₂H₅, CH₂NH-Glycine, CON(CH₃)₂, -CH₂NHCO₂-, CONH₂, CONHC₃H₇, CH₂NH-Serine, CH₂SOCH₃, CH=NOH, CH₂NH-Proline, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-hexyl, CONHCH₃, CO₂(CH₂)₄CH₃; 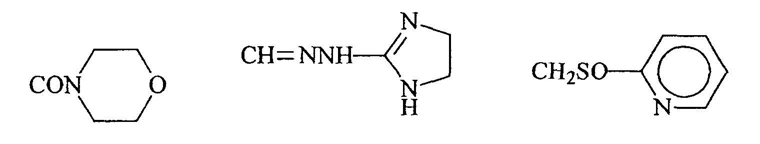 and
R is selected from the group consisting of OH, and OCH₃.

60. The method of claim 59 wherein Z₁ and Z₂ are H; X is CO₂CH₃; R₁ is NHCONHC₂H₅; R₂ is CH₂CH₂(2-Pyridyl); and R is OH.

61. The method of claim 59 wherein Z₁ and Z₂ are H; X is CO₂CH₃; R₁ and R₂ are CH₂OCH₂OCH₂CH₃; and R is OH.

62. The method of claim 59 wherein Z₁ and Z₂ are H; X is CO₂CH₃; R₁ and R₂ are CH₂SCH₂CH₃; and R is OH.

63. The method of claim 59 wherein Z₁, Z₂, R₁, and R₂ are H; X is CO₂CH₃; and R is OH.

64. The method of claim 59 wherein Z₁, Z₂, R₁, and R₂ are H; X is CO₂(CH₂)₄CH₃; and R is OH.

65. The method of claim 59 wherein Z₁, Z₂, and R₁ are H; R₂ is CH₂OH; X is CO₂CH₃; and R is OH.

66. The method of claim 59 wherein Z₁ and Z₂ are H; R₁ and R₂ are H₂S(3-(1,2,4-triazine)); X is CO₂CH₃; and R is OH.

67. The method of claim 59 wherein Z₁ and Z₂ are H; R₁ is Br; R₂ is I; X is CO₂CH₃; and R is OH.

68. The method of claim 59 wherein Z₁ and Z₂ are H; R₁ and R₂ are CH₂CH₂SCH₃; X is CO₂CH₃; and R is OH.

69. The method of claim 59 wherein Z₁, Z₂, R₁, and R₂ are H; X is CO₂CH₃; and R is OCH₃.

70. The method of claim 59 wherein Z₁ and Z₂ together form =O; R₁ and R₂ are Br; X is CO₂CH₃; and R is OH.

## Patentansprüche

1. Verfahren zum Identifizieren einer Verbindung, welche die Aktivität eines Multiple Lineage Kinase-Proteins moduliert und das Zellüberleben oder den Zelltod fördert, umfassend die Schritte
(a) des Inkontaktbringens der das Multiple Lineage Kinase-Protein enthaltenden Zelle mit der Verbindung *in vitro*,
(b) des Bestimmens, ob die Verbindung die Aktivität des Multiple Lineage Kinase-Proteins erhöht oder vermindert und
(c) des Bestimmens, ob die Verbindung das Zellüberleben oder den Zelltod fördert.

2. Verfahren nach Anspruch 1 zum Identifizieren einer Verbindung, die das Zellüberleben fördert, umfassend den Schritt des Bestimmens, ob die Verbindung die Aktivität des Multiple Lineage Kinase-Proteins vermindert.

3. Verfahren nach Anspruch 1 zum ldentifizieren einer Verbindung, die den Zelltod fördert, umfassend den Schritt des Bestimmens, ob die Verbindung die Aktivität des Multiple Lineage Kinase-Proteins erhöht.

4. Verfahren nach Anspruch 2, wobei die Verbindung in der Behandlung einer neurodegenerativen Störung oder Entzündung verwendbar sein kann.

5. Verfahren nach Anspruch 2 oder Anspruch 4, wobei das Protein aus der Gruppe, bestehend aus Multiple Lineage Kinase 1, Multiple Lineage Kinase 2, Multiple Lineage Kinase 3, Leucin-Zipper aufweisende Kinase, zweifachen Leucin-Zipper aufweisende Kinase und Multiple Lineage Kinase 6, ausgewählt ist.

6. Verfahren nach Anspruch 3, wobei das Protein aus der Gruppe, bestehend aus Multiple Lineage Kinase 1, Multiple Lineage Kinase 2, Multiple Lineage Kinase 3, Leucin-Zipper aufweisende Kinase, zweifach Leucin-Zipper aufweisende Kinase und Multiple Lineage Kinase 6, ausgewählt ist.

7. Verfahren nach Anspruch 5 oder Anspruch 6, wobei die Proteinaktivität durch einen *in vitro* Kinase-Assay oder Bindungs-Assay bestimmt wird.

8. Verfahren nach Anspruch 5 oder Anspruch 6, wobei die Zelle eine neuronale Zelle ist.

9. Verfahren nach Anspruch 5, wobei die Proteinaktivität durch das Messen der Aktivität oder des Phosphorylierungszustands eines Substrats des Proteins bestimmt wird.

10. Verfahren nach Anspruch 6, wobei die Proteinaktivität durch das Messen der Aktivität eines Substrats des Proteins bestimmt wird.

11. Verfahren nach den Ansprüchen 9 oder 10, wobei das Substrat aus der Gruppe, bestehend aus JNK1, JNK2, JNK3, ERK1, ERK2, p38α, p38β, p38γ, p38δ, MEK1, MEK2, MKK3, MKK4 (SEK1), MEK5, MKK6, MKK7, jun, ATF2, ELK1 und das Säuger-Homolog von AEX-3, ausgewählt aus.

12. Verfahren nach Anspruch 5, wobei die Proteinaktivität durch das Messen der Aktivität eines Substrats des Proteins, der Menge eines Substrats des Proteins oder der das Substrat des Proteins kodierenden mRNA bestimmt wird.

13. Verfahren nach Anspruch 6, wobei die Proteinaktivität durch das Messen der Aktivität eines Substrats des Proteins, der Menge des Proteins oder der das Protein kodierenden mRNA bestimmt wird.

14. Verfahren nach Anspruch 5 oder Anspruch 6, wobei das Fördern des Zellüberlebens durch das Verwenden von Zellen, die ein Risiko haben zu sterben, und durch das Vergleichen der Menge der lebenden Zellen, die in Kontakt mit der Verbindung gebracht wurden, mit der Menge der lebenden Zellen, die nicht in Kontakt mit der Verbindung gebracht wurden, bestimmt wird.

15. Verfahren nach Anspruch 5 oder 14, wobei die Zellen primäre embryonale motorische Nervenzellen sind.

16. Verfahren nach Anspruch 5 oder 14, wobei die Zellen das Multiple Lineage Kinase-Protein überexprimieren.

17. Verfahren nach Anspruch 5, wobei das Fördern des Zellüberlebens durch das Beobachten eines Abnehmens der Apoptose bestimmt wird.

18. Verfahren nach Anspruch 6, wobei das Fördern des Zelltods durch das Beobachten eines Zunehmens der Apoptose bestimmt wird.

19. Verfahren nach einem der Ansprüche 5 oder 6, wobei die Zelle in eine neurodegenerative Krankheit involviert ist.

20. Verfahren nach Anspruch 2, wobei die Zelle in eine Entzündung involviert ist.

21. Verwendung einer Verbindung der Formel 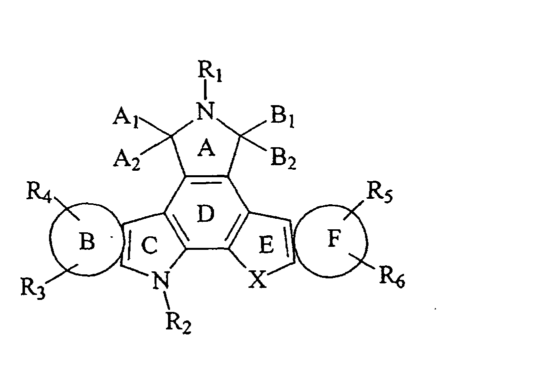 die das Multiple Lineage Kinase-Protein inhibiert, in der Herstellung eines Medikaments zur Verwendung in der Behandlung einer neurodegenerativen Störung oder Entzündung, wobei
der Ring B und der Ring F unabhängig voneinander jeweils zusammen mit den Kohlenstoffatomen, an die sie gebunden sind, entweder
einen ungesättigten 6-gliedrigen carbocyclischen aromatischen Ring, in dem ein bis drei Kohlenstoffatom(e) durch Stickstoffatom(e) ersetzt sein können, oder
einen ungesättigten 5-gliedrigen carbocyclischen aromatischen Ring, in dem entweder ein Kohlenstoffatom durch ein Sauerstoff-, Stickstoff- oder Schwefelatom ersetzt ist oder zwei Kohlenstoffatome durch ein Schwefel- und ein Stickstoffatom oder ein Sauerstoff- und ein Stickstoffatom ersetzt sind, bilden
A¹ und A² zusammen O darstellen und B¹ und B² zusammen O darstellen;
R¹ H, Alkyl mit 1 - 4 Kohlenstoffen (einschließlich), Aryl, Arylalkyl, Heteroaryl und Heteroarylalkyl, COR⁹, wobei R⁹ Alkyl mit 1 - 4 Kohlenstoffen (einschließlich) oder Aryl, vorzugsweise Phenyl oder Naphthyl, -OR¹⁰, wobei R¹⁰ H oder Alkyl mit 1 - 4 Kohlenstoffen (einschließlich) ist, -CONH₂, -NR⁷R⁸, -(CH₂)ₙNR⁷R⁸, wobei n eine ganze Zahl von 1 - 4 (einschließlich) ist, oder -O(CH₂)ₙNR⁷R⁸ ist und entweder
R⁷ und R⁸ unabhängig voneinander H oder Alkyl mit 1 - 4 Kohlenstoffen (einschließlich) sind oder
R⁷ und R⁸ miteinander verbunden sind, um eine Verbindungsgruppe der allgemeinen Formel -(CH₂)₂-X¹-(CH₂)₂- bilden, wobei X¹ O, S oder CH₂ ist,
R² H, -SO₂R⁹, -CO₂R⁹, -COR⁹, Alkyl mit 1 - 8 Kohlenstoffen (einschließlich), vorzugsweise Alkyl mit 1 - 4 Kohlenstoffen (einschließlich), Alkenyl mit 1 - 8 Kohlenstoffen (einschließlich), vorzugsweise Alkenyl mit 1 - 4 Kohlenstoffen (einschließlich) oder Alkinyl mit 1 - 8 Kohlenstoffen (einschließlich), vorzugsweise Alkinyl mit 1 - 4 Kohlenstoffen (einschließlich) oder ein Monosaccharid von 5 - 7 Kohlenstoffen (einschließlich) ist, wobei jede Hydroxylgruppe des Monosaccharids unabhängig voneinander entweder nicht substituiert ist oder durch H, Alkyl mit 1 - 4 Kohlenstoffen (einschließlich), Alkylcarbonyloxy von 2 - 5 Kohlenstoffen (einschließlich) oder Alkoxy von 1 - 4 Kohlenstoffen (einschließlich) ersetzt ist und entweder
jedes Alkyl mit 1 - 8 Kohlenstoffen (einschließlich), Alkenyl mit 1 - 8 Kohlenstoffen (einschließlich) oder Alkinyl mit 1 - 8 Kohlenstoffen (einschließlich) nicht substituiert ist oder
jedes Alkyl mit 1 - 8 Kohlenstoffen (einschließlich), Alkenyl mit 1 - 8 Kohlenstoffen (einschließlich) oder Alkinyl mit 1 - 8 Kohlenstoffen (einschließlich) unabhängig voneinander substituiert ist mit 1 - 3 Arylgruppen mit 6 - 10 Kohlenstoffen (einschließlich), vorzugsweise Phenyl oder Naphthyl, Heteroaryl, F, Cl, Br, I, -CN, -NO₂, OH, -OR⁹, -O(CH₂)ₙNR⁷R⁸, -OCOR⁹, -OCONHR⁹, O-Tetrahydropyranyl, NH₂, -NR⁷R⁸, -NR¹⁰COR⁹, -NR¹⁰CO₂R⁹, -NR¹⁰CONR⁷R⁸, -NHC(=NH)NH₂, -NR¹⁰SO₂R⁹, -S(O)_{y}R¹¹, wobei R¹¹ H oder Alkyl mit 1 - 4 Kohlenstoffen, Aryl mit 6 - 10 Kohlenstoffen, vorzugsweise Phenyl oder Naphthyl, oder Heteroaryl ist und y 1 oder 2 ist, -SR¹¹, -CO₂R⁹, -CONR⁷R⁸, -CHO, COR⁹, -CH₂OR⁷, -CH=NNR¹¹R¹², -CH=NOR¹¹, -CH=NR⁹, -CH=NNHCH(N=NH)NH₂, -SO₂NR¹²R¹³, -PO(OR¹¹)₂ oder OR¹⁴ ist, wobei R¹⁴ der Rest einer Aminosäure ist, nachdem die Hydroxylgruppe der Carboxylgruppe entfernt ist,
und entweder R¹² und R¹³ unabhängig voneinander H, Alkyl mit 1 - 4 Kohlenstoffen (einschließlich), Aryl mit 6 - 10 Kohlenstoffen, vorzugsweise Phenyl oder Naphthyl, oder ein Heteroaryl sind oder
R¹² und R¹³ miteinander verbunden sind, um eine Verbindungsgruppe, vorzugsweise -(CH₂)₂-X¹-(CH₂)₂, zu bilden,
jedes R³, R⁴, R⁵ und R⁶ unabhängig voneinander H, ein Aryl, vorzugsweise Aryl mit 6 - 10 Kohlenstoffen (einschließlich), bevorzugter Phenyl oder Naphthyl, Heteroaryl, F, Cl, Br, I, -CN, CF₃, -NO₂, OH, -OR⁹, -O(CH₂)ₙNR⁷R⁸, -OCOR⁹, -OCONHR⁹, NH₂, -CH₂OH, -CH₂OR¹⁴, -NR⁷R⁸, -NR¹⁰COR⁹, -NR¹⁰CONR⁷R⁸, -SR¹¹, -S(O)_{y}R¹¹, wobei y 1 oder 2 ist, -CO₂R⁹, -COR⁹, -CONR⁷R⁸, -CHO, -CH=NOR¹¹, -CH=NR⁹, -CH=NNR¹¹R¹², -(CH₂)ₙSR⁹, wobei n eine ganze Zahl von 1 - 4 (einschließlich), -(CH₂)ₙS(O)_{y}R⁹, -CH₂SR¹⁵, wobei R¹⁵ Alkyl mit 1 - 4 Kohlenstoffen (einschließlich), -CH₂S(O)_{y}R¹⁴, -(CH₂)ₙNR⁷R⁸, -(CH₂)ₙNHR¹⁴, Alkyl mit 1 - 8 Kohlenstoffen (einschließlich), vorzugsweise Alkyl mit 1 - 4 Kohlenstoffen (einschließlich), Alkenyl mit 1 - 8 Kohlenstoffen (einschließlich), vorzugsweise Alkenyl mit 1 - 4 Kohlenstoffen (einschließlich), Alkinyl mit 1 - 8 Kohlenstoffen (einschließlich), vorzugsweise Alkinyl mit 1 - 4 Kohlenstoffen (einschließlich) ist und entweder
jedes Alkyl mit 1 - 8 Kohlenstoffen (einschließlich), Alkenyl mit 1 - 8 Kohlenstoffen (einschließlich) oder Alkinyl mit 1 - 8 Kohlenstoffen (einschließlich) nicht substituiert ist oder
jedes Alkyl mit 1 - 8 Kohlenstoffen (einschließlich), Alkenyl mit 1 - 8 Kohlenstoffen (einschließlich) oder Alkinyl mit 1 - 8 Kohlenstoffen (einschließlich), wie in (d) (2) von Anspruch 58 beschrieben, substituiert ist,
X entweder
nicht substituiertes Alkylen mit 1 - 3 Kohlenstoffen (einschließlich) ist oder
X Alkylen mit 1 - 3 Kohlenstoffen (einschließlich) ist, welches mit einer R²-Gruppe, vorzugsweise OR¹⁰, -SR¹⁰, R¹⁵, wobei R¹⁵ Alkyl mit 1 - 4 Kohlenstoffen (einschließlich) ist, Phenyl, Naphthyl, Arylalkyl mit 7 - 14 Kohlenstoffen (einschließlich), vorzugsweise Benzyl, substituiert ist, oder
X -CH=CH-, -CH(OH)-CH(OH)-, -O-, -S- -S(=O)-, -S(=O)₂-, -C(R¹⁰)₂-, -C(=O)-, -C(=NOR¹¹)-, -C(OR¹¹)(R¹¹)-, -C(=O)CHR¹⁵-, -CHR¹⁵C(=O)-, -C(=NOR¹¹)CHR¹⁵-, -CHR¹⁵C(=NOR¹¹)-, -CH₂Z-, -Z-CH₂-, -CH₂ZCH₂- ist, wobei Z C(OR¹¹)(R¹¹), O, S, C(=O), C(=NOR¹¹) oder NR¹¹ ist
oder
A¹ und A² zusammen jeweils unabhängig voneinander H, H; H, -OR¹¹; H, -SR¹¹; H, -NR¹¹R¹² sind oder zusammen =S oder =NR¹¹ darstellen, B¹ und B² zusammen O darstellen und jedes R¹, R², R³, R⁴, R⁵, R⁶ und X, wie in (c), (d), (e) und (f) von Anspruch 58 definiert, sind
oder
A¹ und A² zusammen O darstellen und B¹ und B² jeweils unabhängig voneinander H, H; H, -OR¹¹, H, -SR¹¹, H, -NR¹¹R¹² sind oder zusammen =S oder =NR¹¹ darstellen und jedes R¹, R², R³, R⁴, R⁵, R⁶ und X, wie in (c), (d), (e) und (f) von Anspruch 58 definiert, sind.

22. Verwendung nach Anspruch 21, wobei A₁, A₂, R₁, R₃ und R₄ H sind, B₁ und B₂ zusammen 0 darstellen, R₂ CH₂CH₂OH ist, R₅ und R₆ OCH₃ sind und X CH₂ ist.

23. Verwendung nach Anspruch 21, wobei A₁, A₂, R₁, R₃, R₅ und R₆ H sind, B₁ und B₂ zusammen O darstellen, R₂ CH₂CH₂OAc ist, R₄ Br ist und X CH₂ ist.

24. Verwendung nach Anspruch 21, wobei A₁, A₂, R₁, R₃, R₅ und R₆ H sind, B₁ und B₂ zusamamen O darstellen, R₂, CH₂CH₂OAc ist, R₄ CH₂CH₂(2-Pyr) ist und X CH₂ ist.

25. Verwendung nach Anspruch 21, wobei A₁, A₂, R₁, R₃, R₅ und R₆ H sind, B₁ und B₂ zusammen O darstellen, R₂ H ist, R₄ CH₂CH₂(2-Pyrimidinyl) ist und X CH₂ ist.

26. Verwendung nach Anspruch 21, wobei A₁, A₂, R₁, R₃, R₅ und R₆ H sind, B₁ und B₂ zusammen O darstellen, R₂ H ist, R₄ CH₂CH₂(2-Pyr) ist und X CH₂ ist.

27. Verwendung nach Anspruch 21, wobei A₁, A₂, R₁, R₂, R₃, R₅ und R₆ H sind, B₁ und B₂ zusammen O darstellen, R₄ CH₂CH₂(2-Pyridazinyl) ist und X CH₂ ist.

28. Verwendung nach Anspruch 21, wobei A₁, A₂, R₁, R₃, R₄, R₅ und R₆ H sind, B₁ und B₂ zusammen O darstellen, R₂ CH₂CH₂OH ist und X CH₂ ist.

29. Verwendung nach Anspruch 21, wobei A₁, A₂, R₁, R₃, R₄, R₅ und R₆ H sind, B₁ und B₂ zusammen O darstellen, R₂ CH₂CH₂CH₂OH ist und X CH₂ ist.

30. Verwendung nach Anspruch 21, wobei A₁, A₂, R₁, R₂, R₃, R₄, R₅ und R₆ H sind, B₁ und B₂ zusammen O darstellen und X S ist.

31. Verwendung nach Anspruch 21, wobei A₁, A₂, R₁, R₃, R₄, R₅ und R₆ H sind, B₁ und B₂ zusammen O darstellen, R₂ CH₂CH₂CH₂NHCO(4-(OH)Ph) ist und X CH₂ ist.

32. Verwendung nach Anspruch 21, wobei A₁, A₂, R₁, R₃, R₄, R₅ und R₆ H sind, B₁ und B₂ zusammen O darstellen, R₂ CH₂CH₂OH ist und X CH₂ ist.

33. Verwendung einer Verbindung mit der Formel 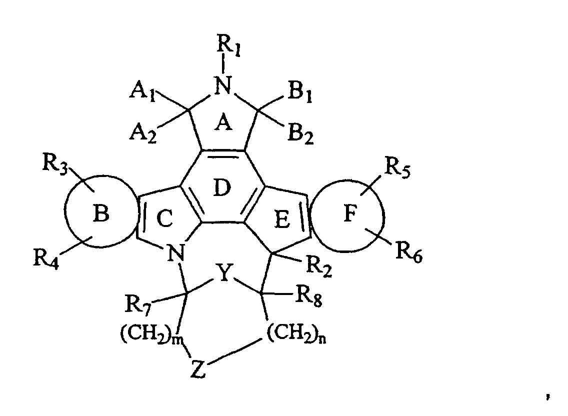 die ein Multiple Lineage Kinase-Protein inhibiert, in der Herstellung eines Medikaments zur Verwendung in der Behandlung einer neurodegenerativen Störung oder Entzündung, wobei
Ring B und Ring F unabhängig voneinander und jeder zusammen mit den Kohlenstoffatomen, an die sie gebunden sind, aus der Gruppe, bestehend aus
(a) einem ungesättigten 6-gliedrigen carbocyclischen aromatischen Ring, in dem 1 bis 3 Kohlenstoffatome durch Stickstoffatome ersetzt sein können,
(b) einem ungesättigten 5-gliedrigen carbocyclischen aromatischen Ring und
(c) einem ungesättigten 5-gliedrigen carbocyclischen aromatischen Ring, in dem entweder
(1) ein Kohlenstoffatom durch ein Sauerstoff-, Stickstoff- oder Schwefelatom ersetzt ist,
(2) zwei Kohlenstoffatome durch ein Schwefel- und ein Stickstoffatom, ein Sauerstoff- und ein Stickstoffatom oder zwei Stickstoffatome ersetzt sind, oder
(3) drei Kohlenstoffatome durch drei Stickstoffatome ersetzt sind,
ausgewählt sind,
R¹ aus der Gruppe, bestehend aus
(a) H, substituiertem oder nicht substituiertem Alkyl mit 1 bis 4 Kohlenstoffen, substituiertem oder nicht substituiertem Aryl, substituiertem oder nicht substituiertem Arylalkyl, substituiertem oder nicht substituiertem Heteroaryl oder substituiertem oder nicht substituiertem Heteroarylalkyl,
(b) -C(=O)R⁹, wobei R⁹ ausgewählt ist aus der Gruppe, bestehend aus Alkyl, Aryl und Heteroaryl,
(c) -OR¹⁰, wobei R¹⁰ ausgewählt ist aus der Gruppe, bestehend aus H und Alkyl mit 1 bis 4 Kohlenstoffen,
(d) -C(=O)NH₂, -NR¹¹R¹², -(CH₂)ₚNR¹¹R¹², -(CH₂)ₚOR¹⁰, -O(CH₂)ₚOR¹⁰ und -O(CH₂)ₚNR¹¹R¹² wobei p 1 bis 4 ist und wobei entweder
(1) R¹¹ und R¹² jeweils unabhängig voneinander ausgewählt sind aus der Gruppe, bestehend aus H und Alkyl mit 1 bis 4 Kohlenstoffen, oder
(2) R¹¹ und R¹² zusammen eine Verbindungsgruppe der Formel -(CH₂)₂-X¹-(CH₂)₂- bilden, wobei X¹ ausgewählt ist aus der Gruppe, bestehend aus -O-, -S-, -CH₂-,
ausgewählt ist,
R² aus der Gruppe, bestehend aus H, Alkyl mit 1 bis 4 Kohlenstoffen, -OH, Alkoxy mit 1 bis 4 Kohlenstoffen, -OC(=O)R⁹, -OC(=O)NR¹¹R¹², -O(CH₂)ₚNR¹¹R¹², -O(CH₂)ₚOR¹⁰, substituiertem oder nicht substituiertem Arylalkyl mit 6 bis 10 Kohlenstoffen und substituiertem oder nicht substituiertem Heteroarylalkyl, ausgewählt ist,
R³, R⁴, R⁵ und R⁶ jeweils unabhängig voneinander aus der Gruppe, bestehend aus
(a) H, Aryl, Heteroaryl, F, Cl, Br, I, -CN, CF₃, -NO₂, -OH, -OR⁹, -O(CH₂)ₚNR¹¹R¹², -OC(=O)R⁹, -OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹,
(b) -CH₂OR¹⁴, wobei R¹⁴ der Rest einer Aminosäure ist, nachdem die Hydroxylgruppe der Carboxylgruppe entfernt ist,
(c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴ oder -CH=NNR²R^{2A}, wobei R^{2A} das Gleiche ist wie R²,
(d) -S(O)_{y}R², -(CH₂)ₚS(O)_{y}R⁹, -CH₂S(O)_{y}R¹⁴, wobei y 0, 1 oder 2 ist,
(e) Alkyl mit 1 bis 8 Kohlenstoffen, Alkenyl mit 2 bis 8 Kohlenstoffen und Alkinyl mit 2 bis 8 Kohlenstoffen, wobei
(1) jede Alkyl-, Alkenyl- oder Alkinylgruppe nicht substituiert ist oder
(2) jede Alkyl-, Alkenyl- oder Alkinylgruppe mit 1 bis 3 Gruppen, ausgewählt aus der Gruppe, bestehend aus Aryl mit 6 bis 10 Kohlenstoffen, Heteroaryl, Arylalkoxy, Heterocycloalkoxy, Hydroxyalkoxy, Alkyloxy-Alkoxy, Hydroxyalkylthio, Alkoxy-Alkylthio, F, Cl, Br, I, -CN, -NO₂, -OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², -O-Tetrahydropyranyl, -NR¹¹R¹², -NR¹⁰C(=O)R⁹, -NR¹⁰CO₂R⁹, -NR¹⁰C(=O)NR¹¹R¹², -NHC(=NH)NH₂, NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴ und einem Monosaccharid mit 5 bis 7 Kohlenstoffen, wobei jede Hydroxylgruppe des Monosaccharids unabhängig voneinander entweder nicht substituiert oder durch H, Alkyl mit 1 bis 4 Kohlenstoffen, Alkylcarbonyloxy mit 2 bis 5 Kohlenstoffen oder Alkoxy mit 1 bis 4 Kohlenstoffen ersetzt ist, substituiert ist,
ausgewählt sind,
X² O, S oder NR¹⁰ ist,
R⁷ und R⁸ jeweils unabhängig voneinander ausgewählt sind aus der Gruppe, bestehend aus H, Alkyl mit 1 bis 4 Kohlenstoffen, Alkoxy mit 1 bis 4 Kohlenstoffen, substituiertem oder nicht substituiertem Arylalkyl mit 6 bis 10 Kohlenstoffen, substituiertem oder nicht substituiertem Heteroarylalkyl, -(CH₂)ₚOR¹⁰, -(CH₂)ₚOC(=O)NR¹¹R¹² und -(CH₂)ₚNR¹¹R¹², oder R⁷ und R⁸ zusammen eine Verbindungsgruppe mit der Formel -CH₂-X³-CH₂- bilden, wobei X³ X² oder eine Bindung ist,
m und n jeweils unabhängig voneinander 0, 1 oder 2 sind,
Y aus der Gruppe, bestehend aus -O-, -S-, -N(R¹⁰)-, -N⁺(O⁻)(R¹⁰)-, -N(OR¹⁰)- und -CH₂- ausgewählt ist,
Z aus der Gruppe, bestehend aus einer Bindung, -O-, -CH=CH-, -S-, -C(=O)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)- und -CH(O(C=O)R⁹)CH(OC(=O)R^{9A})-, ausgewählt ist, wobei R^{9A} das Gleiche wie R⁹ ist,
R¹⁵ und R¹⁶ unabhängig voneinander aus der Gruppe, bestehend aus H, -OH, -C(=O)R¹⁰, -O(C=O)R⁹, Hydroxyalkyl und -CO₂R¹⁰, ausgewählt sind,
R¹⁷ aus der Gruppe, bestehend aus H, Alkyl, Aryl und Heteroaryl, ausgewählt ist,
A¹und A² aus der Gruppe, bestehend aus H, H; H, OR²; H, -SR²; H, -N(R²)₂ und einer Gruppe, in der A¹ und A² zusammen eine Einheit, ausgewählt aus der Gruppe, bestehend aus =O, =S und =NR² bilden, ausgewählt sind,
B¹ und B² aus der Gruppe, bestehend aus H, H; H, -OR²; H, -SR²; H, -N(R²)₂ und einer Gruppe, in der B¹ und B² zusammen eine Einheit, ausgewählt aus der Gruppe, bestehend aus =O, =S und =NR² bilden, ausgewählt sind,
mit der Maßgabe, daß mindestens eines der Paare A¹ und A² oder B¹ und B² =O bilden.

34. Verwendung einer Verbindung mit der Formel 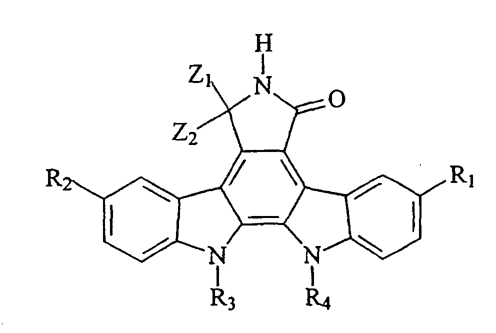 die ein Multiple Lineage Kinase-Protein inhibiert, in der Herstellung eines Medikaments zur Verwendung in der Behandlung einer neurodegenerativen Störung oder Entzündung, wobei
Z₁ H ist und Z₂ H ist oder Z₁ und Z₂ zusammen =O bilden,
R₁ H oder Br ist,
R₂ H ist,
R₃ H, CH₂CH=CH₂, CH₂CH₂CH₂OH oder 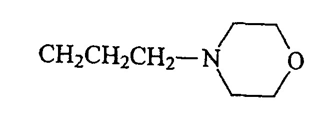 ist und
R₄ H, CH₂CH=CH₂ oder CH₂CH₂CH₂OH ist.

35. Verwendung nach Anspruch 34, wobei R₁, R₂, R₄, Z₁ und Z₂ H sind und R₃ CH₂CH=CH₂ ist.

36. Verwendung nach Anspruch 34, wobei R₁ Br ist und R₂, R₃, R₄, Z₁ und Z₂ H sind.

37. Verwendung nach Anspruch 34, wobei R₁, R₂, Z₁ und Z₂ H sind und R₃ und R₄ CH₂CH=CH₂ sind.

38. Verwendung nach Anspruch 34, wobei R₁, R₂, R₃, Z₁ und Z₂ H sind und R₄ CH₂CH=CH₂ ist.

39. Verwendung nach Anspruch 34, wobei R₁, R₂, Z₁ und Z₂ H sind und R₃ und R₄ CH₂CH=CH₂ sind oder R₁, R₂, R₄, Z₁ und Z₂ H sind und R₃ 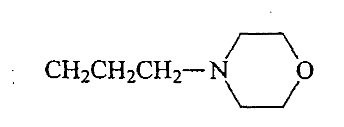 ist.

40. Verwendung einer Verbindung mit der Formel 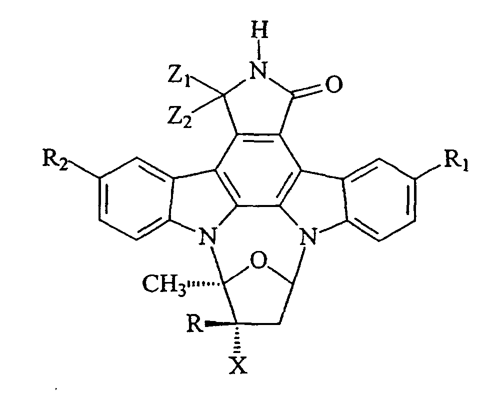 die ein Multiple Lineage Kinase-Protein inhibiert, in der Herstellung eines Medikaments zur Verwendung in der Behandlung einer neurodegenerativen Störung oder Entzündung, wobei
Z₁ H ist und Z₂ H ist oder Z₁ und Z₂ zusammen =O bilden,
R₁ aus der Gruppe, bestehend aus H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-Propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-Triazin)], CH₂CH₂SCH₃ und 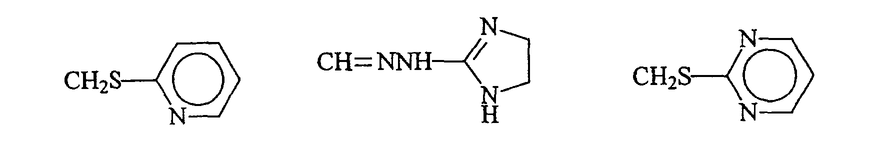 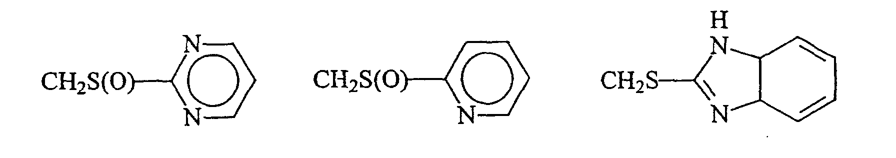 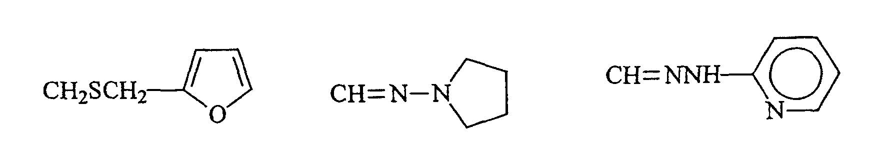 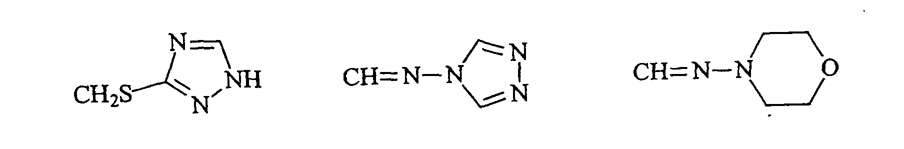 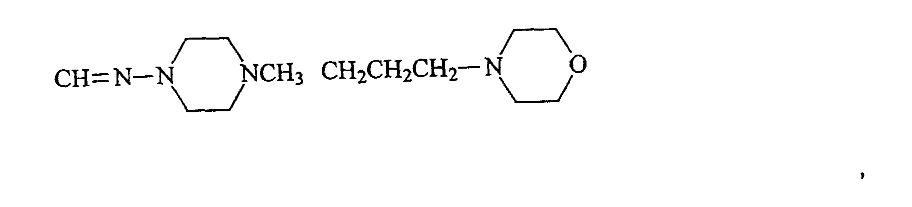 ausgewählt ist,
R₂ aus der Gruppe, bestehend aus H, Br, Cl, I, CH₂S(CH₂)₂N(CH₃)₂, NHCONHC₂H₅, CH₂SC₂H₅, CH₂OCH₂OCH₂CH₃, CH₂S(3-(1,2,4-Triazin)), CH₂CH₂SCH₃ und CH₂OH, ausgewählt ist,
X aus der Gruppe, bestehend aus H, CH₂OH, CH₂NH-SerinH, CO₂CH₃, CONHC₆H₅, CH₂NHCO₂C₆H₅, CH₂NHCO₂CH₃, CH₂N₃, CONHC₂H₅, CH₂NH-Glycin, CON(CH₃)₂, -CH₂NHCO₂-, CONH₂, CONHC₃H₇, CH₂NH-Serin, CH₂SOCH₃, CH=NOH, CH₂NH-Prolin, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-Hexyl, CONHCH₃, CO₂(CH₂)₄CH₃, 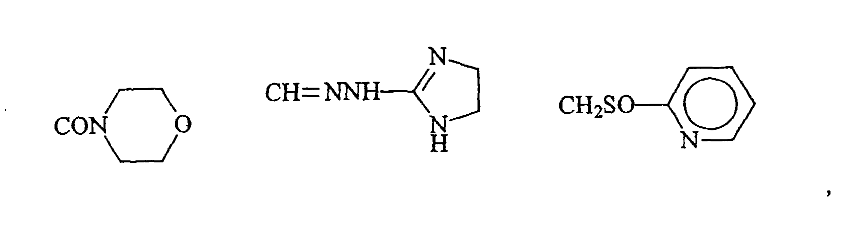 ausgewählt ist und
R aus der Gruppe, bestehend aus OH und OCH₃, ausgewählt ist.

41. Verwendung nach Anspruch 40, wobei Z₁ und Z₂ H sind, X CO₂CH₃ ist, R₁ NHCONHC₂H₅ ist, R₂ CH₂CH₂(2-Pyridyl) ist und R OH ist.

42. Verwendung nach Anspruch 40, wobei Z₁ und Z₂ H sind, X CO₂CH₃ ist, R₁ und R₂ CH₂OCH₂OCH₂CH₃ sind und R OH ist.

43. Verwendung nach Anspruch 40, wobei Z₁ und Z₂ H sind, X CO₂CH₃ ist, R₁ und R₂ CH₂SCH₂CH₃ sind und R OH ist.

44. Verwendung nach Anspruch 40, wobei Z₁, Z₂, R₁ und R₂ H sind, X CO₂CH₃ ist und R OH ist.

45. Verwendung nach Anspruch 40, wobei Z₁, Z₂, R₁ und R₂ H sind, X CO₂(CH₂)₄CH₃ ist und R OH ist.

46. Verwendung nach Anspruch 40, wobei Z₁, Z₂ und R₁ H sind, R₂ CH₂OH ist, X CO₂CH₃ ist und R OH ist.

47. Verwendung nach Anspruch 40, wobei Z₁ und Z₂ H sind, R₁ und R₂ H₂S(3-(1,2,4-Triazin)) sind, X CO₂CH₃ ist und R OH ist.

48. Verwendung nach Anspruch 40, wobei Z₁ und Z₂ H sind, R₁ Br ist, R₂ I ist, X CO₂CH₃ ist und R OH ist.

49. Verwendung nach Anspruch 40, wobei Z₁ und Z₂ H sind, R₁ und R₂ CH₂CH₂SCH₃ sind, X CO₂CH₃ ist und R OH ist.

50. Verwendung nach Anspruch 40, wobei Z₁, Z₂, R₁ und R₂ H sind, X CO₂CH₃ ist und R OCH₃ ist.

51. Verwendung nach Anspruch 40, wobei Z₁ und Z₂ zusammen =O bilden, R₁ und R₂ Br sind, X CO₂CH₃ ist und R OH ist.

52. Verfahren zum Modulieren der Aktivität eines Multiple Lineage Kinase-Proteins, umfassend das Inkontaktbringen des Proteins oder einer Zelle, die das Protein enthält, mit einer Verbindung mit der Formel 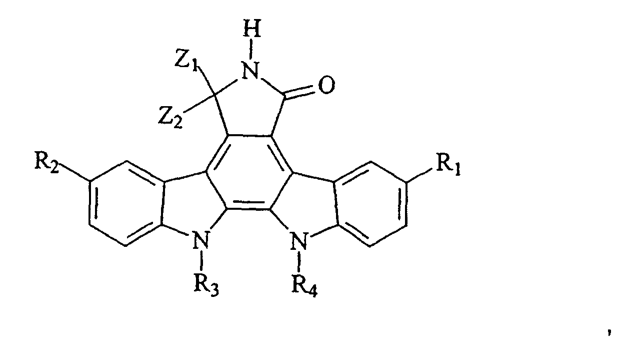 wobei
Z₁ H ist und Z₂ H ist oder Z₁ und Z₂ zusammen =O bilden,
R₁ H oder Br ist,
R₂ H ist,
R₃ H, CH₂CH=CH₂, CH₂CH₂CH₂OH oder 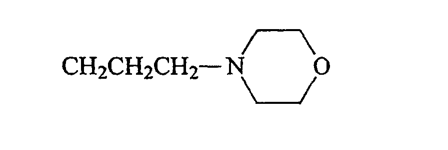 ist und
R₄ H, CH₂CH=CH₂ oder CH₂CH₂CH₂OH ist.

53. Verfahren nach Anspruch 52, wobei R₁, R₂, R₄, Z₁ und Z₂ H sind und R₃ CH₂CH=CH₂ ist.

54. Verfahren nach Anspruch 52, wobei R₁ Br ist und R₂, R₃, R₄, Z₁ und Z₂ H sind.

55. Verfahren nach Anspruch 52, wobei R₁, R₂, Z₁ und Z₂ H sind und R₃ und R₄ CH₂CH=CH₂ sind.

56. Verfahren nach Anspruch 52, wobei R₁, R₂, R₃, Z₁ und Z₂ H sind und R₄ CH₂CH=CH₂ ist.

57. Verfahren nach Anspruch 52, wobei R₁, R₂, Z₁ und Z₂ H sind und R₃ und R₄ CH₂CH=CH₂ sind oder R₁, R₂, R₄, Z₁ und Z₂ H sind und R₃ 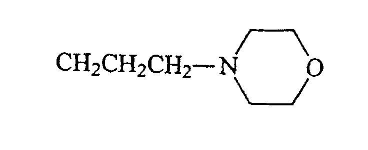 ist.

58. Verfahren zum Modulieren der Aktivität eines Multiple Lineage Kinase-Proteins, umfassend das Inkontaktbringen des Proteins oder einer Zelle, die das Protein enthält, mit einer Verbindung mit der Formel 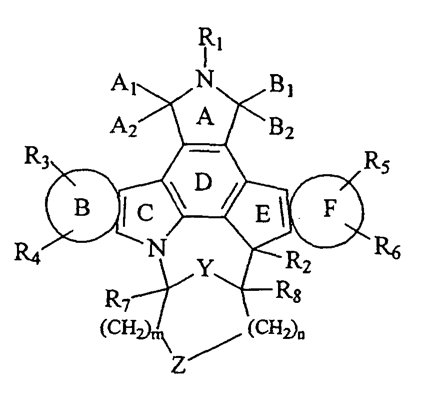 wobei
Ring B und Ring F unabhängig voneinander und jeweils zusammen mit den Kohlenstoffatomen, an die sie gebunden sind, aus der Gruppe, bestehend aus
(a) einem ungesättigten 6-gliedrigen carbocyclischen aromatischen Ring, in dem 1 bis 3 Kohlenstoffatome durch Stickstoffatome ersetzt sein können,
(b) einem ungesättigten 5-gliedrigen carbocyclischen aromatischen Ring und
(c) einem ungesättigten 5-gliedrigen carbocyclischen aromatischen Ring, in dem entweder
(1) ein Kohlenstoffatom durch ein Sauerstoff-, Stickstoff- oder Schwefelatom ersetzt ist,
(2) zwei Kohlenstoffatome durch ein Schwefel- und ein Stickstoffatom, ein Sauerstoff- und ein Stickstoffatom oder zwei Stickstoffatome ersetzt sind oder
(3) drei Kohlenstoffatome durch drei Stickstoffatome ersetzt sind,
ausgewählt sind,
R₁ aus der Gruppe, bestehend aus
(a) H, substituiertem oder nicht substituiertem Alkyl mit 1 bis vier Kohlenstoffen, substituiertem oder nicht substituiertem Aryl, substituiertem oder nicht substituiertem Arylalkyl, substituiertem oder nicht substituiertem Heteroaryl oder substituiertem oder nicht substituiertem Heteroarylalkyl,
(b) -C(=O)R⁹, wobei R⁹ aus der Gruppe, bestehend aus Alkyl, Aryl und Heteroaryl, ausgewählt ist.
(c) -OR¹⁰, wobei R¹⁰ aus der Gruppe, bestehend aus H und Alkyl mit 1 bis 4 Kohlenstoffen, ausgewählt ist,
(d) -C(=O)NH₂, -NR¹¹R¹², -(CH₂)ₚNR¹¹R¹², -(CH₂)ₚOR¹⁰, -O(CH₂)ₚOR¹⁰ und -O(CH₂)ₚNR¹¹R¹², wobei p 1 bis 4 ist und wobei entweder
(1) R¹¹ und R¹² jeweils unabhängig voneinander aus der Gruppe, bestehend aus H und Alkyl mit 1 bis 4 Kohlenstoffen, ausgewählt sind oder
(2) R¹¹ und R¹² zusammen eine Verbindungsgruppe mit der Formel -(CH₂)₂-X¹-(CH₂)₂- bilden, wobei X¹ aus der Gruppe, bestehend aus -O-, -S-, -CH₂-, ausgewählt ist,
ausgewählt ist,
R² aus der Gruppe, bestehend aus H, Alkyl mit 1 - 4 Kohlenstoffen, -OH, Alkoxy mit 1 bis 4 Kohlenstoffen, -OC(=O)R⁹, -OC(=O)NR¹¹R¹², -O(CH₂)ₚ NR¹¹R¹², -O(CH₂)ₚOR¹⁰, substituiertem oder nicht substituiertem Arylalkyl mit 6 bis 10 Kohlenstoffen und substituiertem oder nicht substituiertem Heteroarylalkyl, ausgewählt ist,
R³, R⁴, R⁵ und R⁶ jeweils unabhängig voneinander aus der Gruppe, bestehend aus
(a) H, Aryl, Heteroaryl, F, Cl, Br, I, -CN, CF₃, -NO₂, -OH, -OR⁹, -O(CH₂)ₚNR¹¹R¹², -OC(=O)R⁹, -OC(=O)NR²R⁷, -OC(=O)NR¹¹R¹², -O(CH₂)ₚOR¹⁰, -CH₂OR¹⁰, -NR¹¹R¹², -NR¹⁰S(=O)₂R⁹, -NR¹⁰C(=O)R⁹,
(b) -CH₂OR¹⁴, wobei R¹⁴ der Rest einer Aminosäure ist, nachdem die Hydroxylgruppe der Carboxylgruppe entfernt ist,
(c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴ oder -CH=NNR²R^{2A}, wobei R^{2A} das Gleiche wie R² ist,
(d) -S(O)_{y}R², -(CH₂)ₚS(O)_{y}R⁹, -CH₂S(O)_{y}R¹⁴, wobei y 0, 1 oder 2 ist,
(e) Alkyl mit 1 bis 8 Kohlenstoffen und Alkenyl mit 2 bis 8 Kohlenstoffen und Alkinyl mit 2 bis 8 Kohlenstoffen, wobei
(1) jede Alkyl-, Alkenyl- oder Alkinylgruppe nicht substituiert ist oder
(2) jede Alkyl-, Alkenyl- oder Alkinylgruppe mit 1 bis 3 Gruppen, ausgewählt aus der Gruppe, bestehend aus Aryl mit 6 bis 10 Kohlenstoffen, Heteroaryl, Arylalkoxy, Heterocycloalkoxy, Hydroxyalkoxy, Alkyloxy-Alkoxy, Hydroxyalkylthio, Alkoxy-Alkylthio, F, Cl, Br, I, -CN, -NO₂, -OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², -O-Tetrahydropyranyl, -NR¹¹R¹², -NR¹⁰C(=O)R⁹, -NR¹⁰CO₂R⁹, -NR¹⁰C(=O)NR¹¹R¹², -NHC(=NH)NH₂, NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴ und einem Monosaccharid mit 5 bis 7 Kohlenstoffen, wobei jede Hydroxylgruppe des Monosaccharids unabhängig voneinander entweder nicht substituiert ist oder durch H, Alkyl mit 1 bis 4 Kohlenstoffen, Alkylcarbonyloxy mit 2 bis 5 Kohlenstoffen oder Alkoxy mit 1 bis 4 Kohlenstoffen ersetzt ist, substituiert ist,
ausgewählt sind,
X² O, S oder NR¹⁰ ist,
R⁷ und R⁸ jeweils unabhängig voneinander aus der Gruppe, bestehend aus H, Alkyl mit 1 bis 4 Kohlenstoffen, Alkoxy mit 1 bis 4 Kohlenstoffen, substituiertem oder nicht substituiertem Arylalkyl mit 6 bis 10 Kohlenstoffen, substituiertem oder nicht substituiertem Heteroarylalkyl, -(CH₂)ₚOR¹⁰, -(CH₂)ₚOC(=O)NR¹¹R¹² und -(CH₂)ₚNR¹¹R¹² oder R⁷ und R⁸ zusammen eine Verbindungsgruppe mit der Formel -CH₂-X³-CH₂- bilden, wobei X³ X² oder eine Bindung ist, ausgewählt sind,
m und n jeweils unabhängig voneinander 0, 1 oder 2 sind,
Y aus der Gruppe, bestehend aus -O-, -S-, -N(R¹⁰)-, -N⁺(O⁻)(R¹⁰)-, -N(OR¹⁰)-und -CH₂-, ausgewählt ist,
Z aus der Gruppe, bestehend aus einer Bindung -O-, -CH=CH-, -S-, -C(=O)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)- und -CH(O(C=O)R⁹)CH(OC(=O)R^{9A})-, ausgewählt ist, wobei R^{9A} das Gleiche wie R⁹ ist,
R¹⁵ und R¹⁶ jeweils unabhängig voneinander aus der Gruppe, bestehend aus H, -OH, -C(=O)R¹⁰, -O(C=O)R⁹, Hydroxyalkyl und -CO₂R¹⁰, ausgewählt sind,
R¹⁷ aus der Gruppe, bestehend aus H, Alkyl, Aryl und Heteroaryl, ausgewählt ist,
A¹ und A² aus der Gruppe, bestehend aus H, H; H, OR²; H, -SR²; H, -N(R²)₂ und einer Gruppe, in der A¹ und A² zusammen eine Einheit, ausgewählt aus der Gruppe, bestehend aus =O, =S und =NR², bilden, ausgewählt sind,
B¹ und B² aus der Gruppe, bestehend aus H, H; H, -OR²; H, -SR²; H, -N(R²)₂ und einer Gruppe, in der B¹ und B² zusammen eine Einheit, ausgewählt aus der Gruppe, bestehend aus =O, =S und =NR², bilden, ausgewählt sind,
unter der Maßgabe, daß mindestens eines der Paare A¹ und A² oder B¹ und B² =O bilden.

59. Verfahren zum Modulieren der Aktivität eines Multiple Lineage Kinase-Proteins, umfassend das Inkontaktbringen des Proteins oder einer Zelle, die das Protein enthält, mit einer Verbindung mit der Formel 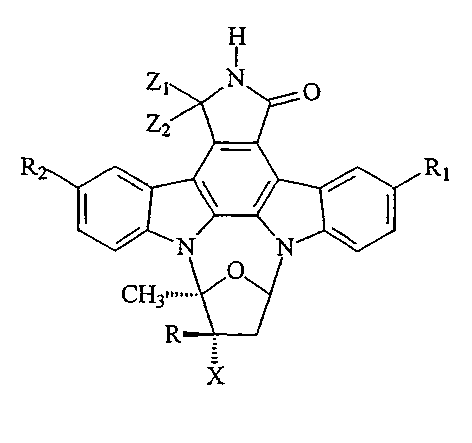 wobei
Z₁ H ist und Z₂ H ist oder Z₁ und Z₂ zusammen =O bilden,
R₁ aus der Gruppe, bestehend aus H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-Propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-Triazin)], CH₂CH₂SCH₃
und 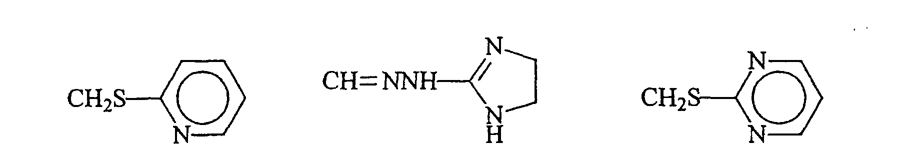 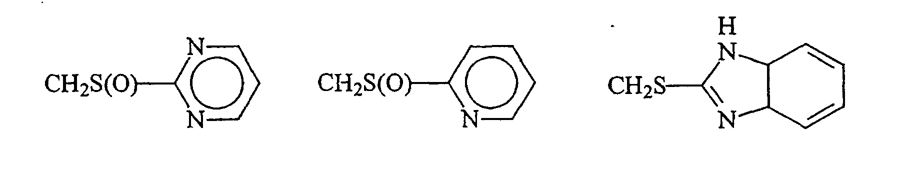 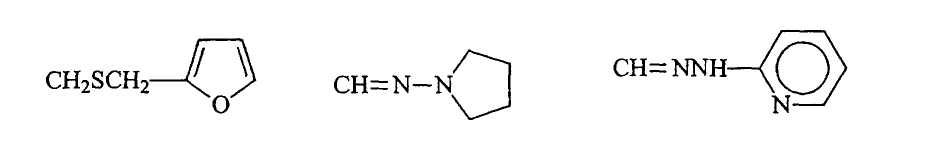 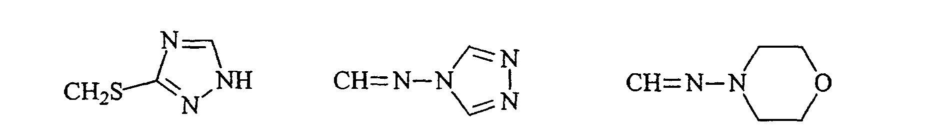 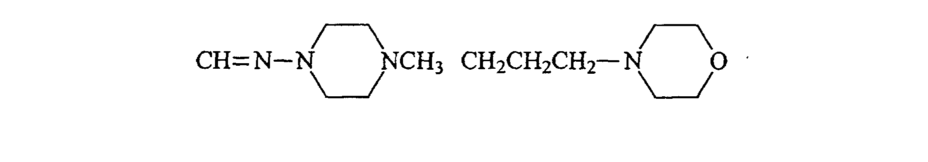 ausgewählt ist,
R₂ aus der Gruppe, bestehend aus H, Br, Cl, I, CH₂S(CH₂)₂N(CH₃)₂, NHCONHC₂H₅, CH₂SC₂H₅, CH₂OCH₂OCH₂CH₃, CH₂S(3-(1,2,4-Triazin)), CH₂CH₂SCH₃ und CH₂OH, ausgewählt ist,
X aus der Gruppe, bestehend aus H, CH₂OH, CH₂NH-SerinH, CO₂CH₃, CONHC₆H₅, CH₂NHCO₂C₆H₅, CH₂NHCO₂CH₃, CH₂N₃, CONHC₂H₅, CH₂NH-Glycin, CON(CH₃)₂, -CH₂NHCO₂-, CONH₂, CONHC₃H₇, CH₂NH-Serin, CH₂SOCH₃, CH=NOH, CH₂NH-Prolin, CH₂CH₂(2-Pyridyl), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-Hexyl, CONHCH₃, CO₂(CH₂)₄CH₃,
und 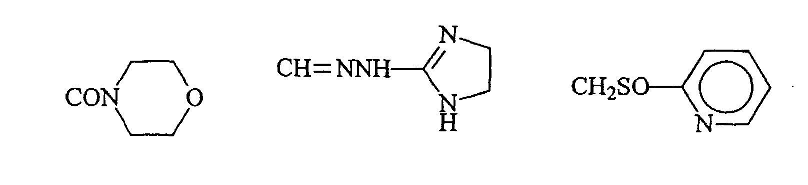 ausgewählt ist,
R aus der Gruppe, bestehend aus OH und OCH₃, ausgewählt ist.

60. Verfahren nach Anspruch 59, wobei Z₁ und Z₂ H sind, X CO₂CH₃ ist, R₁ NHCONHC₂H₅ ist, R₂ CH₂CH₂(2-Pyridyl) ist und R OH ist.

61. Verfahren nach Anspruch 59, wobei Z₁ und Z₂ H sind, X CO₂CH₃ ist, R₁ und R₂ CH₂OCH₂OCH₂CH₃ sind und R OH ist.

62. Verfahren nach Anspruch 59, wobei Z₁ und Z₂ H sind, X CO₂CH₃ ist, R₁ und R₂ CH₂SCH₂CH₃ sind und R OH ist.

63. Verfahren nach Anspruch 59, wobei Z₁, Z₂, R₁ und R₂ H sind, X CO₂CH₃ ist und R OH ist.

64. Verfahren nach Anspruch 59, wobei Z₁, Z₂, R₁ und R₂ H sind, X CO₂(CH₂)₄CH₃ ist und R OH ist.

65. Verfahren nach Anspruch 59, wobei Z₁, Z₂ und R₁ H sind, R₂ CH₂OH ist, X CO₂CH₃ ist und R OH ist.

66. Verfahren nach Anspruch 59, wobei Z₁ und Z₂ H sind, R₁ und R₂ H₂S(3-(1,2,4-Triazin)) sind, X CO₂CH₃ ist und R OH ist.

67. Verfahren nach Anspruch 59, wobei Z₁ und Z₂ H sind, R₁ Br ist, R₂ I ist, X CO₂CH₃ ist und R OH ist.

68. Verfahren nach Anspruch 59, wobei Z₁ und Z₂ H sind, R₁ und R₂ CH₂CH₂SCH₃ sind, X CO₂CH₃ ist und R OH ist.

69. Verfahren nach Anspruch 59, wobei Z₁, Z₂, R₁ und R₂ H sind, X CO₂CH₃ ist und R OCH₃ ist.

70. Verfahren nach Anspruch 59, wobei Z₁ und Z₂ zusammen =O bilden, R₁ und R₂ Br sind, X CO₂CH₃ ist und R OH ist.

## Revendications

1. Procédé d'identification d'un composé qui module l'activité d'une protéine kinase à lignée multiple et qui promeut la survie d'une cellule ou la mort d'une cellule comprenant les étapes consistant à :
(a) mettre en contact *in vitro* ladite cellule contenant ladite protéine kinase à lignée multiple avec ledit composé ;
(b) déterminer si ledit composé augmente ou diminue l'activité de ladite protéine kinase à lignée multiple ; et
(c) déterminer si ledit composé promeut la survie de la cellule ou la mort de la cellule.

2. Procédé selon la revendication 1 visant à identifier un composé qui promeut la survie de la cellule comprenant l'étape consistant à déterminer si ledit composé diminue l'activité de ladite protéine kinase à lignée multiple.

3. Procédé selon la revendication 1 visant à identifier un composé qui promeut la mort de la cellule comprenant l'étape consistant à déterminer si ledit composé augmente l'activité de ladite protéine kinase à lignée multiple.

4. Procédé selon la revendication 2, dans lequel ledit composé peut être utile dans le traitement d'une affection neurodégénérative ou d'une inflammation.

5. Procédé selon la revendication 2 ou la revendication 4 dans lequel ladite protéine est choisie à partir du groupe constitué par une kinase à lignée multiple 1, une kinase à lignée multiple 2, une kinase à lignée multiple 3, une kinase porteuse de fermeture éclair à leucine, une kinase porteuse de fermeture éclair double à leucine, et une kinase à lignée multiple 6.

6. Procédé selon la revendication 3 dans lequel ladite protéine est choisie à partir du groupe constitué par une kinase à lignée multiple 1, une kinase à lignée multiple 2, une kinase à lignée multiple 3, une kinase porteuse de fermeture éclair à leucine, une kinase porteuse de fermeture éclair double à leucine, et une kinase à lignée multiple 6.

7. Procédé selon la revendication 5 ou la revendication 6 dans lequel ladite activité protéique est déterminée par un dosage de fixation ou un dosage de la kinase *in vitro.*

8. Procédé selon la revendication 5 ou la revendication 6 dans lequel ladite cellule est un neurone.

9. Procédé selon la revendication 5 dans lequel ladite activité protéique est déterminée en mesurant l'activité ou l'état de phosphorylation d'un substrat de ladite protéine.

10. Procédé selon la revendication 6 dans lequel ladite activité protéique est déterminée en mesurant l'activité d'un substrat de ladite protéine.

11. Procédé selon la revendication 9 ou 10 dans lequel ledit substrat est choisi à partir du groupe constitué par JNK1, JNK2, JNK3, ERK1, ERK2, p38α, p38β, p38γ,p38δ, MEK1, MEK2, MKK3, MKK4 (SEK1), MEK5, MKK6, MKK7, jun, ATF2, ELK1, et l'homologue mammifère de AEX-3.

12. Procédé selon la revendication 5 dans lequel ladite activité protéique est déterminée en mesurant l'activité d'un substrat de ladite protéine, la quantité d'un substrat de ladite protéine, ou l'ARNm codant ledit substrat de ladite protéine.

13. Procédé selon la revendication 6 dans lequel ladite activité protéique est déterminée en mesurant l'activité d'un substrat de ladite protéine, la quantité de ladite protéine, ou l'ARNm codant ladite protéine.

14. Procédé selon la revendication 5 ou la revendication 6, dans lequel ladite promotion de la survie cellulaire est déterminée en utilisant des cellules présentant un risque de mourir et en comparant la quantité de cellules vivantes qui étaient mises en contact avec ledit composé avec la quantité de cellules vivantes qui n'étaient pas mises en contact avec ledit composé.

15. Procédé selon la revendication 5 ou 14 dans lequel lesdites cellules sont des motoneurones embryonnaires primaires.

16. Procédé selon la revendication 5 ou 14 dans lequel lesdites cellules surexpriment ladite protéine kinase à lignée multiple.

17. Procédé selon la revendication 5 dans lequel ladite promotion de la survie cellulaire est déterminée en observant une diminution de l'apoptose.

18. Procédé selon la revendication 6 dans lequel ladite promotion de la mort cellulaire est déterminée en observant une augmentation de l'apoptose.

19. Procédé selon la revendication 5 ou la revendication 6 dans lequel ladite cellule est impliquée dans une affection neurodégénérative.

20. Procédé selon la revendication 2 dans lequel ladite cellule est impliquée dans une inflammation.

21. Utilisation d'un composé de formule : 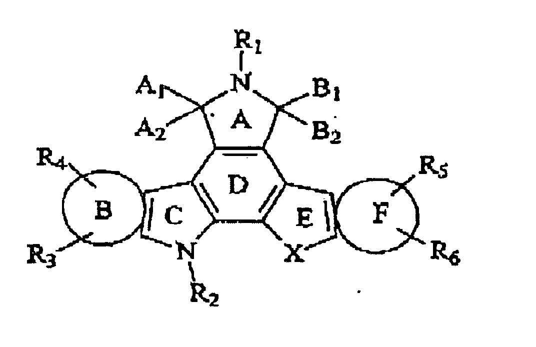 qui inhibe une protéine kinase à lignée multiple dans la fabrication d'un médicament à utiliser dans le traitement d'une affection neurodégénérative ou d'une inflammation,
dans lequel
le cycle B et le cycle F, indépendamment, chacun l'un et l'autre avec les atomes de carbone auxquels ils sont liés, forment soit
un cycle aromatique carbocyclique insaturé à 6 membres dans lequel un à trois atome(s) de carbone peu(ven)t être remplacé(s) par un ou des atomes d'azote ; ou
un cycle aromatique carbocyclique insaturé à 5 membres dans lequel soit
un atome de carbone est remplacé par un atome d'oxygène, d'azote ou de soufre ;
ou
deux atomes de carbone sont remplacés par un atome de soufre et d'azote, ou un atome d'oxygène et d'azote ;
A₁ et A₂ représentent tous deux O, et B₁ et B₂ représentent tous deux O ;
R₁ est H, un alkyle de 1 à 4 atomes de carbone (compris), un aryle, un arylalkyle, un hétéroaryle, et un hétéroarylalkyle ; COR⁹, où R⁹ un alkyle de 1 à 4 atomes de carbone (compris), ou un aryle, de préférence du phényle ou du naphtyle ; -OR¹⁰, où R¹⁰ est H ou un alkyle de 1 à 4 atomes de carbone (compris) ; -CONH₂, -NR⁷R⁸, -(CH₂)ₙNR⁷R⁸, où n est un nombre entier de 1 à 4 (compris) ; ou -O(CH₂)ₙNR⁷R⁸ ; et soit
R⁷ et R⁸ sont indépendamment H ou un alkyle de 1 à 4 atomes de carbone (compris) ; ou
R⁷ et R⁸ sont combinés ensemble pour former un groupe de liaison de formule générale -(CH₂)₂-X¹-(CH₂)₂-, où X¹ est O, S ou CH₂ ;
R₂ est H, -SO₂R⁹ ; -CO₂R⁹, COR⁹, un alkyle de 1 à 8 atomes de carbone (compris), de préférence un alkyle de 1 à 4 atomes de carbone (compris), un alcényle de 1 à 8 atomes de carbone (compris), de préférence un alcényle de 1 à 4 atomes de carbone (compris), ou un alcynyle de 1 à 8 atomes de carbone (compris), de préférence un alcynyle de 1 à 4 atomes de carbone (compris) ; ou un monosaccharide de 5 à 7 atomes de carbone (compris) où chaque groupe hydroxyle du monosaccharide est indépendamment soit non substitué ou est remplacé par H, un alkyle de 1 à 4 atomes de carbone (compris), un alkylcarbonyloxy de 2 à 5 atomes de carbone (compris) ou un alcoxy de 1 à 4 atomes de carbone (compris) ; et soit
chaque alkyle de 1 à 8 atomes de carbone (compris), alcényle de 1 à 8 atomes de carbone (compris), ou alcynyle de 1 à 8 atomes de carbone (compris) est non substitué ; ou
chaque alkyle de 1 à 8 atomes de carbone (compris), alcényle de 1 à 8 atomes de carbone (compris), ou alcynyle de 1 à 8 atomes de carbone (compris) est indépendamment substitué par un 1-3 aryle de 6 à 10 atomes de carbone (compris), de préférence du phényle ou du naphtyle ; un hétéroaryle, F, Cl, Br, I, -CN, -NO₂, OH, -OR⁹, -O(CH₂)ₙNR⁷R⁸, -OCOR⁹, -OCONHR⁹, O-tétrahydropyranyle, NH₂, -NR⁷R⁸, -NR¹⁰COR⁹, - NR¹⁰CO₂R⁹, -NR¹⁰CONR⁷R⁸, -NHC(=NH)NH₂, -NR¹⁰SO₂R⁹, -S(O)_{y}R¹¹, où R¹¹ est H ou un alkyle de 1 à 4 atomes de carbone, un aryle de 6 à 10 atomes de carbone, de préférence du phényle ou du naphtyle, ou un hétéroaryle et y équivaut à 1 ou 2 ; -SR¹¹, -CO₂R⁹, -CONR⁷R⁸, -CHO, COR⁹, -CH₂OR⁷, -CH=NNR¹¹R¹², -CH=NOR¹¹, -CH=NR⁹, -CH=NNHCN(N=NH)NH₂, -SO₂NR¹²R¹³, -PO(OR¹¹)₂, ou OR¹⁴ où R¹⁴ est le résidu d'un acide aminé une fois que le groupe hydroxyle du groupe carboxyle est éliminé ;
et soit
R¹² et R¹³ sont indépendamment H, un alkyle de 1 à 4 atomes de carbone (compris), un aryle de 6 à 10 atomes de carbone, de préférence du phényle ou du naphtyle, ou un hétéroaryle ; ou
R¹² et R¹³ sont combinés ensemble pour former un groupe de liaison, de préférence -(CH₂)₂-X¹-(CH₂)₂ ;
chaque R₃, R₄, R₅ et R₆ est indépendamment H, un aryle, de préférence un aryle de 6 à 10 atomes de carbone (compris), de préférence du phényle ou du naphtyle ; un hétéroaryle ; F, Cl, Br, I, -CN, CF₃, -NO₂, OH, -OR⁹, -O(CH₂)ₙNR⁷R⁸, -OCOR⁹, -OCONHR⁹, NH₂, -CH₂OH, -CH₂OR¹⁴, NR⁷R⁸, -NR¹⁰COR⁹, -NR¹⁰CONR⁷R⁸, SR¹¹, -S(O)_{y}R¹¹ où y équivaut à 1 ou 2 ; -CO₂R⁹, COR⁹, -CONR⁷R⁸, -CHO, -CH=NOR¹¹, -CH=NR⁹, -CH=NNR¹¹R¹², -(CH₂)ₙSR⁹, où n est un nombre entier allant de 1 à 4 (compris), -(CH₂)ₙS(O)_{y}R⁹, -CH₂SR¹⁵ où R¹⁵ est un alkyle de 1 à 4 atomes de carbone (compris) ; -CH₂S(O)_{y}R¹⁴, -(CH₂)ₙR⁷R⁸, -(CH₂)ₙNHR¹⁴, un alkyle de 1 à 8 atomes de carbone (compris), de préférence un alkyle de 1 à 4 atomes de carbone (compris) ; un alcényle de 1 à 8 atomes de carbone (compris), de préférence un alcényle de 1 à 4 atomes de carbone (compris) ; un alcynyle de 1 à 8 atomes de carbone (compris), de préférence un alcynyle de 1 à 4 atomes de carbone (compris) ; et soit
chaque alkyle de 1 à 8 atomes de carbone (compris), alcényle de 1 à 8 atomes de carbone (compris), ou alcynyle de 1 à 8 atomes de carbone (compris) est non substitué ; ou
chaque alkyle de 1 à 8 atomes de carbone (compris), alcényle de 1 à 8 atomes de carbone (compris), ou alcynyle de 1 à 8 atomes de carbone (compris) est substitué comme décrit en (d) (2) de la revendication 58 ;
X est soit
un alkylène non substitué de 1 à 3 atomes de carbone (compris) ; ou
X est un alkylène de 1 à 3 atomes de carbone (compris) substitué par un groupe R₂, de préférence OR¹⁰, -SR¹⁰, R¹⁵ où R¹⁵ est un alkyle de 1 à 4 atomes de carbone (compris) ; de phényle, du naphtyle, un arylalkyle de 7 à 14 atomes de carbone (compris), de préférence du benzyle ; ou
X est -CH=CH-, -CH(OH)-CH(OH)-, -O-, -S-, -S(=O)-, -S(=O)₂-, -CR¹⁰)₂-, -C(=O), -C(=NOR¹¹)-, -C(OR¹¹)(R¹¹)-, -C(=O)CHR¹⁵)-, CHR¹⁵)C(=O), -C(=NOR¹¹)CHR¹⁵)-, -CHR¹⁵)C(=NOR¹¹)-, -CH₂Z, -Z-CH₂-, -CH₂ZCH₂-,
où Z est C(OR¹¹)(R¹¹), O, S, C(=O), C(=NOR¹¹), ou NR¹¹;
ou
A₁ et A₂ sont tous deux chacun indépendamment H, H ; H, -OR¹¹ ; H, -SR¹¹ ; H, -NR¹¹R¹² ; ou représentent tous deux =S ou =NR¹¹ ; B₁ et B₂ représentent tous deux O ; et chaque R₁, R₂, R₃, R₄, R₅, R₆ et X sont définis en (c), (d), (e) et (f) de la revendication 58 ;
ou
A₁ et A₂ représentent tous deux O, et B₁ et B₂ sont tous deux chacun indépendamment H, H ; H, -OR¹¹ ; H, -SR¹¹ ; H, -NR¹¹R¹², ou représentent tous deux =S ou =NR¹¹ ; et chaque R₁, R₂, R₃, R₄, R₅, R₆ et X sont définis en (c), (d), (e) et (f) de la revendication 58.

22. Utilisation selon la revendication 21 dans laquelle A₁, A₂, R₁, R₃ et R₄ sont H ; B₁ et B₂ représentent tous deux O ; R₂ est CH₂CH₂OH ; R₅ et R₆ sont OCH₃ ; et X est CH₂.

23. Utilisation selon la revendication 21 dans laquelle A₁, A₂, R₁, R₃, R₅ et R₆ sont H ; B₁ et B₂ représentent tous deux O ; R₂ est CH₂CH₂OAc ; R⁴ est Br ; et X est CH₂.

24. Utilisation selon la revendication 21 dans laquelle A₁, A₂, R₁, R₃, R₅ et R₆ sont H ; B₁ et B₂ représentent tous deux O ; R₂ est CH₂CH₂OAc ; R₄ est CH₂CH₂(2-Pyr) ; et X est CH₂.

25. Utilisation selon la revendication 21 dans laquelle A₁, A₂, R₁, R₃, R₅ et R₆ sont H ; B₁ et B₂ représentent tous deux O ; R₂ est H ; R₄ est CH₂CH₂(2-pyrimidinyl) ; et X est CH₂.

26. Utilisation selon la revendication 21 dans laquelle A₁, A₂, R₁, R₃, R₅ et R₆ sont H ; B₁ et B₂ représentent tous deux O ; R₂ est H ; R₄ est CH₂CH₂(2-pyr) ; et X est CH₂.

27. Utilisation selon la revendication 21 dans laquelle A₁, A₂, R₁, R₂, R₃, R₅ et R₆ sont H ; B₁ et B₂ représentent tous deux O ; R₄ est CH₂CH₂(2-pyridazinyl) ; et X est CH₂.

28. Utilisation selon la revendication 21 dans laquelle A₁, A₂, R₁, R₃, R₄, R₅ et R₆ sont H ; B₁ et B₂ représentent tous deux O ; R₂ est CH₂CH₂OH ; et X est CH₂.

29. Utilisation selon la revendication 21 dans laquelle A₁, A₂, R₁, R₃, R₄, R₅ et R₆ sont H ; B₁ et B₂ représentent tous deux O ; R₂ est CH₂CH₂CH₂OH ; et X est CH₂.

30. Utilisation selon la revendication 21 dans laquelle A₁, A₂, R₁, R₂, R₃, R₄, R₅ et R₆ sont H ; B₁ et B₂ représentent tous deux O ; et X est S.

31. Utilisation selon la revendication 21 dans laquelle A₁, A₂, R₁, R₃, R₄, R₅ et R₆ sont H ; B¹ et B² représentent tous deux O ; R₂ est CH₂CH₂CH₂NHCO(4-(OH)Ph) ; et X est CH₂.

32. Utilisation selon la revendication 21 dans laquelle A₁, A₂, R₁, R₃, R₄, R₅ et R₆ sont H ; B₁ et B₂ représentent tous deux O ; R₂ est CH₂CH₂CH₂OH ; et X est CH₂.

33. Utilisation d'un composé de formule : 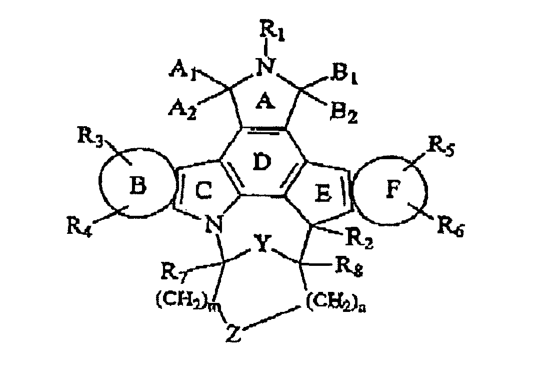 qui inhibe une protéine kinase à lignée multiple dans la fabrication d'un médicament à utiliser dans le traitement d'une affection neurodégénérative ou d'une inflammation,
dans lequel
le cycle B et le cycle F, indépendamment, chacun l'un et l'autre avec les atomes de carbone auxquels ils sont liés, sont choisis à partir du groupe constitué par :
(a) un cycle aromatique carbocyclique insaturé à 6 membres dans lequel 1 à 3 atome(s) de carbone peu(ven)t être remplacé(s) par des atomes d'azote ;
(b) un cycle aromatique carbocyclique insaturé à 5 membres ; et
(c) un cycle aromatique carbocyclique insaturé à 5 membres dans lequel soit
(1) un atome de carbone est remplacé par un atome d'oxygène, d'azote ou de soufre ;
(2) deux atomes de carbone sont remplacés par un atome de soufre et d'azote, un atome d'oxygène et d'azote, ou deux atomes d'azote ; ou
(3) trois atomes de carbone sont remplacés par trois atomes d'azote ;
R₁ est choisi à partir du groupe constitué par :
(a) H, un alkyle substitué ou non de 1 à 4 atomes de carbone, un aryle substitué ou non, un arylalkyle substitué ou non, un hétéroaryle substitué ou non, ou un hétéroarylalkyle substitué ou non ;
(b) -C(=O)R⁹, où R⁹ est choisi à partir du groupe constitué par un alkyle, un aryle et un hétéroaryle ;
(c) -OR¹⁰, où R¹⁰ est choisi à partir du groupe constitué par H et un alkyle ayant de 1 à 4 atomes de carbone ;
(d) -C(=O)NH₂, -NR¹¹R¹², -(CH₂)ₚNR¹¹R¹², -(CH₂)ₚOR¹⁰, -O(CH₂)ₚOR¹⁰ et -O(CH₂)ₚNR¹¹R¹², où p équivaut à une valeur allant de 1 à 4 ; et où soit
(1) R¹¹ et R¹² sont chacun indépendamment choisis à partir du groupe constitué par H et un alkyle ayant de 1 à 4 atomes de carbone ; ou
(2) R¹¹ et R¹² forment ensemble un groupe de liaison de formule -(CH₂)₂-X¹-(CH₂)₂, où X¹ est choisi à partir du groupe constitué par -O-, -S-, ou -CH₂- ;
R² est choisi à partir du groupe constitué par H, un alkyle ayant de 1 à 4 atomes de carbone, -OH, un alcoxy ayant de 1 à 4 atomes de carbone, -OC(=O)R⁹, -OC(=O)NR¹¹R¹², -O (CH₂)ₚNR¹¹R¹², -O(CH₂)ₚOR¹⁰, un arylakyle substitué ou non ayant de 6 à 10 atomes de carbone, et un hétéroarylalkyle substitué ou non ;
R₃, R₄, R₅ et R₆ sont chacun indépendamment choisis à partir du groupe constitué par :
(a) H, un aryle, un hétéroaryle, F, Cl, Br, I, -CN, CF₃, -NO₂, OH, -OR⁹, -O(CH₂)ₚNR¹¹R¹², -OC(=O)R⁹, -OC(=O)NR²R⁷, -OC(=O)NR¹¹R¹², -O (CH₂)ₚOR¹⁰, -CH₂OR¹⁰, -NR¹¹R¹², -NR¹⁰S(=O)₂R⁹, -NR¹⁰C(=O)R⁹ ;
(b) -CH₂OR¹⁴ où R¹⁴ est le résidu d'un acide aminé une fois que le groupe hydroxyle du groupe carboxyle est éliminé ;
(c) -NR¹⁰C (=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, ou -CH=NNR²R^{2A} où R^{2A} est identique à R² ;
(d) -S(O)_{y}R², -(CH₂)ₚS(O)_{y}R⁹, -(CH₂)S(O)_{y}R¹⁴ où y équivaut à 0, 1 ou 2 ;
(e) un alkyle ayant de 1 à 8 atomes de carbone, un alcényle ayant de 2 à 8 atomes de carbone, et un alcynyle ayant de 2 à 8 atomes de carbone, où
(1) chaque groupe alkyle, alcényle ou alcynyle est non substitué ; ou
(2) chaque groupe alkyle, alcényle ou alcynyle est substitué par 1 à 3 groupes choisis à partir du groupe constitué par un aryle ayant de 6 à 10 atomes de carbone, un hétéroaryle, un arylalcoxy, un hétérocycloalcoxy, un hydroxyalcoxy, un alkyloxy-alcoxy, un hydroxyalkylthio, un alcoxy-alkylthio, F, Cl, Br, I, -CN, -NO₂, OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², O-tétrahydropyranyle, -NR¹¹R¹², -NR¹⁰C(=O)R⁹, -NR¹⁰CO₂R⁹, -NR¹⁰C(=O)NR¹¹R¹², -NHC(=NH)NH₂, -NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCN(N=NH)NH₂, -S(=O)₂NR²R^{2A}, -P(=O)(OR¹⁰)₂, -OR¹⁴, et un monosaccharide ayant de 5 à 7 atomes de carbone où chaque groupe hydroxyle du monosaccharide est indépendamment soit non substitué ou est remplacé par H, un alkyle ayant de 1 à 4 atomes de carbone, un alkylcarbonyloxy ayant de 2 à 5 atomes de carbone, ou un alcoxy ayant de 1 à 4 atomes de carbone ;
X² est O, S ou NR¹⁰ ;
R₇ et R₈ sont chacun indépendamment choisis à partir du groupe constitué par H, un alkyle ayant de 1 à 4 atomes de carbone, un alcoxy ayant de 1 à 4 atomes de carbone, un arylalkyle substitué ou non ayant de 6 à 10 atomes de carbone, un hétéroarylalkyle substitué ou non, -(CH₂)ₚOR¹⁰, -(CH₂)ₚOC(=O)NR¹¹R¹², et -(CH₂)ₚNR¹¹R¹² ; ou R⁷ et R⁸ forment ensemble un groupe de liaison de formule -CH₂-X³-CH₂-, où X³ est X² ou une liaison ;
m et n prennent chacun indépendamment la valeur de 0, 1 ou 2 ;
Y est choisi à partir du groupe constitué par -O-, -S-, -N(R¹⁰)-, -N⁺(O⁻)(R¹⁰), -N(OR¹⁰)-, et -CH₂- ;
Z est choisi à partir du groupe constitué d'une liaison, -O-, -CH=CH-, -S-, -C(=O)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, et -CH(O(C=O)R⁹)CH(OC(=O)R^{9A})-, où R^{9A} est identique à R⁹ ;
R¹⁵ et R¹⁶ sont indépendamment choisis à partir du groupe constitué par H, -OH, -C(=O)R¹⁰, -O(C=O)R⁹, un hydroxyalkyle, et -CO₂R¹⁰ ;
R¹⁷ est choisi à partir du groupe constitué par H, un alkyle, un aryle, et un hétéroaryle ;
A₁ et A₂ sont choisis à partir du groupe constitué par H, H ; H, OR² ; H, -SR² ; H, -N(R²)₂ ; et un groupe dans lequel A₁ et A₂ forment ensemble une fraction choisie à partir de groupe constitué par =O, =S, et =NR² ;
B₁ et B₂ sont choisis à partir du groupe constitué par H, H ; H, -OR² ; H, -SR² ; H, -N(R²)₂ ; et un groupe dans lequel B₁ et B₂ forment ensemble une fraction choisie à partir de groupe constitué par =O, =S, et =NR² ;
sous réserve qu'au moins une des paires A₁ et A₂, ou B₁ et B₂, forment =O.

34. Utilisation d'un composé de formule : 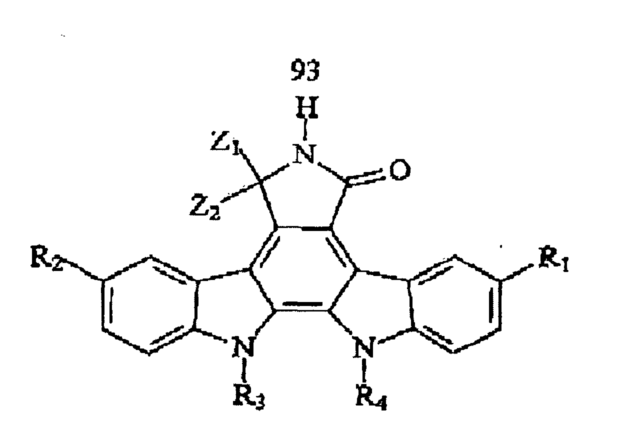 qui inhibe une protéine kinase à lignée multiple dans la fabrication d'un médicament à utiliser dans le traitement d'une affection neurodégénérative ou d'une inflammation,
dans lequel
Z₁ est H et Z₂ est H ou Z₁ et Z₂ forment ensemble =O ;
R₁ est H ou Br ;
R₂ et H ;
R₃ est H, CH₂CH=CH₂, CH₂CH₂CH₂OH, ou 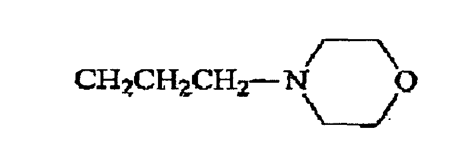 et
R₄ est H, CH₂CH=CH₂ ou CH₂CH₂CH₂OH .

35. Utilisation selon la revendication 34 où R₁, R₂, R₄, Z₁ et Z₂ sont H, et R₃ est CH₂CH=CH₂.

36. Utilisation selon la revendication 34 où R₁ est Br et R₂, R₃, R₄, Z₁ et Z₂ sont H.

37. Utilisation selon la revendication 34 où R₁, R₂, Z₁ et Z₂ sont H et R₃ et R₄ sont CH₂CH=CH₂.

38. Utilisation selon la revendication 34 où R₁, R₂, R₃, Z₁ et Z₂ sont H et R₄ est CH₂CH=CH₂.

39. Utilisation selon la revendication 34 où R₁, R₂, Z₁ et Z₂ sont H et R₃ et R₄ sont CH₂CH=CH₂ ; ou R₁, R₂, R₄, Z₁ et Z₂ sont H, et R₃ est

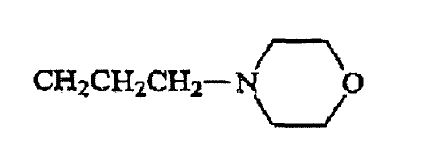

40. Utilisation d'un composé de formule : 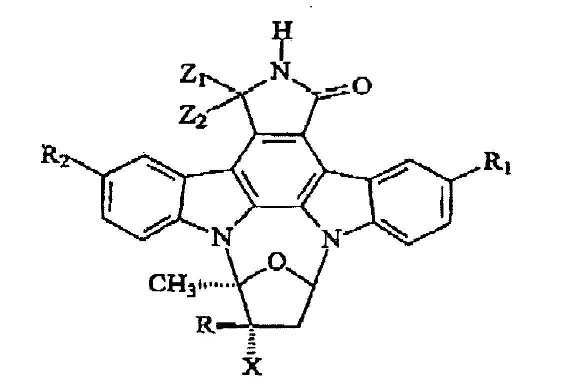 qui inhibe une protéine kinase à lignée multiple dans la fabrication d'un médicament à utiliser dans le traitement d'une affection neurodégénérative ou d'une inflammation,
dans lequel
Z₁ est H et Z₂ est H ou Z₁ et Z₂ forment ensemble =O ;
R₁ est choisi à partir du groupe constitué par H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-propyl, CH=NNH-C(=NH)NH₂, CH=N-N(CH₃)₂, CH₂S(CH₂)₂NH-n-C₄H₉, CH₂OCH₂OCH₂CH₃, CH₂S[3-(1,2,4-triazine)], CH₂CH₂SCH₃ ;
et 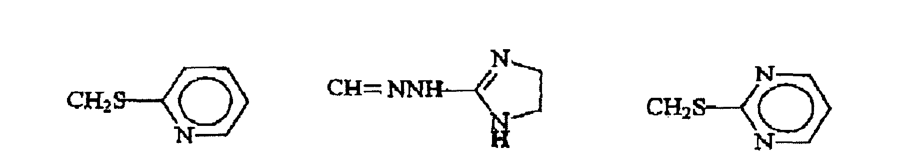 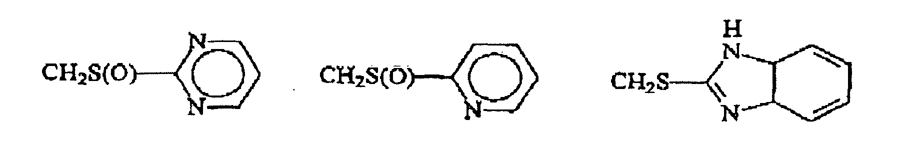 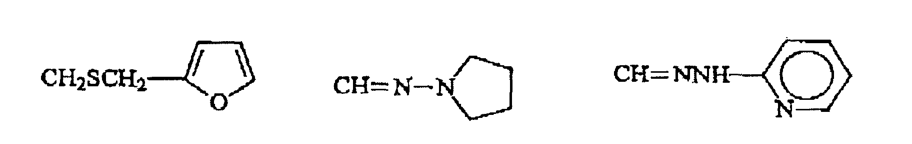 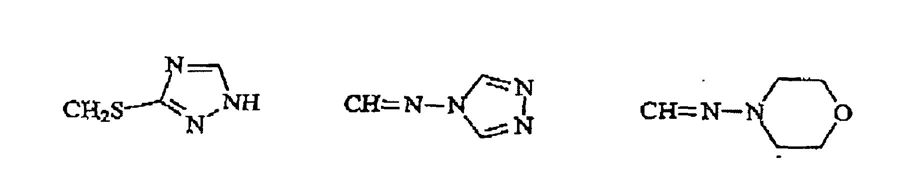 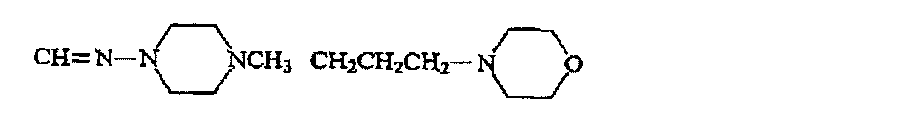 R₂ est choisi à partir du groupe constitué par H, Br, Cl, I, CH₂S(CH₂)₂N(CH₃)₂, NHCONHC₂H₅, CH₂SC₂H₅, CH₂OCH₂OCH₂CH₃, CH₂S(3-(1,2,4-triazine)), CH₂CH₂SCH₃, et CH₂OH ;
X est choisi à partir du groupe constitué par H, CH₂OH, CH₂NH-sérineH, CO₂CH₃, CONHC₆H₅, CH₂NHCO₂C₆H₅, CH₂NHCO₂CH₃, CH₂N₃, CONHC₂H₅, CH₂NH-glycine, CON(CH₃)₂, -CH₂NHCO₂-, CONH₂, CONHC₃H₇, CH₂NH-sérine, CH₂SOCH₃, CH=NOH, CH₂NH-proline, CH₂CH₂ (2-pyridil), CH=NNHC(=NH)NH₂, CONH(CH₂)₂OH, CH=NNHCONH₂, CH₂OCOCH₃, -CH₂OC(CH₃)₂O-, CH₂SC₆H₅, CH₂SOC₆H₅, CO₂n-hexyl, CONHCH₃, CO₂(CH₂)₄CH₃ ; 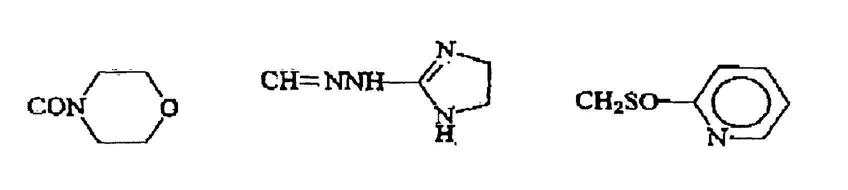 et
R est choisi à partir du groupe constitué par OH, et OCH₃.

41. Utilisation selon la revendication 40 où Z₁ et Z₂ sont H ; X est CO₂CH₃ ; R₁ est NHCONHC₂H₅ ; R₂ est CH₂CH₂(2-pyridyl) ; et R est OH.

42. Utilisation selon la revendication 40 où Z₁ et Z₂ sont H ; X est CO₂CH₃ ; R₁ et R₂ sont CH₂OCH₂OCH₂CH₃ ; et R est OH.

43. Utilisation selon la revendication 40 où Z₁ et Z₂ sont H ; X est CO₂CH₃ ; R₁ et R₂ sont CH₂SCH₂CH₃ ; et R est OH.

44. Utilisation selon la revendication 40 où Z₁, Z₂, R₁ et R₂ sont H ; X est CO₂CH₃ ; et R est OH.

45. Utilisation selon la revendication 40 où Z₁, Z₂, R₁ et R₂ sont H ; X est CO₂(CH₂)₄CH₃ ; et R est OH.

46. Utilisation selon la revendication 40 où Z₁, Z₂, et R₁ sont H ; R₂ est CH₂OH ; X est CO₂CH₃ ; et R est OH.

47. Utilisation selon la revendication 40 où Z₁ et Z₂ sont H ; R₁ et R₂ sont H₂S(3-(1,2,4-triazine)) ; X est CO₂CH₃ ; et R est OH.

48. Utilisation selon la revendication 40 où Z₁ et Z₂ sont H ; R₁ est Br ; R₂ est I ; X est CO₂CH₃ ; et R est OH.

49. Utilisation selon la revendication 40 où Z₁ et Z₂ sont H ; R₁ et R₂ sont CH₂CH₂SCH₃ ; X est CO₂CH₃ ; et R est OH.

50. Utilisation selon la revendication 40 où Z₁, Z₂, R₁ et R₂ sont H ; X est CO₂CH₃ ; et R est OCH₃.

51. Utilisation selon la revendication 40 où Z₁ et Z₂ forment ensemble =O ; R₁ et R₂ sont Br ; X est CO₂CH₃ ; et R est OH.

52. Procédé de modulation de l'activité d'une protéine kinase à lignée multiple comprenant l'étape consistant à mettre en contact ladite protéine ou une cellule contenant ladite protéine avec un composé de formule 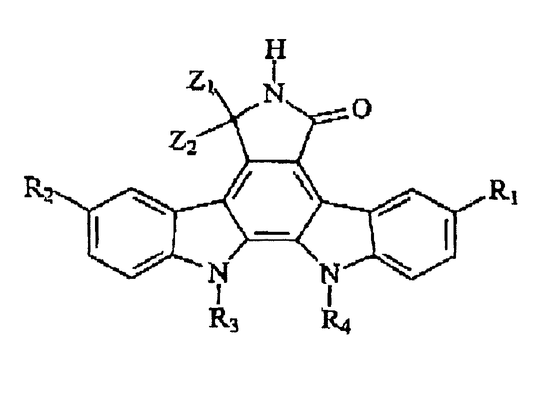 dans laquelle
Z₁ est H et Z₂ est H ou Z₁ et Z₂ forment ensemble =O ;
R₁ est H ou Br ;
R₂ et H ;
R₃ est H, CH₂CH=CH₂, CH₂CH₂CH₂OH, ou 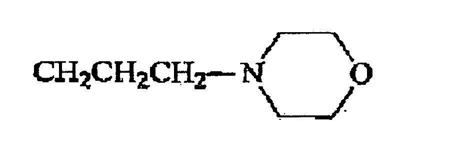 et
R₄ est H, CH₂CH=CH₂ ou CH₂CH₂CH₂OH.

53. Procédé selon la revendication 52 où R₁, R₂, R₄, Z₁ et Z₂ sont H, et R₃ est CH₂CH=CH₂.

54. Procédé selon la revendication 52 où R₁ est Br et R₂, R₃, R₄, Z₁ et Z₂ sont H.

55. Procédé selon la revendication 52 où R₁, R₂, Z₁ et Z₂ sont H et R₃ et R₄ sont CH₂CH=CH₂.

56. Procédé selon la revendication 52 où R₁, R₂, R₃, Z₁ et Z₂ sont H et R₄ est CH₂CH=CH₂.

57. Procédé selon la revendication 52 où R₁, R₂, Z₁ et Z₂ sont H et R₃ et R₄ sont CH₂CH=CH₂ ; ou R₁, R₂, R₄, Z₁ et Z₂ sont H, et R₃ est

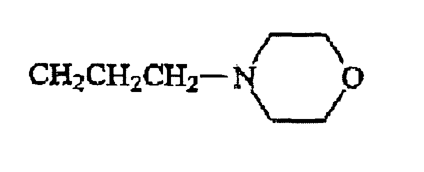

58. Procédé de modulation de l'activité d'une protéine kinase à lignée multiple comprenant l'étape consistant à mettre en contact ladite protéine ou une cellule contenant ladite protéine avec un composé de formule 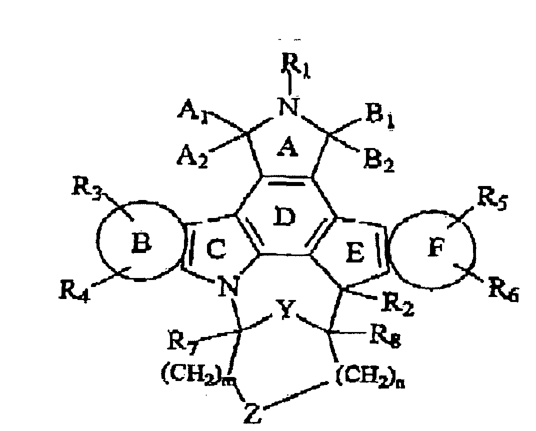 dans laquelle :
le cycle B et le cycle F, indépendamment, chacun l'un et l'autre avec les atomes de carbone auxquels ils sont liés, sont choisis à partir du groupe constitué par :
(a) un cycle aromatique carbocyclique insaturé à 6 membres dans lequel 1 à 3 atome(s) de carbone peu(ven)t être remplacé(s) par des atomes d'azote ;
(b) un cycle aromatique carbocyclique insaturé à 5 membres ;
(c) un cycle aromatique carbocyclique insaturé à 5 membres dans lequel soit
(1) un atome de carbone est remplacé par un atome d'oxygène, d'azote ou de soufre ;
(2) deux atomes de carbone sont remplacés par un atome de soufre et d'azote, un atome d'oxygène et d'azote, ou deux atomes d'azote ; ou
(3) trois atomes de carbone sont remplacés par trois atomes d'azote ;
R₁ est choisi à partir du groupe constitué par :
(a) H, un alkyle substitué ou non ayant de 1 à 4 atomes de carbone, un aryle substitué ou non, un arylalkyle substitué ou non, un hétéroaryle substitué ou non, ou un hétéroarylalkyle substitué ou non ;
(b) -C(=O)R⁹, où R⁹ est choisi à partir du groupe constitué par un alkyle, un aryle et un hétéroaryle ;
(c) -OR¹⁰, où R¹⁰ est choisi à partir du groupe constitué par H et un alkyle ayant de 1 à 4 atomes de carbone ;
(d) -C(=O)NH₂, -NR¹¹R¹², -(CH₂)ₚNR¹¹R¹², -(CH₂)ₚOR¹⁰, -O(CH₂)ₚOR¹⁰ et -O(CH₂)ₚNR¹¹R¹², où p équivaut à une valeur allant de 1 à 4 ; et où soit
(1) R¹¹ et R¹² sont chacun indépendamment choisis à partir du groupe constitué par H et un alkyle ayant de 1 à 4 atomes de carbone ; ou
(2) R¹¹ et R¹² forment ensemble un groupe de liaison de formule -(CH₂)₂-X¹-(CH₂)₂, où X¹ est choisi à partir du groupe constitué par -O, -S-, ou -CH₂- ;
R₂ est choisi à partir du groupe constitué par H, un alkyle ayant de 1 à 4 atomes de carbone, -OH, un alcoxy ayant de 1 à 4 atomes de carbone, -OC(=O)R⁹, -OC(=O)NR¹¹R¹², -O(CH₂)ₚNR¹¹R¹², -O(CH₂)ₚOR¹⁰, un arylakyle substitué ou non ayant de 6 à 10 atomes de carbone, et un hétéroarylalkyle substitué ou non ;
R₃, R₄, R₅ et R₆ sont chacun indépendamment choisis à partir du groupe constitué par :
(a) H, un aryle, un hétéroaryle, F, Cl, Br, I, -CN, CF₃, -NO₂, -OH, -OR⁹, -O (CH₂)ₚNR¹¹R¹², -OC(=O)R⁹, -OC(=O)NR²R⁷, -OC(=O)NR¹¹R¹², -O(CH₂)ₚOR¹⁰, CH₂OR¹⁰, -NR¹¹R¹², -NR¹⁰S (=O)₂R⁹, -NR¹⁰C(=O)R⁹ ;
(b) -CH₂OR¹⁴, où R¹⁴ est le résidu d'un acide aminé une fois que le groupe hydroxyle du groupe carboxyle est éliminé ;
(c) -NR¹⁰C(=O)NR¹¹R¹², -CO₂R², -C(=O)R², -C(=O)NR¹¹R¹², -CH=NOR², -CH=NR⁹, -(CH₂)ₚNR¹¹R¹², -(CH₂)ₚNHR¹⁴, ou -CH=NNR²R^{2A} où R^{2A} est identique à R² ;
(d) -S(O)_{y}R², -(CH₂)ₚS(O)_{y}R⁹, -CH₂S(O)_{y}R¹⁴ où y équivaut à 0, 1 ou 2 ;
(e) un alkyle ayant de 1 à 8 atomes de carbone, un alcényle ayant de 2 à 8 atomes de carbone, et un alcynyle ayant de 2 à 8 atomes de carbone, où
(1) chaque groupe alkyle, alcényle ou alcynyle est non substitué ; ou
(2) chaque groupe alkyle, alcényle ou alcynyle est substitué par 1 à 3 groupes choisis à partir du groupe constitué par un aryle ayant de 6 à 10 atomes de carbone, un hétéroaryle, un arylalcoxy, un hétérocycloalcoxy, un hydroalcoxy, un alkyloxy-alcoxy, un hydroxyalkylthio, un alcoxy-alkylthio, F, Cl, Br, I, -CN, -NO₂, -OH, -OR⁹, -X²(CH)ₚNR¹¹R¹², -X²(CH₂)ₚC(=O)NR¹¹R¹², -X²(CH₂)ₚOC(=O)NR¹¹R¹², -X²(CH₂)ₚCO₂R⁹, -X²(CH₂)ₚS(O)_{y}R⁹, -X²(CH₂)ₚNR¹⁰C(=O)NR¹¹R¹², -OC(=O)R⁹, -OCONHR², O-tétrahydropyranyle, -NR¹¹R¹², -NR¹⁰C (=O) R⁹, -NR¹⁰CO₂R⁹, -NR¹⁰C(=O)NR¹¹R¹², -NHC(=NH)NH₂, -NR¹⁰S(O)₂R⁹, -S(O)_{y}R⁹, -CO₂R², -C(=O)NR¹¹R¹², -C(=O)R², -CH₂OR¹⁰, -CH=NNR²R^{2A}, -CH=NOR², -CH=NR⁹, -CH=NNHCH(N=NH)NH₂, -S(=O)₂NR²R^{2A}, -P(=O)(OR¹⁰)₂, -OR¹⁴, et un monosaccharide ayant de 5 à 7 atomes de carbone où chaque groupe hydroxyle du monosaccharide est indépendamment soit non substitué ou est remplacé par H, un alkyle ayant de 1 à 4 atomes de carbone, un alkylcarbonyloxy ayant de 2 à 5 atomes de carbone, ou un alcoxy ayant de 1 à 4 atomes de carbone ;
X² est O, S ou NR¹⁰ ;
R₇ et R₈ sont chacun indépendamment choisis à partir du groupe constitué par H, un alkyle ayant de 1 à 4 atomes de carbone, un alcoxy ayant de 1 à 4 atomes de carbone, un arylalkyle substitué ou non ayant de 6 à 10 atomes de carbone, un hétéroarylalkyle substitué ou non, -(CH₂)ₚOR¹⁰, -(CH₂)ₚOC(=O)NR¹¹R¹², et -(CH₂)ₚNR¹¹R¹² ; ou R₇ et R₈ forment ensemble un groupe de liaison de formule -CH₂-X³-CH₂-, où X³ est X² ou une liaison ;
m et n prennent chacun indépendamment la valeur de 0, 1 ou 2 ;
Y est choisi à partir du groupe constitué par -O-, -S-, -N(R¹⁰)-, -N⁺(O⁻)(R¹⁰), -N(OR¹⁰)-, et -CH₂- ;
Z est choisi à partir du groupe constitué d'une liaison, -O-, -CH=CH-, -S-, -C(=O)-, -CH(OR¹⁰)-, -N(R¹⁰)-, -N(OR¹⁰)-, -CH(NR¹¹R¹²)-, -C(=O)N(R¹⁷)-, -N(R¹⁷)C(=O)-, -N(S(O)_{y}R⁹)-, -N(S(O)_{y}NR¹¹R¹²)-, -N(C(=O)R¹⁷)-, -C(R¹⁵R¹⁶)-, -N⁺(O⁻)(R¹⁰)-, -CH(OH)-CH(OH)-, et -CH(O(C=O)R⁹)CH(OC(=O)R^{9A}), où R^{9A} est identique à R⁹ ;
R¹⁵ et R¹⁶ sont indépendamment choisis à partir du groupe constitué par H, -OH, -C(=O)R¹⁰, -O(C=O)R⁹, un hydroxyalkyle, et -CO₂R¹⁰ ;
R¹⁷ est choisi à partir du groupe constitué par H, un alkyle, un aryle, et un hétéroaryle ;
A₁ et A₂ sont choisis à partir du groupe constitué par H, H ; H, OR² ; H, -SR² ; H, -N(R²)₂ ; et un groupe dans lequel A₁ et A₂ forment ensemble une fraction choisie à partir de groupe constitué par =O, =S, et =NR² ;
B₁ et B₂ sont choisis à partir du groupe constitué par H, H ; H, -OR² ; H, -SR² ; H, -N(R²)₂ ; et un groupe dans lequel B₁ et B₂ forment ensemble une fraction choisie à partir de groupe constitué par =O, =S, et =NR² ;
sous réserve qu'au moins une des paires A₁ et A₂, ou B₁ et B₂, forment =O.

59. Procédé de modulation de l'activité d'une protéine kinase à lignée multiple comprenant l'étape consistant à mettre en contact ladite protéine ou une cellule contenant ladite protéine avec un composé de formule 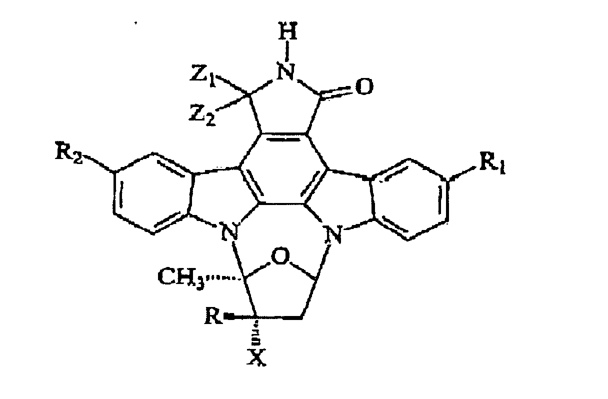 dans laquelle
Z₁ est H et Z₂ est H ou Z₁ et Z₂ forment ensemble =O ;
R₁ est choisi à partir du groupe constitué par H, Cl, CH₂SO₂C₂H₅, Br, CH₂S(CH₂)₂NH₂, CH₂S(CH₂)₂N(CH₃)₂, CH₂S(CH₂)₂NH₂, n-C₄H₉, NHCONHC₆H₅, NHCONHC₂H₅, CH₂SC₂H₅, CH₂SC₆H₅, N(CH₃)₂, CH₃, CH₂OCONHC₂H₅, NHCO₂CH₃, CH₂OC₂H₅, CH₂N(CH₃)₂, OH, O-n-propyl, CH=NNH-C (=NH) NH₂, CH=N-N(CH₃)₂, CH₂S(CH₂)₂NH-n-C₄H₉, CH₂OCH₂OCH₂CH₃, CH₂S[3-(1,2,4-triazine)], CH₂CH₂SCH₃ ;
et 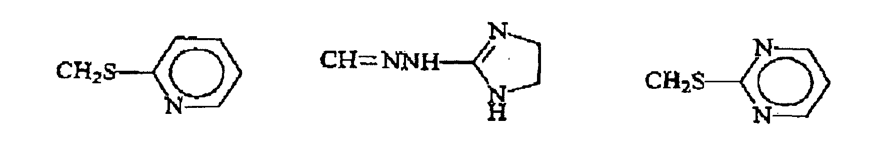 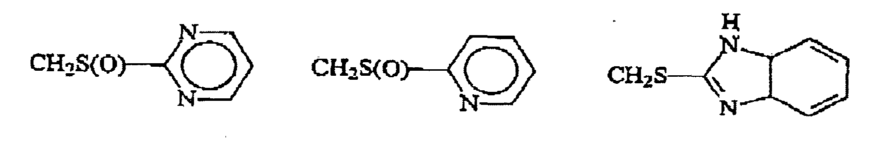 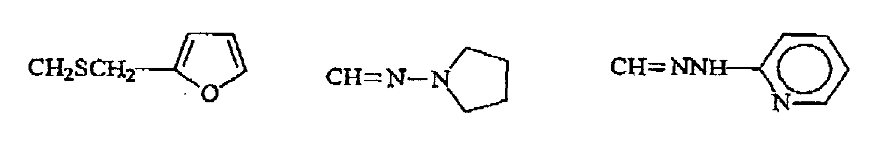 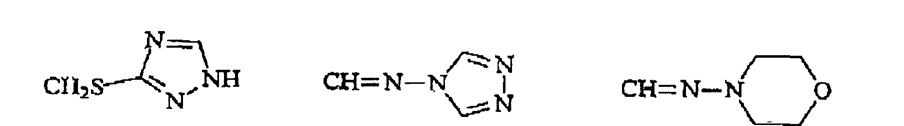 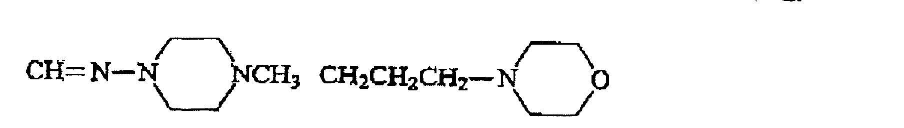 R₂ est choisi à partir du groupe constitué par H, Br, Cl, I, CH₂S(CH₂)₂N(CH₃)₂, NHCONHC₂H₅, CH₂SC₂H₅, CH₂OCH₂OCH₂CH₃, CH₂S (3- (1, 2, 4-triazine)), CH₂CH₂SCH₃, et CH₂OH ;
X est choisi à partir du groupe constitué par H, CH₂OH, CH₂NH-sérineH, CO₂CH₃, CONHC₆H₅, CH₂NHCO₂C₆H₅, CH₂NHCO₂CH₃, CH₂N₃, CONHC₂H₅, CH₂NH-glycine, CON(CH₃)₂, -CH₂NHCO₂-, CONH₂, CONHC₃H₇, CH₂NH-sérine, CH₂SOCH₃, CH=NOH, CH₂NH-proline, CH₂CH₂(2-pyridil), CH=NNHC (=NH) NH₂, CONH (CH₂)₂OH, CH=NNHCONH₂, CH₂OCOCH₃, -CH₂OC(CH₃)₂O-, CH₂SC₆H₅, CH₂SOC₆H₅, CO₂n-hexyl, CONHCH₃, CO₂(CH₂)₄CH₃ ; 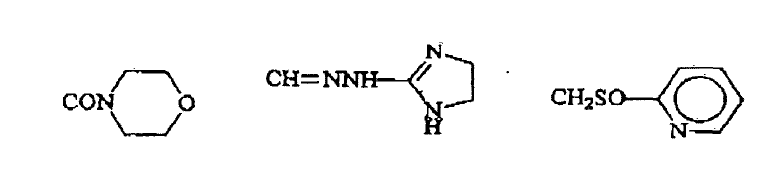 et
R est choisi à partir du groupe constitué par OH, et OCH₃.

60. Procédé selon la revendication 59 où Z₁ et Z₂ sont H ; X est CO₂CH₃ ; R₁ est NHCONHC₂H₅ ; R₂ est CH₂CH₂(2-pyridyl) ; et R est OH.

61. Procédé selon la revendication 59 où Z₁ et Z₂ sont H ; X est CO₂CH₃ ; R₁ et R₂ sont CH₂OCH₂OCH₂CH₃ ; et R est OH.

62. Procédé selon la revendication 59 où Z₁ et Z₂ sont H ; X est CO₂CH₃ ; R₁ et R₂ sont CH₂SCH₂CH₃ ; et R est OH.

63. Procédé selon la revendication 59 où Z₁, Z₂, R₁ et R₂ sont H ; X est CO₂CH₃ ; et R est OH.

64. Procédé selon la revendication 59 où Z₁, Z₂, R₁ et R₂ sont H ; X est CO₂(CH₂)₄CH₃ ; et R est OH.

65. Procédé selon la revendication 59 où Z₁, Z₂, et R₁ sont H ; R₂ est CH₂OH ; X est CO₂CH₃ ; et R est OH.

66. Procédé selon la revendication 59 où Z₁ et Z₂ sont H ; R₁ et R₂ sont H₂S(3-(1,2,4-triazine)) ; X est CO₂CH₃ ; et R est OH.

67. Procédé selon la revendication 59 où Z₁ et Z₂ sont H ; R₁ est Br ; R₂ est I ; X est CO₂CH₃ ; et R est OH.

68. Procédé selon la revendication 59 où Z₁ et Z₂ sont H ; R₁ et R₂ sont CH₂CH₂SCH₃ ; X est CO₂CH₃ ; et R est OH.

69. Procédé selon la revendication 59 où Z₁, Z₂, R₁ et R₂ sont H ; X est CO₂CH₃ ; et R est OCH₃.

70. Procédé selon la revendication 59 où Z₁ et Z₂ forment ensemble =O ; R₁ et R₂ sont Br ; X est CO₂CH₃ ; et R est OH.


### SEQUENCE LISTING

<110> Maroney, Anna
   Walton, Kevin
   Knight, Ernest
   Glicksman, Marcie
   Dionne, Craig
   Neff, Nicola
<120> Methods for Modulating Multiple Lineage Kinase Proteins and Screening Compounds Which Modulate Multiple Lineage Kinase Proteins
< 130> CEPH0431
<140>
<141>
<160> 18
<170> PatentIn Ver. 2.0
<210> 1
<211> 17
<212> PRT
<213> Artificial Sequence
<220>
<223> Description of Artificial Sequence: Novel Sequence
<400> 1
   Cys Gly Gly Ala Thr Cys Cys Ala Cys Met Gly Ile Gly Ala Tyr Tyr
   1 5 10 15
   Thr
<210> 2
<211> 23
<212> PRT
<213> Artificial Sequence
<220>
<223> Description of Artificial Sequence: Novel Sequence
<400> 2
   Gly Gly Ala Ala Thr Thr Cys Cys Ala Trp Ala Gly Gly Ala Cys Cys
   1 5 10 15
   Ala Ser Ala Cys Arg Thr Cys
   20
<210> 3
<211> 33
<212> PRT
<213> Artificial Sequence
<220>
<223> Description of Artificial Sequence: Novel Sequence
<400> 3
   Cys Gly Gly Ala Thr Cys Cys Arg Thr Ile Cys Ala Tyr Met Gly Ile
   1 5 10 15
   Gly Ala Tyr Tyr Thr Ile Gly Cys Ile Gly Cys Ile Met Gly Ile Ala
   20 25 30
   Ala
<210> 4
<211> 30
<212> PRT
<213> Artificial Sequence
<220>
<223> Description of Artificial Sequence: Novel Sequence
<400> 4
   Gly Gly Ala Ala Thr Thr Ile Ala Tyr Ile Gly Gly Ala Trp Ala Ile
   1 5 10 15
   Gly Trp Cys Cys Ala Ile Ala Cys Arg Thr Cys Ile Ser Trp
   20 25 30
<210> 5
<211> 10
<212> PRT
<213> Artificial Sequence
<220>
<223> Description of Artificial Sequence: Novel Sequence
<400> 5
   Met Glu Glu Glu Glu Tyr Met Pro Met Glu
   1 5 10
<210> 6
<211> 24
<212> DNA
<213> Artificial Sequence
<220>
<223> Description of Artificial Sequence: Novel Sequence
<400> 6
   gtggctgtgc gggcagctcg ccag 24
<210> 7
<211> 21
<212> DNA
<213> Artificial Sequence
<220>
<223> Description of Artificial Sequence: Novel Sequence
<400> 7
   gagaccctgg atctcgcgct t 21
<210> 8
<211> 9
<212> PRT
<213> Artificial Sequence
<220>
<223> Description of Artificial Sequence: Novel Sequence
<400> 8
   Met Asp Tyr Lys Asp Asp Asp Asp Lys
   1 5
<210> 9
<211> 27
<212> DNA
<213> Artificial Sequence
<220>
<223> Description of Artificial Sequence: Novel Sequence
<400> 9
   cggatccgtg acaccagtcg gaacctt 27
<210> 10
<211> 28
<212> DNA
<213> Artificial Sequence
<220>
<223> Description of Artificial Sequence: Novel Sequence
<400> 10
   ggaattcacc agtaagctcc agcacatc 28
<210> 11
<211> 33
<212> DNA
<213> Artificial Sequence
<220>
<223> Description of Artificial Sequence: Novel Sequence
<400> 11
   ataattcgtg ctagcgccag agtctagccg gtg 33
<210> 12
<211> 39
<212> DNA
<213> Artificial Sequence
<220>
<223> Description of Artificial Sequence: Novel Sequence
<400> 12
   ataagcttcc tcagtgcaag tggatcgcgc agcccctga 39
<210> 13
<211> 8
<212> PRT
<213> Artificial Sequence
<220>
<223> Description of Artificial Sequence: Novel Sequence
<400> 13
   Asp Tyr Lys Asp Asp Asp Asp Lys
   1               5
<210> 14
<211> 69
<212> DNA
<213> Artificial Sequence
<220>
<223> Description of Artificial Sequence: Novel Sequence
<400> 14
   ataaagcttc cagaggccat ggactacaag gacgacgatg acaaggcctg cctccatgaa 60
   acccgaaca 69
<210> 15
<211> 18
<212> DNA
<213> Artificial Sequence
<220>
<223> Description of Artificial Sequence: Novel Sequence
<400> 15
   gacagggcgg ccggctct 18
<210> 16
<211> 583
<212> DNA
<213> Artificial Sequence
<220>
<223> Description of Artificial Sequence: Novel Sequence
<400> 16
   gaattcggca cgagaggact cgcaggtgtc cggcgacgag ggctggtgga ccgggcagct 60
   gaaccagcgg gtgggcatct tccccagcaa ctacgtgacc ccgcgcagcg ccttctccag 120
   ccgctgccag cccggcggcg aggaccccag ttgctacccg cccattcagt tgttagaaat 180
   tgattttgcg gagctcacct tggaagagat tattggcatc gggggctttg ggaaggtcta 240
   tcgtgctttc tggatagggg atgaggttgc tgtgaaagca gctcgccacg accctgatga 300
   ggacatcagc cagaccatag agaatgttcg ccaagaggcc aagctcttcg ccatgctgaa 360
   gcaccccaac atcattgccc taagaggggt atgtctgaag gagcccaacc tctgcttggt 420
   catggagttt gctcgtggag gacctttgaa tagagtgtta tctgggaaaa ggattccccc 480
   agacatcctg gtgaattggg ctgtgcagat tgccagaggg atgaactact tacatgatga 540
   ggcaattgtt cccatcatcc accgcgacct taagtccagc aac 583
<210> 17
<211> 194
<212> PRT
<213> Artificial Sequence
<220>
<223> Description of Artificial Sequence: Novel Sequence
<400> 17
   Asn Ser Ala Arg Glu Asp Ser Gln Val Ser Gly Asp Glu Gly Trp Trp
   1 5 10 15
   Thr Gly Gln Leu Asn Gln Arg Val Gly Ile Phe Pro Ser Asn Tyr Val
   20 25 30
   Thr Pro Arg Ser Ala Phe Ser Ser Arg Cys Gln Pro Gly Gly Glu Asp
   35 40 45
   Pro Ser Cys Tyr Pro Pro Ile Gln Leu Leu Glu Ile Asp Phe Ala Glu
   50 55 60
   Leu Thr Leu Glu Glu Ile Ile Gly Ile Gly Gly Phe Gly Lys Val Tyr
   65 70 75 80
   Arg Ala Phe Trp Ile Gly Asp Glu Val Ala Val Lys Ala Ala Arg His
   85 90 95
   Asp Pro Asp Glu Asp Ile Ser Gln Thr Ile Glu Asn Val Arg Gln Glu
   100 105 110
   Ala Lys Leu Phe Ala Met Leu Lys His Pro Asn Ile Ile Ala Leu Arg
   115 120 125
   Gly Val Cys Leu Lys Glu Pro Asn Leu Cys Leu Val Met Glu Phe Ala
   130 135 140
   Arg Gly Gly Pro Leu Asn Arg Val Leu Ser Gly Lys Arg Ile Pro Pro
   145 150 155 160
   Asp Ile Leu Val Asn Trp Ala Val Gln Ile Ala Arg Gly Met Asn Tyr
   165 170 175
   Leu His Asp Glu Ala Ile Val Pro Ile Ile His Arg Asp Leu Lys Ser
   180 185 190
   Ser Asn
<210> 18
<211> 8
<212> PRT
<213> Artificial Sequence
<220>
<223> Description of Artificial Sequence: Novel Sequence
<400> 18
   Asp Tyr Lys Asp Asp Asp Asp Lys
   1 5